# EUROPEAN PATENT APPLICATION

(11) **EP 4 741 425 A1**
(43) Date of publication of application: **13.05.2026**
(21) Application number: 24838761.5
(22) Date of filing: 08.07.2024
(51) Int. Cl.: C07K 16/46, C07K 16/32, C07K 16/28, A61K 39/395, A61K 35/74, A61P 35/00, C07K 19/00, C12N 1/21

(54) **ANTI-TUMOR BACTERIA FOR REDUCING NEUTROPHIL LEVEL IN TUMOR**

(30) Priority: 07.07.2023 CN 202310838472
(71) Applicant: Shenzhen Synthetica Pioneering Co., Ltd., Shenzhen, Guangdong 518107 (CN)
(72) Inventor: LIU, Chenli, Shenzhen, Guangdong 518107 (CN); MALEEHA, Saifi, Shenzhen, Guangdong 518107 (CN); DONG, Yuxuan, Shenzhen, Guangdong 518107 (CN); LI, Yang, Shenzhen, Guangdong 518107 (CN); ZANG, Zhongsheng, Shenzhen, Guangdong 518107 (CN); CHEN, Kening, Shenzhen, Guangdong 518107 (CN)
(74) Representative: Michalski Hüttermann & Partner Patentanwälte mbB
(86) International application number: PCT/CN2024/104173
(87) International publication number: WO 2025/011508

(57) **Abstract**

Provided are a fusion protein, comprising an antibody that binds to a neutrophil or an antigen-binding fragment thereof, and a cytotoxin or an active fragment thereof. Also provided are modified bacteria having anti-tumor activity and expressing the fusion protein, the antibody or the antigen-binding fragment thereof, or the toxin or the active fragment thereof in a low-oxygen environment (such as a tumor). Also provided are a pharmaceutical composition comprising the modified bacteria and an anti-tumor use thereof.

## Description

### Technical Field

The present invention relates to a modified bacterium with anti-tumor activity, a pharmaceutical composition comprising the modified bacterium and the anti-tumor use thereof.

### Background

Cancer has become one of the most important diseases threatening human life and health, but existing treatments (radiotherapy, chemotherapy, surgery and targeted drugs) all have shortcomings and there is an urgent need to develop new treatment approaches. Traditional bacterial tumor therapies, represented by Coley's toxin, have a history of 150 years, but are known for their unstable efficacy and safety concerns. The development of genetic engineering technology has made great progress in reducing the toxicity of bacterial strains, and a series of clinical trials have demonstrated that the attenuated engineered bacteria for human tumor therapy are safe but with limited efficacy, i.e., are unable to combine safety and therapeutic efficacy, thus failing to satisfy the needs of clinical tumor therapy. Further optimization for tumor therapy is imperative.

Further, the inventors have found that neutrophils in the human body limit the application of bacterial therapy for tumor. Therefore, there is a need to optimize strains for bacterial therapy for tumor to reduce the effects of neutrophils.

### Summary of the Invention

According to the present invention, a strictly anaerobic bacterium is constructed by means of synthetic biological genetic circuits, which shows therapeutic efficacy against tumors after being administered to an animal body or a human body, and can be cleared by normal tissues and organs in a short period of time, thereby reducing the toxic and side effects on the animal body or the human body due to the long-term retention of the bacterium in vivo, and thus providing a more reliable anti-tumor efficacy and safety. In addition, exogenous proteins (such as antibodies against neutrophils or fusion proteins of the antibodies and cytotoxins) are expressed in the bacterium to inhibit the recruitment of neutrophils or kill neutrophils in tumors, thereby enhancing the anti-tumor efficacy of the bacterium.

In a first aspect, the present invention provides a fusion protein comprising an antibody (e.g., a nanobody) that binds to a neutrophil or an antigen-binding fragment thereof and a cytotoxin or an active fragment thereof.

In some embodiments, the antibody comprises complementary determining regions (CDRs) selected from the group consisting of CDR1 comprising residues 31-35 of SEQ ID NO: 19, CDR2 comprising residues 50-65 of SEQ ID NO: 19, and CDR3 comprising residues 98-104 of SEQ ID NO: 19; CDR1 comprising residues 31-35 of SEQ ID NO: 20, CDR2 comprising residues 50-67 of SEQ ID NO: 20, and CDR3 comprising residues 100-111 of SEQ ID NO: 20; and CDR1 comprising residues 31-35 of SEQ ID NO: 21, CDR2 comprising residues 50-64 of SEQ ID NO: 21, and CDR3 comprising residues 98-115 of SEQ ID NO: 21. In some embodiments, the antibody comprises an amino acid sequence selected from SEQ ID NOs: 19, 20 and 21.

In some embodiments, the cytotoxin is Pseudomonas exotoxin (PE). In some embodiments, the cytotoxin comprises the amino acid sequence of SEQ ID NO: 25. In some embodiments, the fragment of the cytotoxin comprises the amino acid sequence of SEQ ID NO: 26 or 27.

In some embodiments, the fusion protein further comprises a signal peptide. In some embodiments, the signal peptide comprises the amino acid sequence of SEQ ID NO: 28 or 29.

In some embodiments, the fusion protein further comprises a linker and/or a tag sequence.

In a second aspect, the present invention provides an expression construct comprising a nucleotide sequence encoding an antibody (e.g., nanobody) that binds to neutrophils or antigen-binding fragment thereof operably linked to a strictly hypoxia inducible promoter.

In some embodiments, the antibody comprises complementary determining regions (CDRs) selected from the group consisting of CDR1 comprising residues 31-35 of SEQ ID NO: 19, CDR2 comprising residues 50-65 of SEQ ID NO: 19, and CDR3 comprising residues 98-104 of SEQ ID NO: 19; CDR1 comprising residues 31-35 of SEQ ID NO: 20, CDR2 comprising residues 50-67 of SEQ ID NO: 20, and CDR3 comprising residues 100-111 of SEQ ID NO: 20; and CDR1 comprising residues 31-35 of SEQ ID NO: 21, CDR2 comprising residues 50-64 of SEQ ID NO: 21, and CDR3 comprising residues 98-115 of SEQ ID NO: 21. In some embodiments, the antibody comprises an amino acid sequence selected from SEQ ID NOs: 19, 20 and 21.

In some embodiments, the nucleotide sequence further encodes a signal peptide. In some embodiments, the signal peptide comprises the amino acid sequence of SEQ ID NO: 28 or 29.

In some embodiments, the nucleotide sequence further encodes a tag sequence.

In some embodiments, the nucleotide sequence encodes the fusion protein of the present invention.

In a third aspect, the present invention provides a modified bacterium.

In one embodiment, the bacterium comprises the expression construct of the present invention and a hypoxia regulated essential gene expression cassette comprising an essential gene of the bacterium under the control of a second strictly hypoxia inducible promoter, and the bacterium is deficient in at least one gene required for survival in macrophages or a functional expression product thereof, as compared to an unmodified starting strain.

In one embodiment, the bacterium comprises the expression construct of the present invention and a hypoxia regulated essential gene expression cassette comprising an essential gene of the bacterium under the control of a second strictly hypoxia inducible promoter, and the bacterium is deficient in at least one gene involved in or regulating the endogenous anti-oxidative stress response pathway or a functional expression product thereof, as compared to an unmodified starting strain.

In one embodiment, the bacterium comprises the expression construct of the present invention and a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

In one embodiment, the bacterium comprises the expression construct of the present invention and a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium expresses a wild-type lipopolysaccharide (LPS) and is deficient in at least one gene required for survival in macrophages or a functional expression product thereof, as compared to an unmodified starting strain.

In one embodiment, the bacterium is a modified *Salmonella typhimurium* comprising the expression construct of the present invention and a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

In one embodiment, the modified bacterium is a modified Salmonella typhimurium, wherein the bacterium comprise the expression construct of the present invention and a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of an promoter that is active under acid pH condition, and the bacterium expresses a wild-type lipopolysaccharide (LPS) and is deficient in at least one gene required for survival in macrophages or a functional expression product.

In one embodiment, the present invention provides a pharmaceutical composition comprising the bacterium of any one of the preceding embodiments. In one embodiment, the pharmaceutical composition is used for treating a malignant tumor.

In one embodiment, the present invention provides a method of treating a malignant tumor comprising administering the pharmaceutical composition of the present invention to a subject having a malignant tumor.

In one embodiment, the present invention provides use of the modified bacterium in the preparation of a medicament for treating a malignant tumor.

### Brief Description of Drawings

FIG.1 shows a schematic diagram of the construction scheme of DB-ZW1 strain.
FIG.2 shows changes in the distribution of DB-ZW1 in tumors and tissues over time.
FIG. 3 shows the tumor inhibiting effect of DB- ZW1 on bladder cancer, melanoma and orthotopic colon cancer. Among them, FIGs. 3A-C illustrate the effects of DB-ZW1 on the tumor volume of subcutaneous bladder cancer, orthotopic melanoma, and subcutaneous orthotopic colon cancer, respectively; and FIG. 3D illustrates the effect of DB-ZW1 on the tumor number of orthotopic colon cancer.
FIG. 4 shows the map of plasmid pET-26b.
FIG. 5 shows the map of plasmid pET26b-pelB-Nb127D01-HA-his.
FIG. 6 shows the detection of strains BL21(DE3)-pET26b-pelB-Nb127D01-HA-his (FIG. 6A), BL21(DE3)-pET26b-pelB-Nb163E3-HA-his (FIG. 6B) and BL21(DE3)-pET26b-pelB-Nb97A9-HA-his (FIG. 6C) by western blot for the expression of nanobodies induced by IPTG at different concentrations. P represents the lysed bacterial precipitate, S represents the supernatant of the lysate, and LB represents the LB medium supernatant.
FIG. 7 shows the detection of the IPTG induced expression (FIG. 7A) and the anaerobic inducible expression (FIG. 7B) of the fusion protein by western blot.
FIG. 8 shows that the DB-ZW1 level in tumor of MB49 bladder cancer is increased by neutralizing intratumoral neutrophils. Left panel: intratumoral image of mice administered with DB-ZW1 only; right panel: intratumoral image of mice administered with DB-ZW1+Ly6G/Ly6c(Gr-1) antibody.
FIG. 9 shows that the therapeutic effect of DB-ZW1 on 4T1 breast cancer is increased by neutralizing intratumoral neutrophils. FIG. 9A shows the result of flow cytometry of neutralizing neutrophils in the tumor of the 4T1 breast cancer model with the Ly6G/Ly6c(Gr-1) antibody (αGR-1); FIG. 9B shows the change in size of the 4T1 tumor injected with the artificial anti-tumor bacterium ZW1 one day after the neutralization with the αGR-1 antibody; FIG. 9C shows the typical images of tumors from each group one day after the injection with the bacterium.
FIG. 10 shows the proportion of the neutrophils successfully bound by the nanobody in the total number of neutrophils, wherein No Nb represents a sample without adding the nanobody, and Control strain represents a sample added with LB supernatant for culturing E. coli BL21 strain.
FIG. 11 shows the therapeutic effect of DB-ZW1 expressing nanobody/fusion protein in mouse tumor model.
FIG. 12 shows the blood and intratumoral neutrophil contents of mice after the treatment with DB-ZW1 expressing nanobody/fusion protein.
FIG. 13 shows the expression of the nanobody, toxin protein or nanobody fused to toxin protein under IPTG induction. M represents Marker, LB represents the secreted protein in the culture medium supernatant, S represents the expressed but unsecreted soluble protein comprised in the supernatant of the lysis, and P represents the functional inclusion body protein comprised in the precipitate of bacterial debris.

### Detailed Description of the Invention

### DEFINITIONS

In the present invention, the scientific and technical terms used herein have the meaning as commonly understood by a person skilled in the art unless otherwise specified. Also, the protein and nucleic acid chemistry, molecular biology, cell and tissue culture, microbiology, immunology related terms, and laboratory procedures used herein are terms and routine steps that are widely usedin the corresponding field. At the same time, for a better understanding of the present invention, the definitions and explanations of the related terms are provided below.

"Hypoxia regulated essential gene expression cassette" herein means a stretch of DNA capable of promoting the expression of an essential gene under hypoxic conditions, which contains an essential gene under the control of a hypoxia inducible promoter and, if necessary, may further contain other regulatory elements required for the expression of the essential gene.

As used herein, "essential gene" refers to a gene that is decisive for the growth and / or survival of a bacterium, in the absence of which or its functional expression product the bacterium fails to survive, divide and / or grow normally. Typical examples of deletion of an essential gene or a functional expression product thereof are nutritionally deficient strains that fail to survive, divide and / or grow normally under in vitro culture conditions or in vivo environment in the absence of specific exogenous supplements. Essential genes usually appear as a single copy on the bacterial chromosome.

As used herein, "strictly hypoxia inducible promoter" refers to a promoter that is capable of initiating the transcription of a specific gene under anaerobic conditions but is virtually impossible to activate and produce transcripts under aerobic conditions.

"Strictly hypoxia inducible promoters" useful in the present invention can be identified by the following experiments:
An expression strain containing an essential gene (e.g., dapA/dapE) under the control of a promoter to be determined is constructed, and subjected to Luria-Bertani (abbreviated as LB) plate streaking in an anaerobic incubator and anaerobic culture at 37°C for 24 h. Under anaerobic conditions, single clones are picked and resuspended in 20 µL of LB broth. The 20 µl resuspension of a single clone is equally added to the liquid in the following four tubes (A, B, C and D, 5 µl per tube):
A. 2 ml LB broth,
B. 2 ml LB broth,
C. 2 ml LB broth+DAP,
D. 2 ml LB broth+DAP.

The tubes A and C are incubated at 37°C in an anaerobic incubator for 24 h, and the tubes B and D are incubated at 37°C in an aerobic shaker for 24 h.
If the bacteria in the tubes A, C and D can grow, and the bacteria in the tube B cannot grow (the detected OD600 value is less than 0.05), the promoter is considered as a strictly hypoxia inducible promoter.

Exemplary strictly hypoxia inducible promoters that can be used in the present invention as identified by the experiments above are shown in Table 1.

**Table 1**

| Name of Promoter | Sequence | SEQ ID NO: |
|---|---|---|
| pepTp | GCAGGGGTAAAAGTGACCTGACGCAATATTTGTCT TTTCTTGCTTCTTAATAATGTTGTCACAAAAAGTG AGGGTGACTACATGG | 9 |
| fnrSp | CGCCGCAAAGTTTGAGCGAAGTCAATAAACTCTCT ACCCATTCAGGGCAATATCTCTCTTGCAGGTGAAT GCAACGTCAAGCGAT | 10 |
| ysgAp | TCAGAAGAAGCAAAAAGACACTTTACCGAAGGGTT TAACATTTTTTCGTGATACTCATCACCATGACGCA AATGCGTTGCATAAA | 11 |
| ssbp1 | AACCGAGGTCACAACATAGTAAAAGCGCTATTGGT AATGGTACAATCGCGCGTTTACACTTATTCAGAAC GATTTTTTTCAGGAG | 12 |
| Hip1 | GGATAAAAGTGACCTGACGCAATATTTGTCTTTTC TTGCTTAATAATGTTGTCA | 13 |
| BBa_I14018 | TGTAAGTTTATACATAGGCGAGTACTCTGTTATGG | 14 |
| BBa_R1074 | TTAAATTTCCTCTCGTCAGGCCGGAATAACTCCCT ATAATGCGCCACCACACTGATAGTGCTAGTGTAGA TCAC | 15 |
| Ptet-Fnr | AAAATTGATCTGAATCAATATTTTGACAAAAATTG ATCTGAATCAATATTTACTGAGC | 16 |
| Ptet-arcA | GTTAATAAAATGTTATTGACAGTTAATAAAATGTT ATACTGAGC | 17 |
| sseA | TTAGCACGTTAATTATCTATCGTGTATATG | 18 |

As used herein, "gene involved in or regulating the endogenous anti-oxidative stress response pathway" refers to a gene involved in the resistance of bacteria (e.g., intracellular bacteria, such as Salmonella) against reactive oxygen species (ROS) in an in vivo or in vitro environment.

As used herein, "gene required for survival in macrophages" refers to a gene involved in maintaining or enhancing the viability of intracellular bacteria in macrophages. The function of the products of these genes is associated to resistance to the killing effect of macrophages on microorganisms, and thus deletion of these genes or their functional expression products can reduce the viability of bacteria (e.g., intracellular bacteria, such as Salmonella) in macrophages.

As used herein, the "functional expression product" includes RNA, proteins, and the like.

As used herein, "facultative anaerobic bacteria" refers to bacteria that can survive under both aerobic and anaerobic conditions.

As used herein, the "pH regulated expression cassette" refers to a group of gene expression elements in which the expression of the corresponding gene (s) is regulated by environmental pH.

As used herein, "a promoter that is active under acid pH condition" refers to a promoter that can be activated under acid pH condition and regulate the expression of the corresponding gene (s). In some embodiments, the promoter that is active under acid pH condition of the present invention may be activated or still have promoter activity at a pH less than 7.0, 6.9, 6.8, 6.7, 6.6, 6.5, 6.4, 6.3, 6.2, 6.1, 6.0, 5.9, 5.8, 5.7, 5.6, or 5.5. In some embodiments, the above promoter that is active under acid pH condition also has promoter activity at neutral pH (e.g., pH 7.0 to 7.4).

"Polypeptide", "peptide", and "protein" are used interchangeably in the present invention to refer to a polymer of amino acid residues. The terms apply to an amino acid polymer in which one or more amino acid residues is artificial chemical analogue of corresponding naturally occurring amino acid (s), as well as to a naturally occurring amino acid polymer. The terms "polypeptide", "peptide", "amino acid sequence", and "protein" may also include modified forms including, but not limited to, glycosylation, lipid ligation, sulfation, γcarboxylation of glutamic acid residues, and ADP-ribosylation.

As used herein, "polynucleotide" refers to a macromolecule formed by linking multiple nucleotides via phosphodiester linkages, wherein the nucleotide comprises ribonucleotide and deoxyribonucleotide. The sequence of the polynucleotide of the present invention can be subjected to codon optimization for different host cells (such as Escherichia coli), thereby improving the expression of the polypeptide. Methods for codon optimization are known in the art.

"Sequence identity" between two polypeptides or two polynucleotide sequences refers to percentage of identical amino acids or nucleotides between the sequences. Methods for assessing the level of sequence identity between polypeptide or polynucleotide sequences are known in the art. Sequence identity can be assessed using various known sequence analysis software. For example, sequence identity can be assessed by an on-line alignment tool of EMBL-EBI (https: / / www.ebi.ac.uk / Tools / psa /). Sequence identity between two sequences can be assessed using default parameters through Needleman-Wunsch algorithm.

As used herein, the term "live bacterium" refers to a strain that is viable, and characterized by active nutrient metabolism, and is capable of performing its own biological functions. Live bacteria may include bacterial biomass produced in the metabolic process of the strain.

The term "Gram-negative bacteria" used herein refers to bacteria that do not retain the initial basic dye stain (e.g., crystal violet) that is part of the procedure known as the Gram stain. In an exemplary Gram stain, cells are first fixed to a slide by heat and stained with a basic dye (e.g., crystal violet), which is taken up by both Gram-negative and Gram-positive bacteria. The slides are then treated with a mordant (e.g., Gram's iodine), which binds to basic dye (e.g. crystal violet) and traps it in the cell. The cells are then washed with acetone or alcohol, and then counterstained with a second dye of different color (e.g., safranin). Gram-positive organisms retain the initial violet stain, while Gram-negative organisms are decolorized by the wash solvent organic and hence show the counterstain. Exemplary Gram-negative bacteria include, but are not limited to, Escherichia spp., Shigella spp., Salmonella spp., Campylobacter spp., Neisseria spp., Haemophilus spp., Aeromonas spp., Francisella spp., Yersinia spp., Klebsiella spp., Bordetella spp., Legionella spp., Corynebacteria spp., Citrobacter spp., Chlamydia spp., Brucella spp., Pseudomonas spp., Helicobacter spp. and Vibrio spp.

As used herein, the term "malignant solid tumor" refers to an abnormal mass of tissues, usually not comprising cysts or liquid areas. Solid tumors may be benign (not cancerous) or malignant (cancerous). Different types of malignant solid tumors are named for the type of cells that form them. Examples of malignant solid tumors are sarcomas, carcinomas, and lymphomas. Leukemia (blood cancer) does not usually form a malignant solid tumor (as defined by the NIH National Cancer Institute). Malignant solid tumors include, but are not limited to, clusters of abnormal cells that may originate from different tissue types, for example, the liver, colon, colorectum, skin, breast, pancreas, cervix, corpus uteri, bladder, gallbladder, kidney, larynx, lip, oral cavity, esophagus, ovary, prostate, stomach, testis, thyroid, or lungs, and thus malignant solid tumors include malignant solid liver tumors, colon tumors, colorectal tumors, skin tumors, breast tumors, pancreatic tumors, cervical tumors, uterine corpus tumors, bladder tumors, gallbladder tumors, renal tumors, laryngeal tumors, lip tumors, oral tumors, esophageal tumors, ovarian tumors, prostate tumors, stomach tumors, testicular tumors, thyroid tumors, or lung tumors.

For purposes of treatment herein, the term "subject" is preferably a subject in need of treatment for a target pathological condition, such as a tumor. For the purpose of prevention, the subject is preferably a subject at risk of or predisposed to developing the target pathological condition. The term "subject" includes living organisms such as prokaryotes and eukaryotes. Examples of subjects include mammals, such as humans, dogs, cows, horses, pigs, sheep, goats, cats, mice, rabbits, rats, and genetically modified non-human animals. In some preferred embodiments, the subject is a human.

As used herein, "treatment" is an approach for obtaining beneficial or desired clinical results. For purposes of this invention, beneficial or desired clinical results include, but are not limited to, one or more of the following: reducing the proliferation of (or destroying) neoplastic or cancerous cells, inhibiting metastasis of neoplastic cells, shrinking or decreasing the size of a tumor, remission of malignant tumor, decreasing symptoms resulting from malignant tumor, increasing the quality of life of those suffering from malignant tumor, decreasing the dose of other medications required to treat malignant tumor, delaying the progression of malignant tumor, curing a malignant tumor, and / or prolong survival of patients having malignant tumor.

As used herein, an "effective amount" or "effective dosage" of bacterium, medicament, or pharmaceutical composition is an amount sufficient to affect any one or more beneficial or desired results. For prophylactic use, beneficial or desired results include eliminating or reducing the risk, lessening the severity, or delaying the outset of the disease, including biochemical, histological and / or behavioral symptoms of the disease, its complications and intermediate pathological phenotypes presenting during development of the disease. For therapeutic use, beneficial or desired results include such as reducing one or more symptoms of a disease such as tumor, decreasing the dose of other medications required to treat the disease, enhancing the effect of another medication, and / or delaying the progression of the cancer in patients. For example, an "effective amount" preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition in vitro by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

As used herein, "pharmaceutically acceptable carrier" includes any material which, when combined with an active ingredient, allows the ingredient to retain biological activity and is non-reactive with the subject's immune system, including but not limited to a disintegrating agent, an adhesive, a filler, a buffer, a tension agent, a stabilizer, an antioxidant, a surfactant or a lubricant. Preferably, the carrier is suitable for intravenous, intramuscular, subcutaneous, parenteral, spinal or epidermal administration (e.g., by injection or infusion). Depending on the route of administration, the bacterium of the present invention may be coated in a material to protect the compound from the action of acids and other natural conditions that may inactivate the bacterium. Pharmaceutically acceptable carriers include normal saline, PBS buffer, sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated.

As used herein, the term "cytotoxin" refers primarily to protein toxins that are toxic to cells, such as exotoxins and endotoxins expressed by bacteria.

### Fusion Protein

The present invention provides a fusion protein comprising an antibody (e.g., a nanobody) that binds to neutrophils or an antigen-binding fragment thereof and a cytotoxin or an active fragment thereof.

Examples of the antibody (nanobody) that binds to neutrophils include, but are not limited to, an antibody that binds to CXCR2 (such as the nanobody of SEQ ID NOs: 19, 20 and 21, or an antibody comprising the CDRs thereof), an antibody that binds to G-CSF-R (such as the nanobodies of SEQ ID NOs: 61 and 62, or an antibody comprising the CDRs thereof), an antibody that binds to EGFR (such as the nanobody of SEQ ID NO: 63, or an antibody comprising the CDRs thereof), an antibody that binds to CXCR4 (such as the nanobodies of SEQ ID NOs: 64 and 65, or an antibody comprising CDRs thereof), an antibody that binds to IL6R (such as the nanobodies of SEQ ID NOs: 66-70, or an antibody comprising the CDRs thereof), and an antibody that binds to human TNFR1 (such as the nanobodies of SEQ ID NOs: 71-78, or an antibody comprising the CDRs thereof).

Examples of the cytotoxin include, but are not limited to, Cholix toxin (e.g., SEQ ID NO: 79), cholera toxin (e.g., SEQ ID NO: 81), heat-sensitive enterotoxin (e.g., subunit A comprising SEQ ID NO: 82 and subunit B comprising SEQ ID NO: 83), diphtheria toxin (DT, e.g., SEQ ID NO: 84), Shiga toxin (e.g., the A chain comprising SEQ ID NO: 86 and the B chain comprising SEQ ID NO: 87), anthrax toxin (e.g., anthrax toxin edema factor of SEQ ID NO: 88, anthrax toxin lethal factor of SEQ ID NO: 89, and anthrax toxin protective antigen of SEQ ID NO: 90), ricin (e.g., ricin A chain of SEQ ID NO: 91), Pseudomonas exotoxin (PE, e.g., Pseudomonas exotoxin A of SEQ ID NO: 25). Active fragments of these toxins are also encompassed in the present invention and are known in the art, such as fragment CET40 of the Cholix toxin (domains II and III of the Cholix toxin, SEQ ID NO: 80), fragment DT390 of the diphtheria toxin (SEQ ID NO: 85), and fragment PE38 and PE [LR] of the Pseudomonas exotoxin (SEQ ID NO: 26 and 27).

In some embodiments, the antibody is an antibody that binds to CXCR2. In some embodiments, the antibody comprises the complementary determining regions (CDRs) selected from the group consisting of CDR1 comprising residues 31-35 of SEQ ID NO: 19, CDR2 comprising residues 50-65 of SEQ ID NO: 19, and CDR3 comprising residues 98-104 of SEQ ID NO: 19; CDR1 comprising residues 31-35 of SEQ ID NO: 20, CDR2 comprising residues 50-67 of SEQ ID NO: 20, and CDR3 comprising residues 100-111 of SEQ ID NO: 20; CDR1 comprising residues 31-35 of SEQ ID NO: 21, CDR2 comprising residues 50-64 of SEQ ID NO: 21, and CDR3 comprising residues 98-115 of SEQ ID NO: 21. In some embodiments, the antibody comprises an amino acid sequence selected from SEQ ID NOs: 19, 20 and 21.

In some embodiments, the cytotoxin is a protein toxin. In some embodiments, the cytotoxin is a toxin capable of killing neutrophils. In some embodiments, the cytotoxin is Pseudomonas exotoxin (PE). In some embodiments, the cytotoxin comprises the amino acid sequence of SEQ ID NO: 25. In some embodiments, the fragment of the cytotoxin comprises the amino acid sequence of SEQ ID NO: 26 or 27.

In some embodiments, the fusion protein further comprises a signal peptide. In some embodiments, the signal peptide comprises the amino acid sequence of SEQ ID NO: 28 or 29.

In some embodiments, the fusion protein further comprises a linker. In some embodiments, the linker is a flexible linker, e.g., a GS linker, such as (GₙS) ₘ, wherein m and n are independently integers of 1-4. In some embodiments, the fusion protein further comprises a tag. In some embodiments, the tag is a His tag (e.g., 6×His) or an HA tag.

In some embodiments, the cytotoxin or the active fragment thereof is fused to the C-terminus of the antibody or the antigen-binding fragment thereof. In some embodiments, the cytotoxin or the active fragment thereof is directly fused to the antibody or the antigen-binding fragment thereof. In some embodiments, the cytotoxin or the active fragment thereof is fused to the antibody or the antigen-binding fragment thereof through a linker.

In some embodiments, the fusion protein comprises SEQ ID NO: 19 and SEQ ID NO: 26 from N-terminus to C-terminus. In some embodiments, the fusion protein comprises SEQ ID NO: 19 and SEQ ID NO: 27 from N-terminus to C-terminus. In some embodiments, the fusion protein comprises SEQ ID NO: 20 and SEQ ID NO: 26 from N-terminus to C-terminus. In some embodiments, the fusion protein comprises SEQ ID NO: 20 and SEQ ID NO: 27 from N-terminus to C-terminus. In some embodiments, the fusion protein comprises SEQ ID NO: 21 and SEQ ID NO: 26 from N-terminus to C-terminus. In some embodiments, the fusion protein comprises SEQ ID NO: 21 and SEQ ID NO: 27 from N-terminus to C-terminus.

In some embodiments, the fusion protein comprises SEQ ID NO: 28, SEQ ID NO: 19, and SEQ ID NO: 26 from N-terminus to C-terminus. In some embodiments, the fusion protein comprises SEQ ID NO: 28, SEQ ID NO: 19, and SEQ ID NO: 27 from N-terminus to C-terminus. In some embodiments, the fusion protein comprises SEQ ID NO: 28, SEQ ID NO: 20, and SEQ ID NO: 26 from N-terminus to C-terminus. In some embodiments, the fusion protein comprises SEQ ID NO: 28, SEQ ID NO: 20, and SEQ ID NO: 27 from N-terminus to C-terminus. In some embodiments, the fusion protein comprises SEQ ID NO: 28, SEQ ID NO: 21, and SEQ ID NO: 26 from N-terminus to C-terminus. In some embodiments, the fusion protein comprises SEQ ID NO: 28, SEQ ID NO: 21, and SEQ ID NO: 27 from N-terminus to C-terminus. In some embodiments, the fusion protein comprises SEQ ID NO: 29, SEQ ID NO: 19, and SEQ ID NO: 26 from N-terminus to C-terminus. In some embodiments, the fusion protein comprises SEQ ID NO: 29, SEQ ID NO: 19, and SEQ ID NO: 27 from N-terminus to C-terminus. In some embodiments, the fusion protein comprises SEQ ID NO: 29, SEQ ID NO: 20, and SEQ ID NO: 26 from N-terminus to C-terminus. In some embodiments, the fusion protein comprises SEQ ID NO: 29, SEQ ID NO: 20, and SEQ ID NO: 27 from N-terminus to C-terminus. In some embodiments, the fusion protein comprises SEQ ID NO: 29, SEQ ID NO: 21, and SEQ ID NO: 26 from N-terminus to C-terminus. In some embodiments, the fusion protein comprises SEQ ID NO: 29, SEQ ID NO: 21, and SEQ ID NO: 27 from N-terminus to C-terminus.

### Polynucleotide and Expression Construct

The present invention provides a polynucleotide encoding the fusion protein of the present invention.

The present invention also provides an expression construct comprising a nucleotide sequence encoding the fusion protein of the present invention operably linked to a strictly hypoxia inducible promoter.

In one embodiment, the strictly hypoxia inducible promoter is selected from pepTp, fnrSp, ysgAp, ssbp1, Hip1, BBa_I14018, BBa_R1074, Ptet-arcA, and Ptet-Fnr. In some embodiments, the strictly hypoxia inducible promoter comprises a nucleotide sequence selected from SEQ ID NOs: 9-18.

The present invention also provides an expression construct comprising a nucleotide sequence encoding an antibody that binds to neutrophils or antigen-binding fragment thereof operably linked to a strictly hypoxia inducible promoter.

Examples of the antibody (nanobody) that binds to neutrophils include, but are not limited to, an antibody that binds to CXCR2 (such as the nanobodies of SEQ ID NOs: 19, 20 and 21, or an antibody comprising the CDRs thereof), an antibody that binds to G-CSF-R (such as the nanobodies of SEQ ID NOs: 61 and 62, or an antibody comprising the CDRs thereof), an antibody that binds to EGFR (such as the nanobody of SEQ ID NO: 63, or an antibody comprising the CDRs thereof), an antibody that binds to CXCR4 (such as the nanobodies of SEQ ID NOs: 64 and 65, or an antibody comprising the CDRs thereof), an antibody that binds to IL6R (such as the nanobodies of SEQ ID NOs: 66-70, or an antibody comprising CDRs thereof), and an antibody that binds to human TNFR1 (such as the nanobodies of SEQ ID NOs: 71-78, or an antibody comprising CDRs thereof).

In some embodiments, the antibody is an antibody that binds to CXCR2. In some embodiments, the antibody comprises the complementary determining regions (CDRs) selected from the group consisting of CDR1 comprising residues 31-35 of SEQ ID NO: 19, CDR2 comprising residues 50-65 of SEQ ID NO: 19, and CDR3 comprising residues 98-104 of SEQ ID NO: 19; CDR1 comprising residues 31-35 of SEQ ID NO: 20, CDR2 comprising residues 50-67 of SEQ ID NO: 20, and CDR3 comprising residues 100-111 of SEQ ID NO: 20; CDR1 comprising residues 31-35 of SEQ ID NO: 21, CDR2 comprising residues 50-64 of SEQ ID NO: 21, and CDR3 comprising residues 98-115 of SEQ ID NO: 21. In some embodiments, the antibody comprises an amino acid sequence selected from SEQ ID NOs: 19, 20 and 21.

In some embodiments, the nucleotide sequence further encodes a signal peptide. In some embodiments, the signal peptide comprises the amino acid sequence of SEQ ID NO: 28 or 29.

In some embodiments, the nucleotide sequence further encodes a tag sequence. In some embodiments, the tag is a His tag (e.g., 6×His) or an HA tag.

The present invention also provides an expression construct comprising a nucleotide sequence encoding a cytotoxin or an active fragment thereof operably linked to a strictly hypoxia inducible promoter.

In some embodiments, the cytotoxin is a protein toxin. In some embodiments, the cytotoxin is a toxin capable of killing neutrophils. In some embodiments, the cytotoxin is capable of killing tumor cells.

Examples of the cytotoxin include, but are not limited to, a Cholix toxin (e.g., SEQ ID NO: 79), a cholera toxin (e.g., SEQ ID NO: 81), a heat-sensitive enterotoxin (e.g., subunit A comprising SEQ ID NO: 82 and subunit B comprising SEQ ID NO: 83), a diphtheria toxin (DT, e.g., SEQ ID NO: 84), a Shiga toxin (e.g., the A chain comprising SEQ ID NO: 86 and the B chain comprising SEQ ID NO: 87), an anthrax toxin (e.g., the anthrax toxin edema factor of SEQ ID NO: 88, the anthrax toxin lethal factor of SEQ ID NO: 89, and the anthrax toxin protective antigen of SEQ ID NO: 90), a ricin (e.g., the ricin A chain of SEQ ID NO: 91), or a Pseudomonas exotoxin (PE, e.g., Pseudomonas exotoxin A of SEQ ID NO: 25). The active fragments of these toxins are also encompassed in the present invention and are known in the art, such as fragment CET40 of the Cholix toxin (domains II and III of the Cholix toxin, SEQ ID NO: 80), fragment DT390 of the diphtheria toxin (SEQ ID NO: 85), and fragment PE38 and PE [LR] of the Pseudomonas exotoxin (SEQ ID NO: 26 and 27).

In some embodiments, the cytotoxin is Pseudomonas exotoxin (PE). In some embodiments, the cytotoxin comprises the amino acid sequence of SEQ ID NO: 25. In some embodiments, the fragment of the cytotoxin comprises the amino acid sequence of SEQ ID NO: 26 or 27.

In some embodiments, the nucleotide sequence further encodes a signal peptide. In some embodiments, the signal peptide comprises the amino acid sequence of SEQ ID NO: 28 or 29.

In some embodiments, the nucleotide sequence further encodes a tag sequence. In some embodiments, the tag is a His tag (e.g., 6×His) or an HA tag.

### Modified bacterium

The present invention provides a modified bacterium, wherein the bacterium comprises the expression construct of the present invention and a hypoxia regulated essential gene expression cassette, as compared to an unmodified starting strain. In one embodiment, the essential gene expression cassette comprises an essential gene of the bacterium under the control of a strictly hypoxia inducible promoter. In one embodiment, the bacterium is deficient in at least one gene involved in or regulating the endogenous anti-oxidative stress response pathway or a functional expression product thereof. In one embodiment, the bacterium is deficient in at least one gene required for survival in macrophages or a functional expression product thereof. In one embodiment, the bacterium expresses a wild-type lipopolysaccharide (LPS).

In one embodiment, the strictly hypoxia inducible promoter is selected from pepTp, fnrSp, ysgAp, ssbp1, Hip1, BBa_I14018, BBa_R1074, Ptet-arcA, and Ptet-Fnr. Preferably, the strictly hypoxia inducible promoter is ssbp1 promoter.

In one embodiment, the expression product of the essential gene is responsible for the synthesis of 2,6-diaminopimelic acid (DAP) in the bacterium. In one embodiment, when cultured under aerobic conditions, the bacterial growth relies on the additional DAP or an analog thereof added to the medium. In one embodiment, the essential gene is selected from dapA, dapB, dapD, dapE, argD, dapF, and any combination thereof. Preferably, the essential gene is selected from dapA and dapE. Preferably, the dapE comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 57. Preferably, the nucleotide sequence of dapE comprises SEQ ID NO: 57. Preferably, the nucleotide sequence of dapE consists of SEQ ID NO: 57.

In one embodiment, the gene involved in or regulating the endogenous anti-oxidative stress response pathway is an HtrA serine protease family gene. In one embodiment, the functional expression product of the gene involved in or regulating the endogenous anti-oxidative stress response pathway is an HtrA serine protease family related protein. In one embodiment, the bacterium is deficient in the activity of HtrA serine protease. In one embodiment, the gene involved in or regulating the endogenous anti-oxidative stress response pathway is htrA. In one embodiment, the bacterium is deficient in htrA. Preferably, the htrA comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 58. Preferably, the nucleotide sequence of htrA comprises SEQ ID NO: 58. Preferably, the nucleotide sequence of htrA consists of SEQ ID NO: 58.

In one embodiment, the essential gene is a gene naturally occurring in the bacterial chromosome. In one embodiment, the native promoter of the essential gene is functionally replaced by the strictly hypoxia inducible promoter. As a result, the expression of the essential gene in the bacterium is completely under the control of the strictly hypoxia inducible promoter.

In one embodiment, the essential gene expression cassette is exogenous. In one embodiment, the essential gene naturally occurring in the bacterial chromosome is deleted or functionally inactivated. As a result, the expression of the essential gene in the bacterium is completely under the control of the strictly hypoxia inducible promoter. In one embodiment, the exogenous essential gene expression cassette is integrated into the bacterial chromosome. In one embodiment, the exogenous essential gene expression cassette is outside the bacterial chromosome. In one embodiment, the exogenous essential gene expression cassette is present in a plasmid carried by the bacterium.

In one embodiment, the modified bacterium further comprise a pH regulated expression cassette. In one embodiment, the modified bacterium comprises a hypoxia regulated essential gene expression cassette and a pH regulated expression cassette, as compared to an unmodified starting strain. In one embodiment, the pH regulated expression cassette comprises a gene encoding a bacteria-derived hemolysin protein under the control of a promoter that is active under acid pH condition. According to some embodiments of the present invention, the promoter that is active under acid pH condition is active in a pH range of about pH 4.0 to 7.0. In some embodiments, the promoter that is active under acid pH condition is also active at pH 6.0 ± 1. In some embodiments, the promoter that is active under acid pH condition is active at pH values below 7.0, 6.9, 6.8, 6.7, 6.6, 6.5, 6.4, 6.3, 6.2, 6.1, 6.0, 5.9, 5.8, 5.7, 5.6, or 5.5, or between any two of these values. In one embodiment, the promoter that is active under acid pH conditions is selected from the group consisting of sseA, ssrA, ssaB, ssaG, ssaM, and ssaR. In one embodiment, the promoter that is active under acid pH condition is sseA. In one embodiment, the bacteria-derived hemolysin protein is a Gram-negative bacterial hemolysin protein. In one embodiment, the gene encoding the gram-negative bacterial hemolysin protein is hlyA or hlyE. In one embodiment, the hlyA or hlyE is derived from Listeria monocytogenes, Vibrio cholerae, or Escherichia coli. Preferably, the hlyA comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 59. Preferably, the nucleotide sequence of hlyA comprises SEQ ID NO: 59. Preferably, the nucleotide sequence of hlyA consists of SEQ ID NO: 59.

In one embodiment, the unmodified starting strain is a facultative anaerobic bacterium. In one embodiment, the bacterium is of Enterobacteriaceae. Preferably, the bacterium is a bacteirum from Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp. Preferably, the bacterium is selected from the group consisting of Escherichia coli, Enterobacter blattae (E. blattae), Enterobacter fergusonii (E. fergusonii), Enterobacter hermannii (E. hermannii), Enterobacter vulneris (E. vulneris), Salmonella enterica (S. enterica), Salmonella bongori (S. bongori), Salmonella typhi (S. typhi), Salmonella choleraesuis (S. choleraesuis), Salmonella typhimurium (S. typhimurium), Shigella dysenteriae (S. dysenteriae), Shigella flexneri (S. flexneri), Shigella boydii (S. boydii), Shigella sonnei (S. sonnei), Klebsiella pneumoniae (K. peumoniae), Klebsiella oxytoca (K. oxytoca), Yersinia pestis (Y. pestis), Yersinia enterocolitica (Y. enterocolitica), Yersinia pseudotuberculosis (Y. pseudotuberculosis), Yersinia aldouae (Y. aldouae), Yersinia bercovieri (Y. bercovieri), Yersinia frederiksenii (Y. frederiksenii), Yersinia intermedia (Y. intermedia), Yersinia kristersenii (Y. kristersenii), Yersinia mollaretti (Y. mollaretti), Yersinia rohdei (Y. rohdei), Yersinia ruckeri (Y. ruckeri), Citrobacter freundii (C. freundii), Citrobacter koseri (C. kaseri), Citrobacter braakii (C. braakii), Enterobacter aerogenes (E. aerogenes), Enterobacter cloacae (E. cloacae), Enterobacter gergoviae (E. gergoviac), Enterobacter sakazakii (E. sakazakii), Enterobacter taylorae (E. tavlorae), Enterobacter amnigenus (E. aminigenus), Enterobacter intermedius (E. intermedius), Enterobacter asburiae (E. asburiac), Enterobacter cancerogenus (E. cancerogenus), Enterobacter dissolvens (E. dissolvens), Enterobacter nimipressuralis (E. nimipressuralis), Serratia marcescens (S. marcescens), Serratia entomophila (S. entomophila), Serratia ficaria (S. ficaria), Serratia fonticola (S. fonticola), Serratia grimesii (S. grimesii), Serratia liquefaciens (S. liquefaciens), Serratia odorifera (S. odorifera), Serratia plymuthica (S. plymuthica), Serratia proteamaculans (S. proteamaculans), Serratia rubidaea (S. rubidaea), Serratia ureilytica (S. ureilytica), Proteus mirabilis (P. mirabilis), Proteus vulgaris (P. vulgaris), Proteus myxofaciens (P. myxofaciens), Proteus penneri (P. penneri), Proteus hauseri (P. hauseri)), Morganella morganii (M. morganii), Providencia alcalifaciens (P. alcalifaciens), Providencia rustigianii (P. rustigianii), Providencia stuartii (P. stuartii), Providencia rettgeri (P. rettgeri), Providencia heimbachae (P. heimbochae), Hafnia alvei (H. alvei), and Pantoea agglomerans (P. agglomerans). Preferably, the bacterium is of Salmonella typhimurium. Preferably, the starting strain is Salmonella typhimurium SL7207.

In one embodiment, when administrated to a subject with a tumor, the bacterium can survive and proliferate in the tumor tissue but is rapidly cleared in normal tissue. In one embodiment, when administrated to a subject with a malignant tumor, the bacterium can induce anti-tumor specific immune response. In one embodiment, when administrated to a subject with a malignant tumor, the bacterium can induce anti-tumor immune memory.

In one embodiment, the bacterium does not express a wild-type flagellin. In one embodiment, the modified bacterium is deficient in the fliC gene.

In one embodiment, the survival level of the bacterium in macrophages ranges from about 50% to about 30% of the survival level of the unmodified starting strain. In one embodiment, the survival level of the bacterium in macrophages ranges from about 30% to about 10% of the survival level of the unmodified starting strain. Preferably, the survival level of the bacterium in macrophages ranges from about 10% to about 1% of the survival level of the unmodified starting strain.

### Pharmaceutical composition

The present invention provides a pharmaceutical composition comprising an effective amount of the modified bacterium of the present invention. In one embodiment, the modified bacterium is a live bacterium.

In one embodiment, the pharmaceutical composition is used for treating a malignant tumor. In one embodiment, the pharmaceutical composition is used for inducing an anti-tumor specific immune response in a subject with a malignant tumor. In one embodiment, the pharmaceutical composition is used for inducing anti-tumor immune memory in a subject with a malignant tumor. In one embodiment, the pharmaceutical composition is used for preventing or treating metastasis or recurrence of a malignant tumor. In one embodiment, the pharmaceutical composition is used for treating a malignant tumor that is resistant to, or has failed to, a prior anti-tumor therapy.

In one embodiment, the malignant tumor is a malignant tumor from the nervous system, respiratory system, digestive system, urinary system, reproductive system, hematopoietic system, lymphatic system, endocrine system, or skin mucosa. In some embodiments, the malignant tumor is a sarcoma or cancer. In one embodiment, the malignant tumor is a solid tumor. In one embodiment, the malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

In one embodiment, the pharmaceutical composition further comprises a pharmaceutically acceptable carrier.

### Treatment Methods and Uses

The present invention provides a method for treating a malignant tumor, comprising administering an effective amount of the modified bacterium of the present invention or the pharmaceutical composition of the present invention to a subject with the malignant tumor.

The present invention provides a method for inducing an anti-tumor specific immune response in a subject with a malignant tumor, comprising administering an effective amount of the modified bacterium of the present invention or the pharmaceutical composition of the present invention to a subject with the malignant tumor.

The present invention provides a method for inducing anti-tumor immune memory in a subject with a malignant tumor, comprising administering an effective amount of the modified bacterium of the present invention or the pharmaceutical composition of the present invention to a subject with the malignant tumor.

The present invention provides a method for preventing or treating metastasis or recurrence of a malignant tumor, comprising administering an effective amount of the modified bacterium of the present invention or the pharmaceutical composition of the present invention to a subject with the malignant tumor.

The present invention provides a method for preventing or treating metastasis or recurrence of a malignant tumor, comprising administering an effective amount of the modified bacterium of the present invention or the pharmaceutical composition of the present invention to a subject with the malignant tumor or at a high risk of metastasis or recurrence of the malignant tumor.

The present invention provides a method for treating a malignant tumor that is resistant to, or has failed to, a prior anti-tumor therapy, comprising administering an effective amount of the modified bacterium of the present invention or the pharmaceutical composition of the present invention to a subject with the malignant tumor that is resistant to, or has failed to, a prior anti-tumor therapy.

The present invention provides use of the modified bacterium of the present invention in the manufacture of a medicament in the treatment of a malignant tumor.

The present invention provides use of the modified bacterium of the present invention in the manufacture of a medicament for inducing an anti-tumor specific immune response in a subject with a malignant tumor.

The present invention provides use of the modified bacterium of the present invention in the manufacture of a medicament for inducing anti-tumor immune memory in a subject with a malignant tumor.

The present invention provides use of the modified bacterium of the present invention in the manufacture of a medicament for preventing or treating metastasis or recurrence of a malignant tumor.

The present invention provides use of the modified bacterium of the present invention in the manufacture of a medicament for treating a malignant tumor that is resistant to, or has failed to, a prior anti-tumor therapy.

In one embodiment, the modified bacterium is a live bacterium.

In one embodiment, the malignant tumor is a malignant tumor from the nervous system, respiratory system, digestive system, urinary system, reproductive system, hematopoietic system, lymphatic system, endocrine system, or skin mucosa. In some embodiments, the malignant tumor is a sarcoma or cancer. In one embodiment, the malignant tumor is a solid tumor. In one embodiment, the malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

In one embodiment, the bacterium, medicament, or pharmaceutical composition of the present invention is administered by intravenous, intratumoral, intramuscular, subcutaneous, intraperitoneal, transcerebral, gastrointestinal, body surface, oral mucosal, transnasal, transrectal, or transvaginal routes. In one embodiment, the bacterium, medicament, or pharmaceutical composition of the present invention may be formulated in a form that is administered by intravenous, intratumoral, intramuscular, subcutaneous, intraperitoneal, transcerebral, gastrointestinal, body surface, oral mucosal, transnasal, transrectal, or transvaginal routes.

In one embodiment, the dosage form of the medicament or pharmaceutical composition of the present invention may, for example, be in the form of a solution, an emulsion, a freeze-dried preparation or suspension; for oral administration, the dosage form may be in the form of tablets or capsules; for intranasal dosage, the dosage form may be in the form of powder, drop or aerosol; for body surface administration, the dosage form may be in the form of an aqueous solution, a suspension, an ointment, a cream or a gel; and for rectal or vaginal administration, the dosage form may be a suppository, an enema, or delivered as part of an endoscopy or colonoscopy procedure. In one embodiment, the medicament or pharmaceutical composition is formulated in the form of a solution, an emulsion, a freeze-dried preparation or suspension; for oral administration, it may be formulated in the form of a tablet or capsule; for intranasal dosage form, it may be formulated in the form of powder, nasal drops, or aerosol; for body surface administration, it may be formulated in the form of an aqueous solution, a suspension, an ointment, a cream, or a gel; and for rectal or vaginal administration, it can be formulated as a suppository, an enema or delivered as part of an endoscopy or colonoscopy procedure. For methods known in the art for preparing dosage forms see, for example, "Remington's Pharmaceutical Sciences" (20th ed.), edited by A. Gennaro, 2000, Mack Publishing Company, Inc. Easton, PA.

In some embodiments, the bacterium, medicament, or pharmaceutical composition of the present invention may be administered at various intervals, including but not limited to: 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 2 days, 3 days, 4 days, 5 days, 6 days, 7 days, 8 days, 9 days, or 10 days, including the range between any two of the listed values, for example: 1 minute to 10 minutes, 1 minute to 30 minutes, 1 minute to 1 hour, 1 minute to 2 hours, 1 minute to 4 hours, 1 minute to 12 hours, 1 minute to 18 hours, 1 minute to 1 day, 10 minutes to 30 minutes, 10 minutes to 1 hour, 10 minutes to 2 hours, 10 minutes to 4 hours, 10 minutes to 12 hours, 10 minutes to 18 hours, 10 minutes to 1 day, 30 minutes to 1 hour, 30 minutes to 2 hours, 30 minutes to 4 hours, 30 minutes to 12 hours, 30 minutes to 18 hours, 30 minutes to 1 day, 30 minutes to 2 days, 1 hour to 2 hours, 1 hour to 4 hours, 1 hour to 12 hours, 1 hour to 18 hours, 1 hour to 1 day, 4 hours to 12 hours, 4 hours to 18 hours, 4 hours to 1 day, 1 hour to 2 days, 1 hour to 3 days, 1 hour to 4 days, 1 hour to 5 days, 1 hour to 7 days, 1 hour to 10 days, 2 hours to 3 days, 2 hours to 4 days, 2 hours to 5 days, 2 hours to 7 days, 2 hours to 10 days, or 5 days to 10 days. In some embodiments, the bacterium, medicament, or pharmaceutical composition is administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. In some embodiments, the bacterium, medicament, or pharmaceutical composition of the present invention may be formulated to be administered at various intervals, including but not limited to: 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 2 days, 3 days, 4 days, 5 days, 6 days, 7 days, 8 days, 9 days, or 10 days, including the range between any two of the listed values, for example: 1 minute to 10 minutes, 1 minute to 30 minutes, 1 minute to 1 hour, 1 minute to 2 hours, 1 minute to 4 hours, 1 minute to 12 hours, 1 minute to 18 hours, 1 minute to 1 day, 10 minutes to 30 minutes, 10 minutes to 1 hour, 10 minutes to 2 hours, 10 minutes to 4 hours, 10 minutes to 12 hours, 10 minutes to 18 hours, 10 minutes to 1 day, 30 minutes to 1 hour, 30 minutes to 2 hours, 30 minutes to 4 hours, 30 minutes to 12 hours, 30 minutes to 18 hours, 30 minutes to 1 day, 30 minutes to 2 days, 1 hour to 2 hours, 1 hour to 4 hours, 1 hour to 12 hours, 1 hour to 18 hours, 1 hour to 1 day, 4 hours to 12 hours, 4 hours to 18 hours, 4 hours to 1 day, 1 hour to 2 days, 1 hour to 3 days, 1 hour to 4 days, 1 hour to 5 days, 1 hour to 7 days, 1 hour to 10 days, 2 hours to 3 days, 2 hours to 4 days, 2 hours to 5 days, 2 hours to 7 days, 2 hours to 10 days, or 5 days to 10 days. In some embodiments, the bacterium, medicament, or pharmaceutical composition is formulated to be administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. The dosage regimen may depend on the pharmacokinetic decay pattern expected to be achieved by the practitioner. Progress in therapy can be monitored by routine techniques and assays. The dosing regimen can change over time.

In some embodiments, the effective amount of bacteria in the medicament or pharmaceutical composition comprises at least about 10⁴ colony forming units (cfu), e.g., at least about 10⁴, 10⁵, 10⁶, 10⁷, 10⁸, 10⁹, 10¹⁰, 10¹¹, 10¹², or 10¹³ cfu, including ranges between any of the listed values, e.g., 10⁴-10⁸cfu, 10⁴-10⁹cfu, 10⁴-10¹⁰cfu, 10⁴-10¹¹cfu, 10⁴-10¹²cfu, 10⁴-10¹²cfu, 10⁵-10⁸cfu, 10⁵-10⁹cfu, 10⁵-10¹⁰cfu, 10⁵-10¹⁰cfu, 10⁵-10¹²cfu, 10⁵-10¹²cfu, 10⁶-10⁸cfu, 10⁶-10⁹cfu, 10⁶-10¹⁰cfu, 10⁶-10¹¹cfu, 10⁶-10¹²cfu, 10⁶-10¹²cfu, 10⁷-10⁸cfu, 10⁷-10⁹cfu, 10⁷-10¹⁰cfu, 10⁵-10¹⁰cfu, 10⁷-10¹²cfw, 10⁷-10¹²cfw, 10⁸-10⁹cfu, 10⁸-10¹⁰cfu, 10⁸-10¹¹cfu, 10⁸-10¹²cfu or 10⁸-10¹²cfu.

In some embodiments, the volume of an effective dose of the bacterium, medicament, or pharmaceutical composition of the present invention is less than or equal to about 3 mL, about 2.5 mL, about 2 mL, about 1.5 mL, about 1 mL, about 0.75 mL, about 0.5 mL, about 0.25 mL, or about 0.1 mL. In some embodiments, the volume of an effective dose of the bacterium, medicament, or pharmaceutical composition is about 20 mL, about 19 mL, about 18 mL, about 17 mL, about 16 mL, about 15 mL, about 14 mL, about 13 mL, about 12 mL, about 11 mL, about 10 mL, about 9 mL, about 8 mL, about 7 mL, about 6 mL, about 5 mL, about 4 mL, about 3 mL, about 2 mL, or about 1 mL. Alternatively, the volume of an effective dose of the bacterium, medicament, or pharmaceutical composition is about 20.5 mL, about 19.5 mL, about 18.5 mL, about 17.5 mL, about 16.5 mL, about 15.5 mL, about 14.5 mL, about 13.5 mL, about 12.5 mL, about 11.5 mL, about 10.5 mL, about 9.5 mL, about 8.5 mL, about 7.5 mL, about 6.5 mL, about 5.5 mL, about 4.5 mL, about 3.5 mL, about 2.5 mL, about 1.5 mL, or about 0.5 mL. In some embodiments, the volume of an effective dose of the bacterial, medicament, or pharmaceutical composition is about 200 mL, about 190 mL, about 180 mL, about 170 mL, about 160 mL, about 150 mL, about 140 mL, about 130 mL, about 120 mL, about 110 mL, about 100 mL, about 90 mL, about 80 mL, about 70 mL, about 60 mL, about 50 mL, about 40 mL, or about 30 mL, as well as the range between any two of the listed values, for example, 100 mL-110 mL, 100 mL-120 mL, 90 mL-120 mL, 90 mL-130 mL. Alternatively, the volume of an effective dose of the bacterium, medicament, or pharmaceutical composition is about 205 mL, about 195 mL, about 185 mL, about 175 mL, about 165 mL, about 155 mL, about 145 mL, about 135 mL, about 125 mL, about 115 mL, about 105 mL, about 95 mL, about 85 mL, about 75 mL, about 65 mL, about 55 mL, about 45 mL, about 35 mL, or about 25 mL, as well as the range between any two of the listed values, for example, 105 mL-115 mL, 105 mL-125 mL, 95 mL-125 mL, 95 mL-135 mL. Alternatively, the volume of an effective dose of the bacterium, medicament or pharmaceutical composition is about 900 microlitres, about 800 microlitres, about 700 microlitres, about 600 microlitres, about 500 microlitres, about 400 microlitres, about 300 microlitres, about 200 microlitres, or about 100 microlitres, optionally about 950 microlitres, about 850 microlitres, about 750 microlitres, about 650 microlitres, about 550 microlitres, about 450 microlitres, about 350 microlitres, about 250 microlitres, about 150 microlitres, or about 50 microlitres. In some embodiments, the volume of an effective dose of the bacterium, medicament, or pharmaceutical composition is less than or equal to about 2.0 mL.

In the medicament or pharmaceutical composition, for example, the concentration of bacterium of the present invention may be at least about 10 ³, 10 ⁴, 10 ⁵, 10 ⁶, 10 ⁷, 10 ⁸, 10⁹, 10 ¹⁰, 10 ¹¹, 10 ¹² or 10 ¹³cfu/ml, including a range between any of the listed values, for example, 10 ³-10 ⁸cfu/ml, 10 ³-10 ⁹cfu/ml, 10 ³-10 ¹⁰cfu/ml , 10 ³-10 ¹¹cfu/ml , 10 ³-10 ¹²cfu/ml, 10³-10 ¹³cfu/ml, 10 ⁴-10 ⁸cfu/ml, 10 ⁴-10 ⁹cfu/ml, 10 ⁴-10 ¹⁰cfu/ml, 10 ⁴-10 ¹¹cfu/ml, 10 ⁴-10 ¹²cfu/ml, 10 ⁴-10 ¹³cfu/ml, 10 ⁵-10 ⁸cfu/ml, 10 ⁵-10 ⁹cfu/ml, 10 ⁵-10 ¹⁰cfu/ml, 10 ⁵-10 ¹¹cfu/ml, 10 ⁵-10 ¹²cfu/ml, 10⁵-10 ¹³cfu/ml, 10 ⁶-10 ⁸cfu/ml, 10 ⁶-10 ⁹cfu/ml, 10 ⁶-10 ¹⁰cfu/ml, 10 ⁶-10 ¹¹cfu/ml, 10 ⁶-10 ¹²cfu/ml, 10 ⁶-10 ¹³cfu/ml, 10 ⁷-10 ⁸cfu/ml, 10 ⁷-10 ⁹cfu/ml, 10 ⁷-10 ¹⁰cfu/ml, 10⁷-10 ¹¹cfu/ml, 10 ⁷-10 ¹²cfu/ml, 10 ⁷-10 ¹³cfu/ml, 10 ⁸-10 ⁹cfu/ml, 10 ⁸-10 ¹⁰cfu/ml, 10 ⁸-10 ¹¹cfu/ml, 10 ⁸-10 ¹²cfu/ml or 10 ⁸-10 ¹³cfu/ml. In the medicament or pharmaceutical composition, for example, the concentration of bacterium of the present invention may be at least about 10³, 10⁴, 10⁵, 10⁶, 10⁷, 10⁸, 10⁹, 10¹⁰, 10¹¹, 10¹² or 10¹³cfu/g, including a range between any of the listed values, for example, 10³-10⁸cfu/g, 10³-10⁹cfu/g, 10³-10¹⁰cfu/g, 10³-10¹¹cfu/g, 10³-10¹²cfu/g, 10³-10¹³cfu/g, 10⁴-10⁸cfu/g, 10⁴-10⁹cfu/g, 10⁴-10¹⁰cfu/g, 10⁴-10¹¹cfu/g, 10⁴-10¹²cfu/g, 10⁴-10¹³cfu/g, 10⁵-10⁸cfu/g, 10⁵-10⁹cfu/g, 10⁵-10¹⁰cfu/g, 10⁵-10¹¹cfu/g, 10⁵-10¹²cfu/g, 10⁵-10¹³cfu/g, 10⁶-10⁸cfu/g, 10⁶-10⁹cfu/g, 10⁶-10¹⁰cfu/g, 10⁶-10¹¹cfu/g, 10⁶-10¹²cfu/g, 10⁶-10¹³cfu/g, 10⁷-10⁸cfu/g, 10⁷-10⁹cfu/g, 10⁷-10¹⁰cfu/g, 10⁷-10¹¹cfu/g, 10⁷-10¹²cfu/g, 10⁷-10¹³cfu/g, 10⁸-10⁹cfu/g, 10⁸-10¹⁰cfu/g, 10⁸-10¹¹cfu/g, 10⁸-10¹²cfu/g or 10⁸-10¹³cfu/g.

Dosage ranges set forth herein are exemplary only and do not limit the dosage ranges that may be selected by medical practitioners. The amount of active compound (e.g., the bacterium of the invention) in the pharmaceutical composition of the present invention may vary according to factors such as the disease state, age, sex, and weight of the individual, including the presence or absence of the malignant tumor, the type and severity of the malignant tumor to be treated, the activity or viability of the bacterium, medicament or pharmaceutical composition, the route of administration, the duration of treatment, the medicament, if any, used in combination with the bacterium, medicament or pharmaceutical composition, the dietary and general health status of the subject, and similar factors well known in the art. Dosage regimens may be adjusted to provide the optimum therapeutic response. For example, a single dose may be administered, several divided doses may be administered over time or the dose may be proportionally reduced or increased as indicated by the exigencies of the therapeutic situation.

The bacterium, medicament or pharmaceutical composition of the present invention may be manufactured by methods well known in the art such as microbial growth in fermenters, followed by concentration and washing by centrifugation, filtration or dialysis, conventional granulating, mixing, dissolving, encapsulating, lyophilizing, or emulsifying processes, among others. The bacterium, medicament or pharmaceutical composition of the present invention may be produced in various forms, including granules, precipitates, or particulates, powders, including freeze dried, rotary dried or spray dried powders, amorphous powders, injections, emulsions, elixirs, suspensions or solutions. Formulations may optionally contain stabilizers, pH modifiers, surfactants, bioavailability modifiers and combinations of these.

In one embodiment, the bacterium, medicament or pharmaceutical composition of the present invention may be administered alone or in combination with other compounds or compositions in the presence of a carrier. In one embodiment, the bacterium, medicament or pharmaceutical composition may be administered in combination with other malignancy therapies, including, but not limited to, radiotherapy, chemotherapy, and surgery. The bacterium, medicament or pharmaceutical composition may be used as an adjuvant in therapies in such cases. Therefore, another aspect of the embodiment relates to a bacterium, medicament, or pharmaceutical composition for use as an adjuvant in a malignancy therapy selected from radiotherapy and chemotherapy.

### Exemplary Embodiments

Embodiment 1: A fusion protein comprising an antibody (e.g., a nanobody) that binds to a neutrophil or antigen-binding fragment thereof and a cytotoxin or active fragment thereof.

Embodiment 2: The fusion protein of embodiment 1, wherein the antibody comprises the complementary determining regions (CDRs) selected from the group consisting of:
CDR1 comprising residues 31-35 of SEQ ID NO: 19, CDR2 comprising residues 50-65 of SEQ ID NO: 19, and CDR3 comprising residues 98-104 of SEQ ID NO: 19;
CDR1 comprising residues 31-35 of SEQ ID NO: 20, CDR2 comprising residues 50-67 of SEQ ID NO: 20, and CDR3 comprising residues 100-111 of SEQ ID NO: 20; and
CDR1 comprising residues 31-35 of SEQ ID NO: 21, CDR2 comprising residues 50-64 of SEQ ID NO: 21, and CDR3 comprising residues 98-115 of SEQ ID NO: 21_{∘}

Embodiment 3: The fusion protein of embodiment 1 or 2, wherein the antibody comprises an amino acid sequence selected from SEQ ID NOs: 19, 20 and 21.

Embodiment 4: The fusion protein of any one of embodiments 1-3, wherein the cytotoxin is a protein toxin capable of killing neutrophils.

Embodiment 5: The fusion protein of any one of embodiments 1-4, wherein the cytotoxin is Pseudomonas exotoxin (PE).

Embodiment 6: The fusion protein of any one of embodiments 1-5, wherein the cytotoxin comprises the amino acid sequence of SEQ ID NO: 25.

Embodiment 7: The fusion protein of any one of embodiments 1-6, wherein the active fragment of the cytotoxin comprises the amino acid sequence of SEQ ID NO: 26 or 27.

Embodiment 8: The fusion protein of any one of embodiments 1-7, wherein the fusion protein further comprises a signal peptide, for example, the signal peptide comprises the amino acid sequence of SEQ ID NO: 28 or 29.

Embodiment 9: The fusion protein of any of embodiments 1-8, wherein the fusion protein further comprises a linker, such as a flexible linker, e.g., a GS linker, such as (GₙS)ₘ, wherein m and n are independently integers of 1-4.

Embodiment 10: The fusion protein of any one of embodiments 1-9, wherein the fusion protein further comprises a tag, such as a His tag (e.g., 6×His) or a HA tag.

Embodiment 11: The fusion protein of any one of embodiments 1-10, wherein the cytotoxin or the active fragment thereof is fused to the C-terminus of the antibody or the antigen-binding fragment thereof.

Embodiment 12: The fusion protein of any one of embodiments 1-11, wherein the cytotoxin or the active fragment thereof is fused to the antibody or the antigen-binding fragment thereof directly, or via a linker.

Embodiment 13: The fusion protein of any one of embodiments 1-12, wherein the fusion protein comprises from the N-terminus to the C-terminus:
SEQ ID NO: 19 and SEQ ID NO: 26,
SEQ ID NO: 19 and SEQ ID NO: 27,
SEQ ID NO: 20 and SEQ ID NO: 26,
SEQ ID NO: 20 and SEQ ID NO: 27,
SEQ ID NO: 21 and SEQ ID NO: 26, or
SEQ ID NO: 21 and SEQ ID NO: 27.

Embodiment 14: The fusion protein of any of embodiments 1-13, wherein the fusion protein comprises from the N-terminus to the C-terminus:
SEQ ID NO: 28, SEQ ID NO: 19, and SEQ ID NO: 26,
SEQ ID NO: 28, SEQ ID NO: 19, and SEQ ID NO: 27,
SEQ ID NO: 28, SEQ ID NO: 20, and SEQ ID NO: 26,
SEQ ID NO: 28, SEQ ID NO: 20, and SEQ ID NO: 27,
SEQ ID NO: 28, SEQ ID NO: 21, and SEQ ID NO: 26,
SEQ ID NO: 28, SEQ ID NO: 21, and SEQ ID NO: 27,
SEQ ID NO: 29, SEQ ID NO: 19, and SEQ ID NO: 26,
SEQ ID NO: 29, SEQ ID NO: 19, and SEQ ID NO: 27,
SEQ ID NO: 29, SEQ ID NO: 20, and SEQ ID NO: 26,
SEQ ID NO: 29, SEQ ID NO: 20, and SEQ ID NO: 27,
SEQ ID NO: 29, SEQ ID NO: 21, and SEQ ID NO: 26, or
SEQ ID NO: 29, SEQ ID NO: 21, and SEQ ID NO: 27.

Embodiment 15: An isolated polynucleotide encoding the nucleotide sequence of the fusion protein of any one of embodiments 1-14.

Embodiment 16: An expression construct comprising the polynucleotide of embodiment 15 operably linked to a strictly hypoxia inducible promoter.

Embodiment 17: The expression construct of embodiment 16, wherein the strictly hypoxia inducible promoter is selected from pepTp, fnrSp, ysgAp, ssbp1, Hip1, BBa_I14018, BBa_R1074, Ptet-arcA, and Ptet-Fnr.

Embodiment 18: The expression construct of embodiment 16 or 17, wherein the hypoxia inducible promoter comprises a nucleotide sequence selected from SEQ ID NOs: 9-18.

Embodiment 19: An expression construct comprising a nucleotide sequence encoding an antibody (e.g., a nanobody) that binds to neutrophils or antigen-binding fragment thereof, or a toxin or active fragment thereof operably linked to a strictly hypoxia inducible promoter.

Embodiment 20: The expression construct of embodiment 19, wherein the antibody comprises the complementary determining regions (CDRs) selected from the group consisting of:
CDR1 comprising residues 31-35 of SEQ ID NO: 19, CDR2 comprising residues 50-65 of SEQ ID NO: 19, and CDR3 comprising residues 98-104 of SEQ ID NO: 19;
CDR1 comprising residues 31-35 of SEQ ID NO: 20, CDR2 comprising residues 50-67 of SEQ ID NO: 20, and CDR3 comprising residues 100-111 of SEQ ID NO: 20; and
CDR1 comprising residues 31-35 of SEQ ID NO: 21, CDR2 comprising residues 50-64 of SEQ ID NO: 21, and CDR3 comprising residues 98-115 of SEQ ID NO: 21_{∘}
the toxin comprises the amino acid sequence of SEQ ID NO: 25; or
the active fragment of the toxin comprises the active fragment of SEQ ID NO: 26 or 27.

Embodiment 21: The expression construct of embodiment 19 or 20, wherein the antibody comprises an amino acid sequence selected from SEQ ID NOs: 19, 20 and 21.

Embodiment 22: The expression construct of any one of embodiments 19-21, wherein the nucleotide sequence further encodes a signal peptide, e.g., wherein the signal peptide comprises the amino acid sequence of SEQ ID NO: 28 or 29.

Embodiment 23: The expression construct of any one of embodiments 19-22, wherein the nucleotide sequence further encodes a tag, such as a His tag (e.g., 6×His) or a HA tag.

Embodiment 24: An expression construct comprising a nucleotide sequence encoding a cytotoxin or an active fragment thereof operably linked to a strictly hypoxia inducible promoter.

Embodiment 25: The expression construct of embodiment 24, wherein the cytotoxin is a protein toxin capable of killing neutrophils.

Embodiment 26: The expression construct of embodiment 24 or 25, wherein the cytotoxin is Pseudomonas exotoxin (PE).

Embodiment 27: The expression construct of any one of embodiments 24-26, wherein the cytotoxin comprises the amino acid sequence of SEQ ID NO: 25.

Embodiment 28: The expression construct of any one of embodiments 24-27, wherein the active fragment of the cytotoxin comprises the amino acid sequence of SEQ ID NO: 26 or 27.

Embodiment 29: The expression construct of any one of embodiments 24-28, wherein the nucleotide sequence further encodes a signal peptide, e.g., wherein the signal peptide comprises the amino acid sequence of SEQ ID NO: 28 or 29.

Embodiment 30: The expression construct of any one of embodiments 24-29, wherein the nucleotide sequence further encodes a tag, such as a His tag (e.g., 6×His) or a HA tag.

### Embodiments A

Embodiment A1: A modified bacterium, wherein the bacterium comprises the expression construct of any one of embodiments 16-30 and a hypoxia-regulatory essential gene expression cassette comprising an essential gene of the bacterium under the control of a strictly hypoxia inducible promoter, and the bacterium is deficient in at least one gene involved in or regulating the endogenous anti-oxidative stress response pathway or a functional expression product thereof, as compared to an unmodified starting strain.

Embodiment A2: The modified bacterium of embodiment A1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment A3: The modified bacterium of embodiment A1, wherein the strictly hypoxia inducible promoter is ssbp1 promoter.

Embodiment A4: The modified bacterium of any one of the preceding embodiments A, wherein the expression product of the essential gene is responsible for synthesis of 2,6-diaminopimelic acid (DAP) in the bacterium, and when cultured under aerobic conditions, the bacterial growth is dependent on the additional DAP or an analog thereof added to the medium.

Embodiment A5: The modified bacterium of embodiment A4, wherein the essential gene is selected from dapA, dapB, dapD, dapE, argD, dapF, and any combination thereof.

Embodiment A6: The modified bacterium of embodiment A4, wherein the essential gene is selected from dapA and dapE.

Embodiment A7: The modified bacterium of any one of the preceding embodiments A, wherein the gene involved in or regulating the endogenous anti-oxidative stress response pathway is an HtrA serine protease family gene.

Embodiment A8: The modified bacterium of any one of the preceding embodiments A, wherein the functional expression product of the gene involved in or regulating the endogenous anti-oxidative stress response pathway is an HtrA serine protease family related protein.

Embodiment A9: The modified bacterium of any one of the preceding embodiments A, wherein the bacterium is deficient in the activity of HtrA serine protease.

Embodiment A10: The modified bacterium of any one of the preceding embodiments A, wherein the gene involved in or regulating the endogenous anti-oxidative stress response pathway is htrA.

Embodiment A11: The modified bacterium of any one of the preceding embodiments A, wherein the bacterium is deficient in htrA.

Embodiment A12: The modified bacterium of any one of embodiments A1-A11, wherein the essential gene is a gene naturally occurring in the bacterial chromosome, and wherein the native promoter of the essential gene is functionally replaced by the strictly hypoxia inducible promoter, whereby the expression of the essential gene in the bacterium is completely under the control of the strictly hypoxia inducible promoter.

Embodiment A13: The modified bacterium of any one of embodiments A1-A11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment A14: The modified bacterium of embodiments A13, wherein the exogenous essential gene expression cassette is integrated into the bacterial chromosome.

Embodiment A15: The modified bacterium of embodiments A13, wherein the exogenous essential gene expression cassette is outside the bacterial chromosome.

Embodiment A16: The modified bacterium of embodiments A15, wherein the exogenous essential gene expression cassette is present in the plasmid carried by the bacterium.

Embodiment A17: The modified bacterium of any one of the preceding embodiments A, which further comprises a pH regulated expression cassette, wherein the pH regulated expression cassette comprises a gene encoding a bacteria-derived hemolysin protein under the control of a promoter that is active under acid pH condition.

Embodiment A18: The modified bacterium of embodiments A17, wherein the bacteria-derived hemolysin protein is a gram-negative bacterial hemolysin protein.

Embodiment A19: The modified bacterium of embodiments A18, wherein the gene encoding the gram-negative bacterial hemolysin protein is hlyA or hlyE.

Embodiment A20: The modified bacterium of embodiments A19, wherein the hlyA or hlyE is derived from Listeria monocytogenes, Vibrio cholerae, or Escherichia coli.

Embodiment A21: The modified bacterium of any one of embodiments A17-A20, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment A22: The modified bacterium of any one of embodiments A17-A21, wherein the promoter that is active under acid pH condition is selected from the group consisting of sseA, ssrA, ssaB, ssaG, ssaM, and ssaR.

Embodiment A23: The modified bacterium of any one of embodiments A17-A21, wherein the promoter that is active under acid pH condition is sseA.

Embodiment A24: The modified bacterium of any one of the preceding embodiments A, wherein the unmodified starting strain is a facultative anaerobic bacterium.

Embodiment A25: The modified bacterium of any one of the preceding embodiments A, wherein the bacterium is a bacterium of Enterobacteriaceae.

Embodiment A26: The modified bacterium of any one of the preceding embodiments A, wherein the bacterium is a bacteirum of Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.

Embodiment A27: The modified bacterium of any one of the preceding embodiments A, wherein the bacterium is selected from the group consisting of Escherichia coli, Enterobacter blattae (E. blattae), Enterobacter fergusonii (E. fergusonii), Enterobacter hermannii (E. hermannii), Enterobacter vulneris (E. vulneris), Salmonella enterica (S. enterica), Salmonella bongori (S. bongori), Salmonella typhi (S. typhi), Salmonella choleraesuis (S. choleraesuis), Salmonella typhimurium (S. typhimurium), Shigella dysenteriae (S. dysenteriae), Shigella flexneri (S. flexneri), Shigella boydii (S. boydii), Shigella sonnei (S. sonnei), Klebsiella pneumoniae (K. peumoniae), Klebsiella oxytoca (K. oxytoca), Yersinia pestis (Y. pestos), Yersinia enterocolitica (Y. enterocolitica), Yersinia pseudotuberculosis (Y. pseudotuberculosis), Yersinia aldouae (Y. aldouae), Yersinia bercovieri (Y. bercovieri), Yersinia frederiksenii (Y. frederiksenii), Yersinia intermedia (Y. intermedia), Yersinia kristersenii (Y. kristersenii), Yersinia mollaretti (Y. mollaretti), Yersinia rohdei (Y. rohdei), Yersinia ruckeri (Y. ruckeri), Citrobacter freundii (C. freundii), Citrobacter koseri (C. kaseri), Citrobacter braakii (C. braakii), Enterobacter aerogenes (E. aerogenes), Enterobacter cloacae (E. cloacae), Enterobacter gergoviae (E. gergoviac), Enterobacter sakazakii (E. sakazakii), Enterobacter taylorae (E. tavlorae), Enterobacter amnigenus (E. aminigenus), Enterobacter intermedius (E. intermedius), Enterobacter asburiae (E. asburiac), Enterobacter cancerogenus (E. cancerogenus), Enterobacter dissolvens (E. dissolvens), Enterobacter nimipressuralis (E. nimipressuralis), Serratia marcescens (S. marcescens), Serratia entomophila (S. entomophila), Serratia ficaria (S. ficaria), Serratia fonticola (S. fonticola), Serratia grimesii (S. grimesii), Serratia liquefaciens (S. liquefaciens), Serratia odorifera (S. odorifera), Serratia plymuthica (S. plymuthica), Serratia proteamaculans (S. proteamaculans), Serratia rubidaea (S. rubidaea), Serratia ureilytica (S. ureilytica), Proteus mirabilis (P. mirabilis), Proteus vulgaris (P. vulgaris), Proteus myxofaciens (P. myxofaciens), Proteus penneri (P. penneri), Proteus hauseri (P. hauseri)), Morganella morganii (M. morganii), Providencia alcalifaciens (P. alcalifaciens), Providencia rustigianii (P. rustigianii), Providencia stuartii (P. stuartii), Providencia rettgeri (P. rettgeri), Providencia heimbachae (P. heimbochae), Hafnia alvei (H. alvei), and Pantoea agglomerans (P. agglomerans).

Embodiment A28: The modified bacterium of any one of the preceding embodiments A, wherein the bacterium is of Salmonella typhimurium.

Embodiment A29: The modified bacterium of embodimentA28, wherein the starting strain is Salmonella typhimurium SL7207.

Embodiment A30: The modified bacterium of any one of the preceding embodiments A, wherein the bacterium expresses a wild-type lipopolysaccharide (LPS).

Embodiment A31: The modified bacterium of any one of the preceding embodiments A, wherein, when administrated to a subject with a tumor, the bacterium is capable of surviving and proliferating in tumor tissue and is rapidly cleared in normal tissue.

Embodiment A32: The modified bacterium of any one of the preceding embodiments A, wherein, when administrated to a subject with a malignant tumor, the bacterium is capable of inhibiting the growth of the malignant tumor.

Embodiment A33: The modified bacterium of any one of the preceding embodiments A, wherein, when administrated to a subject with a malignant tumor, the bacterium is capable of inducing an anti-tumor specific immune response.

Embodiment A34: The modified bacterium of any one of the preceding embodiments A, wherein, when administrated to a subject with a malignant tumor, the bacterium is capable of inducing anti-tumor immune memory.

Embodiment A35: The modified bacterium of any one of the preceding embodiments A, which does not express a wild-type flagellin.

Embodiment A36: The modified bacterium of any one of the preceding embodiments A, which is deficient in the fliC gene.

Embodiment A37: The modified bacterium of any one of the preceding embodiments A, wherein the survival level of the bacterium in macrophages ranges from about 50% to about 30% of the survival level of the unmodified starting strain.

Embodiment A38: The modified bacterium of any one of embodiments A1-A36, wherein the survival level of the bacterium in macrophages ranges from about 30% to about 10% of the survival level of the unmodified starting strain.

Embodiment A39: The modified bacterium of any one of embodiments A1-A36, wherein the survival level of the bacterium in macrophages ranges from about 10% to about 1% of the survival level of the unmodified starting strain.

### Embodiments B

Embodiment B1: A modified bacterium comprising the expression construct of any one of embodiments 16-30, a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

Embodiment B2: The modified bacterium of embodiment B1, wherein the strictly hypoxia inducible promoter is selected from pepTp, fnrSp, ysgAp, ssbp1, Hip1, BBa_I14018, BBa_R1074, Ptet-arcA, and Ptet-Fnr.

Embodiment B3: The modified bacterium of embodiment B1, wherein the strictly hypoxia inducible promoter is ssbp1 promoter.

Embodiment B4: The modified bacterium of any one of the preceding embodiments B, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment B5: The modified bacterium of embodiment B4, wherein the essential gene is selected from dapA, dapB, dapD, dapE, argD, dapF, and any combination thereof.

Embodiment B6: The modified bacterium of embodiment B4, wherein the essential gene is selected from dapA and dapE.

Embodiment B7: The modified bacterium of any one of the preceding embodiments B, wherein the gene involved in or regulating the endogenous anti-oxidative stress response pathway is an HtrA serine protease family gene.

Embodiment B8: The modified bacterium of any one of the preceding embodiments B, wherein the functional expression product of the gene involved in or regulating the endogenous anti-oxidative stress response pathway is an HtrA serine protease family related protein.

Embodiment B9: The modified bacterium of any one of the preceding embodiments B, wherein the bacterium is deficient in the activity of HtrA serine protease.

Embodiment B10: The modified bacterium of any one of the preceding embodiments B, wherein the gene involved in or regulating the endogenous anti-oxidative stress response pathway is htrA.

Embodiment B11: The modified bacterium of any one of the preceding embodiments B, wherein the bacterium is deficient in htrA.

Embodiment B12: The modified bacterium of any one of embodiments B1-B11, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment B13: The modified bacterium of any one of embodiments B1-B11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment B14: The modified bacterium of embodiments B13, wherein the exogenous essential gene expression cassette is integrated into the bacterial chromosome.

Embodiment B15: The modified bacterium of embodiments B13, wherein the exogenous essential gene expression cassette is outside the bacterial chromosome.

Embodiment B16: The modified bacterium of embodiments B15, wherein the exogenous essential gene expression cassette is present in the plasmid carried by the bacterium.

Embodiment B17: The modified bacterium of embodiments any one of the preceding embodiments B, wherein the bacteria-derived hemolysin protein is a gram-negative bacterial hemolysin protein.

Embodiment B18: The modified bacterium of embodiment B17, wherein the gene encoding the gram-negative bacterial hemolysin protein is hlyA or hlyE.

Embodiment B19: The modified bacterium of embodiment B18, wherein the hlyA or hlyE is derived from Listeria monocytogenes, Vibrio cholerae, or Escherichia coli.

Embodiment B20: The modified bacterium of any one of the preceding embodiments B, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment B21: The modified bacterium of any one of the preceding embodiments B, wherein the promoter that is active under acid pH condition is selected from the group consisting of sseA, ssrA, ssaB, ssaG, ssaM, and ssaR.

Embodiment B22: The modified bacterium of any one of the preceding embodiments B, wherein the promoter that is active under acid pH condition is sseA.

Embodiment B23: The modified bacterium of any one of the preceding embodiments B, wherein the unmodified starting strain is a facultative anaerobic bacterium.

Embodiment B24: The modified bacterium of any one of the preceding embodiments B, wherein the bacterium is a bacterium of Enterobacteriaceae.

Embodiment B25: The modified bacterium of any one of embodiments B, wherein the bacterium is a bacteirum of Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.

Embodiment B26: The modified bacterium of any one of the preceding embodiments B, wherein the bacterium is selected from the group consisting of Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei, and Pantoea agglomerans.

Embodiment B27: The modified bacterium of any one of the preceding embodiments B, wherein the bacterium is of Salmonella typhimurium.

Embodiment B28: The modified bacterium of embodiment B27, wherein the starting strain is Salmonella typhimurium SL7207.

Embodiment B29: The modified bacterium of any one of the preceding embodiments B, wherein the bacterium expresses a wild-type lipopolysaccharide (LPS).

Embodiment B30: The modified bacterium of any one of the preceding embodiments B, wherein, when administrated to a subject with a tumor, the bacterium is capable of surviving and proliferating in tumor tissue and is rapidly cleared in normal tissue.

Embodiment B31: The modified bacterium of any one of the preceding embodiments B, wherein, when administrated to a subject with a malignant tumor, the bacterium is capable of inhibiting the growth of the malignant tumor.

Embodiment B32: The modified bacterium of any one of the preceding embodiments B, wherein, when administrated to a subject with a malignant tumor, the bacterium is capable of inducing an anti-tumor specific immune response.

Embodiment B33: The modified bacterium of any one of the preceding embodiments B, wherein, when administrated to a subject with a malignant tumor, the bacterium is capable of inducing anti-tumor immune memory.

Embodiment B34: The modified bacterium of any one of the preceding embodiments B, which does not express a wild-type flagellin.

Embodiment B35: The modified bacterium of any one of the preceding embodiments B, which is deficient in the fliC gene.

Embodiment B36: The modified bacterium of any one of the preceding embodiments B, wherein the survival level of the bacterium in macrophages ranges from about 50% to about 30% of the survival level of the unmodified starting strain.

Embodiment B37: The modified bacterium of any one of embodiments B1-B35, wherein the survival level of the bacterium in macrophages ranges from about 30% to about 10% of the survival level of the unmodified starting strain.

Embodiment B38: The modified bacterium of any one of embodiments B1-B35, wherein the survival level of the bacterium in macrophages ranges from about 10% to about 1% of the survival level of the unmodified starting strain.

### Embodiments C

Embodiment C1: A modified Salmonella typhimurium bacterium, wherein, the bacterium comprises the expression construct of any one of embodiments 16-30, a hypoxia-regulatory essential gene expression cassette comprising an essential gene of the bacterium under the control of a strictly hypoxia-inducible promoter, and a pH-regulatory expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

Embodiment C2: The modified bacterium of embodiment C1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment C3: The modified bacterium of embodiment C1, wherein the strictly hypoxia inducible promoter is ssbp1 promoter.

Embodiment C4: The modified bacterium of any one of the preceding embodiments C, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment C5: The modified bacterium of embodiment C4, wherein the essential gene is selected from dapA, dapB, dapD, dapE, argD, dapF, and any combination thereof.

Embodiment C6: The modified bacterium of embodiment C4, wherein the essential gene is selected from dapA and dapE.

Embodiment C7: The modified bacterium of any one of the preceding embodiments C, wherein the gene involved in or regulating the endogenous anti-oxidative stress response pathway is an HtrA serine protease family gene.

Embodiment C8: The modified bacterium of any one of the preceding embodiments C, wherein the functional expression product of the gene involved in or regulating the endogenous anti-oxidative stress response pathway is an HtrA serine protease family related protein.

Embodiment C9: The modified bacterium of any one of the preceding embodiments C, wherein the bacterium is deficient in the activity of HtrA serine protease.

Embodiment C10: The modified bacterium of any one of the preceding embodiments C, wherein the gene involved in or regulating the endogenous anti-oxidative stress response pathway is htrA.

Embodiment C11: The modified bacterium of any one of the preceding embodiments C, wherein the bacterium is deficient in htrA.

Embodiment C12: The modified bacterium of any one of embodiments C1-C11, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment C13: The modified bacterium of any one of embodiments C1-C11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment C14: The modified bacterium of embodiment C13, wherein the exogenous essential gene expression cassette is integrated into the bacterial chromosome.

Embodiment C15: The modified bacterium of embodiment C13, wherein the exogenous essential gene expression cassette is outside the bacterial chromosome.

Embodiment C16: The modified bacterium of embodiment C15, wherein the exogenous essential gene expression cassette is present in the plasmid carried by the bacterium.

Embodiment C17: The modified bacterium of embodiments any one of the preceding embodiments C, wherein the bacteria-derived hemolysin protein is a gram-negative bacterial hemolysin protein.

Embodiment C18: The modified bacterium of embodiment C17, wherein the gene encoding the gram-negative bacterial hemolysin protein is hlyA or hlyE.

Embodiment C19: The modified bacterium of embodiment C18, wherein the hlyA or hlyE is derived from Listeria monocytogenes, Vibrio cholerae, or Escherichia coli.

Embodiment C20: The modified bacterium of any one of the preceding embodiments C, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment C21: The modified bacterium of any one of the preceding embodiments C, wherein the promoter that is active under acid pH condition is selected from the group consisting of sseA, ssrA, ssaB, ssaG, ssaM, and ssaR.

Embodiment C22: The modified bacterium of any one of the preceding embodiments C, wherein the promoter that is active under acid pH condition is sseA.

Embodiment C23: The modified bacterium of any one of the preceding embodiments C, wherein the unmodified starting strain is a facultative anaerobic bacterium.

Embodiment C24: The modified bacterium of any one of the preceding embodiments C, wherein the starting strain is Salmonella typhimurium SL7207.

Embodiment C25: The modified bacterium of any one of embodiments C1-C24, wherein the bacterium expresses a wild-type lipopolysaccharide (LPS).

Embodiment C26: The modified bacterium of any one of the preceding embodiments C, wherein, when administrated to a subject with a tumor, the bacterium is capable of surviving and proliferating in tumor tissue but is rapidly cleared in normal tissue.

Embodiment C27: The modified bacterium of any one of the preceding embodiments C, wherein, when administrated to a subject with a malignant tumor, the bacterium is capable of inhibiting the growth of the malignant tumor.

Embodiment C28: The modified bacterium of any one of the preceding embodiments C, wherein, when administrated to a subject with a malignant tumor, the bacterium is capable of inducing an anti-tumor specific immune response.

Embodiment C29: The modified bacterium of any one of the preceding embodiments C, wherein, when administrated to a subject with a malignant tumor, the bacterium is capable of inducing anti-tumor immune memory.

Embodiment C30: The modified bacterium of any one of the preceding embodiments C, which does not express a wild-type flagellin.

Embodiment C31: The modified bacterium of any one of the preceding embodiments C, which is deficient in the fliC gene.

Embodiment C32: The modified bacterium of any one of the preceding embodiments C, wherein the survival level of the bacterium in macrophages ranges from about 50% to about 30% of the survival level of the unmodified starting strain.

Embodiment C33: The modified bacterium of any one of embodiments C1-C31, wherein the survival level of the bacterium in macrophages ranges from about 30% to about 10% of the survival level of the unmodified starting strain.

Embodiment C34: The modified bacterium of any one of embodiments C1-C31, wherein the survival level of the bacterium in macrophages ranges from about 10% to about 1% of the survival level of the unmodified starting strain.

### Embodiments D

Embodiment D1: A modified bacterium, wherein the bacterium comprises the expression construct of any one of embodiments 16-30 and a hypoxia-regulatory essential gene expression cassette comprising an essential gene of the bacterium under the control of a strictly hypoxia inducible promoter, and the bacterium is deficient in at least one gene required for survival in macrophages or a functional expression product thereof, as compared to an unmodified starting strain.

Embodiment D2: The modified bacterium of embodiment D1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment D3: The modified bacterium of embodiment D1, wherein the strictly hypoxia inducible promoter is ssbp1 promoter.

Embodiment D4: The modified bacterium of any one of the preceding embodiments D, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment D5: The modified bacterium of embodiment D4, wherein the essential gene is selected from dapA, dapB, dapD, dapE, argD, dapF, and any combination thereof.

Embodiment D6: The modified bacterium of embodiment D4, wherein the essential gene is selected from dapA and dapE.

Embodiment D7: The modified bacterium of any one of the preceding embodiments D, wherein the gene required for survival in macrophages is gene STM3120, STM3119, slyA, sifA, SPI-2, phoP, or htrA.

Embodiment D8: The modified bacterium of any one of the preceding embodiments D, wherein the functional expression product of the gene required for survival in macrophages is an HtrA serine protease family related protein.

Embodiment D9: The modified bacterium of any one of the preceding embodiments D, wherein the bacterium is deficient in the activity of HtrA serine protease.

Embodiment D10: The modified bacterium of any one of the preceding embodiments D, wherein the gene required for survival in macrophages is htrA.

Embodiment D11: The modified bacterium of any one of the preceding embodiments D, wherein the bacterium is deficient in htrA.

Embodiment D12: The modified bacterium of any one of embodiments D1-D11, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment D13: The modified bacterium of any one of embodiments D1-D11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment D14: The modified bacterium of embodiment D13, wherein the exogenous essential gene expression cassette is integrated into the bacterial chromosome.

Embodiment D15: The modified bacterium of embodiment D13, wherein the exogenous essential gene expression cassette is outside the bacterial chromosome.

Embodiment D16: The modified bacterium of embodiments D15, wherein the exogenous essential gene expression cassette is present in the plasmid carried by the bacterium.

Embodiment D17: The modified bacterium of any one of the preceding embodiments D, which further comprises a pH regulated expression cassette, wherein the pH regulated expression cassette comprises a gene encoding a bacteria-derived hemolysin protein under the control of a promoter that is active under acid pH condition.

Embodiment D18: The modified bacterium of embodiments D17, wherein the bacteria-derived hemolysin protein is a gram-negative bacterial hemolysin protein.

Embodiment D19: The modified bacterium of embodiments D18, wherein the gene encoding the gram-negative bacterial hemolysin protein is hlyA or hlyE.

Embodiment D20: The modified bacterium of embodiments D19, wherein the hlyA or hlyE is derived from Listeria monocytogenes, Vibrio cholerae, or Escherichia coli.

Embodiment D21: The modified bacterium of any one of embodiments D17-D20, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment D22: The modified bacterium of any one of embodiments D17-D21, wherein the promoter that is active under acid pH condition is selected from the group consisting of sseA, ssrA, ssaB, ssaG, ssaM, and ssaR.

Embodiment D23: The modified bacterium of any one of embodiments D17-D21, wherein the promoter that is active under acid pH condition is sseA.

Embodiment D24: The modified bacterium of any one of the preceding embodiments D, wherein the unmodified starting strain is a facultative anaerobic bacterium.

Embodiment D25: The modified bacterium of any one of the preceding embodiments D, wherein the bacterium is a bacterium of Enterobacteriaceae.

Embodiment D26: The modified bacterium of any one of the preceding embodiments D, wherein the bacterium is a bacteirum of Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.

Embodiment D27: The modified bacterium of any one of the preceding embodiments D, wherein the bacterium is selected from the group consisting of Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei, and Pantoea agglomerans.

Embodiment D28: The modified bacterium of any one of the preceding embodiments D, wherein the bacterium is of Salmonella typhimurium.

Embodiment D29: The modified bacterium of embodiment D28, wherein the starting strain is Salmonella typhimurium SL7207.

Embodiment D30: The modified bacterium of any one of the preceding embodiments D, wherein the bacterium expresses a wild-type lipopolysaccharide (LPS).

Embodiment D31: The modified bacterium of any one of the preceding embodiments D, wherein, when administrated to a subject with a tumor, the bacterium is capable of surviving and proliferating in tumor tissue and is rapidly cleared in normal tissue.

Embodiment D32: The modified bacterium of any one of the preceding embodiments D, wherein, when administrated to a subject with a malignant tumor, the bacterium is capable of inhibiting the growth of the malignant tumor.

Embodiment D33: The modified bacterium of any one of the preceding embodiments D, wherein, when administrated to a subject with a malignant tumor, the bacterium is capable of inducing an anti-tumor specific immune response.

Embodiment D34: The modified bacterium of any one of the preceding embodiments D, wherein, when administrated to a subject with a malignant tumor, the bacterium is capable of inducing anti-tumor immune memory.

Embodiment D35: The modified bacterium of any one of the preceding embodiments D, which does not express a wild-type flagellin.

Embodiment D36: The modified bacterium of any one of the preceding embodiments D, which is deficient in the fliC gene.

Embodiment D37: The modified bacterium of any one of the preceding embodiments D, wherein the survival level of the bacterium in macrophages ranges from about 50% to about 30% of the survival level of the unmodified starting strain.

Embodiment D38: The modified bacterium of any one of embodiments D1-D36, wherein the survival level of the bacterium in macrophages ranges from about 30% to about 10% of the survival level of the unmodified starting strain.

Embodiment D39: The modified bacterium of any one of embodiments D1-D36, wherein the survival level of the bacterium in macrophages ranges from about 10% to about 1% of the survival level of the unmodified starting strain.

### Embodiments E

Embodiment E1: A modified bacterium, wherein the bacterium comprise the expression construct of any one of embodiments 16-30, a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium expresses a wild-type lipopolysaccharide (LPS) and is deficient in at least one gene required for survival in macrophages or a functional expression product thereof, as compared to an unmodified starting strain.

Embodiment E2: The modified bacterium of embodiment E1, wherein the strictly hypoxia inducible promoter is selected from pepTp, fnrSp, ysgAp, ssbp1, Hip1, BBa_I14018, BBa_R1074, Ptet-arcA, and Ptet-Fnr.

Embodiment E3: The modified bacterium of embodiment E1, wherein the strictly hypoxia inducible promoter is ssbp1 promoter.

Embodiment E4: The modified bacterium of any one of the preceding embodiments E, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment E5: The modified bacterium of embodiment E4, wherein the essential gene is selected from dapA, dapB, dapD, dapE, argD, dapF, and any combination thereof.

Embodiment E6: The modified bacterium of embodiment E4, wherein the essential gene is selected from dapA and dapE.

Embodiment E7: The modified bacterium of any one of the preceding embodiments E, wherein the gene required for survival in macrophages is gene STM3120, STM3119, slyA, sifA, SPI-2, phoP, or htrA.

Embodiment E8: The modified bacterium of any one of the preceding embodiments E, wherein the functional expression product of the gene required for survival in macrophages is an HtrA serine protease family related protein.

Embodiment E9: The modified bacterium of any one of the preceding embodiments E, wherein the bacterium is deficient in the activity of HtrA serine protease.

Embodiment E10: The modified bacterium of any one of the preceding embodiments E, wherein the gene required for survival in macrophages is htrA.

Embodiment E11: The modified bacterium of any one of the preceding embodiments E, wherein the bacterium is deficient in htrA.

Embodiment E12: The modified bacterium of any one of embodiments E1-E11, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment E13: The modified bacterium of any one of embodiments E1-E11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment E14: The modified bacterium of embodiment E13, wherein the exogenous essential gene expression cassette is integrated into the bacterial chromosome.

Embodiment E15: The modified bacterium of embodiment E13, wherein the exogenous essential gene expression cassette is outside the bacterial chromosome.

Embodiment E16: The modified bacterium of embodiments E15, wherein the exogenous essential gene expression cassette is present in the plasmid carried by the bacterium.

Embodiment E17: The modified bacterium of any one of the preceding embodiments E, wherein the bacteria-derived hemolysin protein is a gram-negative bacterial hemolysin protein.

Embodiment E18: The modified bacterium of embodiment E17, wherein the gene encoding the gram-negative bacterial hemolysin protein is hlyA or hlyE.

Embodiment E19: The modified bacterium of embodiment E18, wherein the hlyA or hlyE is derived from Listeria monocytogenes, Vibrio cholerae, or Escherichia coli.

Embodiment E20: The modified bacterium of any one of preceding embodiments E, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment E21: The modified bacterium of any one of embodiments E, wherein the promoter that is active under acid pH condition is selected from the group consisting of sseA, ssrA, ssaB, ssaG, ssaM, and ssaR.

Embodiment E22: The modified bacterium of any one of embodiments E, wherein the promoter that is active under acid pH condition is sseA.

Embodiment E23: The modified bacterium of any one of embodiments E, wherein the unmodified starting strain is a facultative anaerobic bacterium.

Embodiment E24: The modified bacterium of any one of embodiments E, wherein the bacterium is a bacterium of Enterobacteriaceae.

Embodiment E25: The modified bacterium of any one of embodiments E, wherein the bacterium is a bacteirum of Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.

Embodiment E26: The modified bacterium of any one of the preceding embodiments E, wherein the bacterium is selected from the group consisting of Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei, and Pantoea agglomerans.

Embodiment E27: The modified bacterium of any one of the preceding embodiments E, wherein the bacterium is of Salmonella typhimurium.

Embodiment E28: The modified bacterium of embodiment E27, wherein the starting strain is Salmonella typhimurium SL7207.

Embodiment E29: The modified bacterium of any one of the preceding embodiments E, wherein, when administrated to a subject with a tumor, the bacterium is capable of surviving and proliferating in tumor tissue and is rapidly cleared in normal tissue.

Embodiment E30: The modified bacterium of any one of the preceding embodiments E, wherein, when administrated to a subject with a malignant tumor, the bacterium is capable of inhibiting the growth of the malignant tumor.

Embodiment E31: The modified bacterium of any one of the preceding embodiments E, wherein, when administrated to a subject with a malignant tumor, the bacterium is capable of inducing an anti-tumor specific immune response.

Embodiment E32: The modified bacterium of any one of the preceding embodiments E, wherein, when administrated to a subject with a malignant tumor, the bacterium is capable of inducing anti-tumor immune memory.

Embodiment E33: The modified bacterium of any one of the preceding embodiments E, which does not express a wild-type flagellin.

Embodiment E34: The modified bacterium of any one of the preceding embodiments E, which is deficient in the fliC gene.

Embodiment E35: The modified bacterium of any one of the preceding embodiments E, wherein the survival level of the bacterium in macrophages ranges from about 50% to about 30% of the survival level of the unmodified starting strain.

Embodiment E36: The modified bacterium of any one of embodiments E1-E34, wherein the survival level of the bacterium in macrophages ranges from about 30% to about 10% of the survival level of the unmodified starting strain.

Embodiment E37: The modified bacterium of any one of embodiments E1-E34, wherein the survival level of the bacterium in macrophages ranges from about 10% to about 1% of the survival level of the unmodified starting strain.

### Embodiments F

Embodiment F1: A modified Salmonella typhimurium bacterium, wherein the bacterium comprise the expression construct of any one of embodiments 16-30, a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of an promoter that is active under acid pH condition, and the bacterium expresses a wild-type lipopolysaccharide (LPS) and is deficient in at least one gene required for survival in macrophages or a functional expression product, as compared to an unmodified starting strain.

Embodiment F2: The modified bacterium of embodiment F1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment F3: The modified bacterium of embodiment F1, wherein the strictly hypoxia inducible promoter is ssbp1 promoter.

Embodiment F4: The modified bacterium of any one of the preceding embodiments F, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment F5: The modified bacterium of embodiment F4, wherein the essential gene is selected from dapA, dapB, dapD, dapE, argD, dapF, and any combination thereof.

Embodiment F6: The modified bacterium of embodiment F4, wherein the essential gene is selected from dapA and dapE.

Embodiment F7: The modified bacterium of any one of the preceding embodiments F, wherein the gene required for survival in macrophages is gene STM3120, STM3119, slyA, sifA, SPI-2, phoP, or htrA.

Embodiment F8: The modified bacterium of any one of the preceding embodiments F, wherein the gene required for survival in macrophages is the gene involved in or regulating the endogenous anti-oxidative stress response pathway.

Embodiment F9: The modified bacterium of embodiment F8, wherein the gene involved in or regulating the endogenous anti-oxidative stress response pathway is an HtrA serine protease family gene.

Embodiment F10: The modified bacterium of embodiment F9, wherein the functional expression product of the gene involved in or regulating the endogenous anti-oxidative stress response pathway is an HtrA serine protease family related protein.

Embodiment F11: The modified bacterium of embodiment F9, wherein the bacterium is deficient in the activity of HtrA serine protease.

Embodiment F12: The modified bacterium of embodiment F9, wherein the gene involved in or regulating the endogenous anti-oxidative stress response pathway is htrA.

Embodiment F13: The modified bacterium of embodiment F9, wherein the bacterium is deficient in htrA.

Embodiment F14: The modified bacterium of any one of embodiments F1-F13, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment F15: The modified bacterium of any one of embodiments F1-F13, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment F16: The modified bacterium of embodiment F15, wherein the exogenous essential gene expression cassette is integrated into the bacterial chromosome.

Embodiment F17: The modified bacterium of embodiment F15, wherein the exogenous essential gene expression cassette is outside the bacterial chromosome.

Embodiment F18: The modified bacterium of embodiments F17, wherein the exogenous essential gene expression cassette is present in the plasmid carried by the bacterium.

Embodiment F19: The modified bacterium of any one of the preceding embodiments F, wherein the bacteria-derived hemolysin protein is a gram-negative bacterial hemolysin protein.

Embodiment F20: The modified bacterium of embodiment F19, wherein the gene encoding the gram-negative bacterial hemolysin protein is hlyA or hlyE.

Embodiment F21: The modified bacterium of embodiment F20, wherein the hlyA or hlyE is derived from Listeria monocytogenes, Vibrio cholerae, or Escherichia coli.

Embodiment F22: The modified bacterium of any one of the preceding embodiments F, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment F23: The modified bacterium of any one of embodiments F, wherein the promoter that is active under acid pH condition is selected from the group consisting of sseA, ssrA, ssaB, ssaG, ssaM, and ssaR.

Embodiment F24: The modified bacterium of any one of the preceding embodiments F, wherein the promoter that is active under acid pH condition is sseA.

Embodiment F25: The modified bacterium of any one of the preceding embodiments F, wherein the starting strain is Salmonella typhimurium SL7207.

Embodiment F26: The modified bacterium of any one of the preceding embodiments F, wherein, when administrated to a subject with a tumor, the bacterium is capable of surviving and proliferating in tumor tissue and is rapidly cleared in normal tissue.

Embodiment F27: The modified bacterium of any one of the preceding embodiments F, wherein, when administrated to a subject with a malignant tumor, the bacterium is capable of inhibiting the growth of the malignant tumor.

Embodiment F28: The modified bacterium of any one of the preceding embodiments F, wherein, when administrated to a subject with a malignant tumor, the bacterium is capable of inducing an anti-tumor specific immune response.

Embodiment F29: The modified bacterium of any one of the preceding embodiments F, wherein, when administrated to a subject with a malignant tumor, the bacterium is capable of inducing anti-tumor immune memory.

Embodiment F30: The modified bacterium of any one of the preceding embodiments F, which does not express a wild-type flagellin.

Embodiment F31: The modified bacterium of any one of the preceding embodiments F, which is deficient in the fliC gene.

Embodiment F32: The modified bacterium of any one of the preceding embodiments F, wherein the survival level of the bacterium in macrophages ranges from about 50% to about 30% of the survival level of the unmodified starting strain.

Embodiment F33: The modified bacterium of any one of embodiments F1-F31, wherein the survival level of the bacterium in macrophages ranges from about 30% to about 10% of the survival level of the unmodified starting strain.

Embodiment F34: The modified bacterium of any one of embodiments F1-F31, wherein the survival level of the bacterium in macrophages ranges from about 10% to about 1% of the survival level of the unmodified starting strain.

### Embodiments K

Embodiment K1: A pharmaceutical composition comprising an effective amount of the modified bacterium of any one of the preceding embodiments A, B, C, D, E, or F.

Embodiment K2: The pharmaceutical composition of embodiment K1, which is used for treating a malignant tumor.

Embodiment K3: The pharmaceutical composition of embodiment K1, which is used for inducing an anti-tumor specific immune response in a subject with a malignant tumor.

Embodiment K4: The pharmaceutical composition of embodiment K1, which is used for inducing anti-tumor immune memory in a subject with a malignant tumor.

Embodiment K5: The pharmaceutical composition of embodiment K1, which is used for preventing or treating metastasis or recurrence of a malignant tumor.

Embodiment K6: The pharmaceutical composition of embodiment K1, which is used for treating a malignant tumor that is resistant to, or has failed to, a prior anti-tumor therapy.

Embodiment K7: The pharmaceutical composition of embodiments K1-K6, wherein the modified bacterium is a live bacterium.

### Embodiments L

Embodiment L1: A method of treating a malignant tumor comprising administering to a subject with a malignant tumor an effective amount of the modified bacterium of any one of the preceding embodiments A, embodiments B, embodiments C, embodiments D, embodiments E, or embodiments F or the pharmaceutical composition of any one of embodiments G.

Embodiment L2: A method of inducing a specific immune response against tumor in a subject with a malignant tumor comprising administering to the subject an effective amount of the modified bacterium of any one of the preceding embodiments A, embodiments B, embodiments C, embodiments D, embodiments E, or embodiments F or the pharmaceutical composition of any one of embodiments G.

Embodiment L3: A method of inducing immune memory against tumor in a subject with a malignant tumor comprising administering to the subject an effective amount of the modified bacterium of any one of the preceding embodiments A, embodiments B, embodiments C, embodiments D, embodiments E, or embodiments F or the pharmaceutical composition of any one of embodiments G.

Embodiment L4: A method of preventing or treating the metastasis or recurrence of a malignant tumor comprising administering to a subject with a malignant tumor an effective amount of the modified bacteria of any one of the preceding embodiments A, embodiments B, embodiments C, embodiments D, embodiments E, or embodiments F or the pharmaceutical composition of any one of embodiments G.

Embodiment L5: A method for preventing or treating the metastasis or recurrence of a malignant tumor, comprising administering to a subject with or at a high risk of the metastasis or recurrence of the malignant tumor an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L6: A method for treating a malignant tumor that is resistant to, or has failed to, a prior anti-tumor therapy, comprising administering to a subject with the malignant tumor that is resistant to, or has failed to, the prior anti-tumor therapy, an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L7: The method of embodiments L1-L6, wherein the modified bacterium is a live bacterium.

### Embodiments M

Embodiment M1: Use of the modified bacterium of any one of the preceding embodiments A, embodiments B, embodiments C, embodiments D, embodiments E, or embodiments F in the preparation of a medicament for treating a malignant tumor.

Embodiment M2: Use of the modified bacteria of any one of the preceding embodiments A, embodiments B, embodiments C, embodiments D, embodiments E, or embodiments F in the preparation of a medicament for inducing a specific immune response against tumor in a subject with a malignant tumor.

Embodiment M3: Use of the modified bacteria of any of the preceding embodiments A, embodiments B, embodiments C, embodiments D, embodiments E, or embodiments F in the preparation of a medicament for inducing an immune memory against tumor in a subject with a malignant tumor.

Embodiment M4: Use of the modified bacteria of any of the preceding embodiments A, embodiments B, embodiments C, embodiments D, embodiments E, or embodiments F in the preparation of a medicament for preventing or treating the metastasis or recurrence of a malignant tumor.

Embodiment M5: Use of the modified bacteria of any of the preceding embodiments A, embodiments B, embodiments C, embodiments D, embodiments E, or embodiments F in the preparation of a medicament for treating a malignant tumor that is resistant to, or has failed to a prior anti-tumor therapy.

Embodiment M6: The use of embodiments M1-M5, wherein the modified bacterium is a live bacterium.

### Embodiments N

Embodiment N1: The pharmaceutical composition of any one of embodiments K, the method of any one of embodiments L, or the use of any one of embodiments M, wherein the malignant tumor is a malignant tumor from the nervous system, respiratory system, digestive system, urinary system, reproductive system, hematopoietic system, lymphatic system, endocrine system, or skin mucosa.

Embodiment N2: The pharmaceutical composition of any one of embodiments K, the method of any one of embodiments L, or the use of any one of embodiments M, wherein the malignant tumor is a sarcoma or cancer.

Embodiment N3: The pharmaceutical composition of any one of embodiments K, the method of any one of embodiments L, or the use of any one of embodiments M, wherein the malignant tumor is a solid tumor.

Embodiment N4: The pharmaceutical composition of any one of embodiments K, the method of any one of embodiments L, or the use of any one of embodiments M, wherein malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

Embodiment N5: The pharmaceutical composition of any one of embodiments K, the method of any one of embodiments L, or the use of any one of embodiments M, wherein the bacterium, medicament, or pharmaceutical composition of the present invention is administered by intravenous, intratumoral, intramuscular, subcutaneous, intraperitoneal, transcerebral, gastrointestinal, body surface, oral mucosal, transnasal, transrectal, or transvaginal routes.

### Examples

The following Examples are used for illustrating certain specific features and/or embodiments of the invention. These Examples should not be interpreted as limiting the invention to the specific features or embodiments as described.

### Example 1: Construction of Variant Strain of Salmonella typhimurium SL7207 Strain

1.1 The target fragment was obtained by PCR amplification using the PsseA-hlyA-loxp-KnaR-loxp plasmid (SEQ ID NO: 3) and the Pssbp1- dapE-Cm plasmid (SEQ ID NO: 4) as templates. The following primer sequences were used for PsseA-hlyA-loxp-KnaR-loxp: forward primer (SEQ ID NO: 5) and reverse primer (SEQ ID NO: 6); for Pssbp1- dapE-Cm: forward primer (SEQ ID NO: 7) and reverse primer (SEQ ID NO: 8), annealing temperature 55°C. The target fragment 1 is PsseA-hlyA-loxp-KnaR-loxp (SEQ ID NO: 1), and the target fragment 2 is Pssbp1- dapE-Cm (SEQ ID NO: 2).

1.2 The pSim6 plasmid containing a lambda phage Red recombinase (BioVector NTCC Inc.: 3574840) was introduced into a facultative anaerobic Salmonella SL7207 strain (NCBI: ASM1320710v1) to prepare an electrotransformation competent cell SL7207 (pSim6) of the SL7207 strain containing the pSim6 plasmid.

1.3 The target fragment 1 was introduced into SL7207 (pSim6) competent cells by electrotransformation (E=18KV/cm). The dapE gene (which regulates synthesis of diaminopimelic acid essential for cell wall synthesis) in the SL7207 genome was replaced with desired fragment 1 using λ-red homologous recombination. Specifically, the pSim6 plasmid-containing target strain was cultured at 30 ° C; the recombinase expression was conducted at 42 ° C for 15 min; the homologous recombination process was completed by adding homologous arms containing 50 bp away from each upstream and downstream of the target gene locus to the PCR primers under the effect of a homologous recombinase to obtain the strain SL7207 (ΔdapE::PsseA-hlyA-KnaR).

1.4 The Cre plasmid (Gene Bridges: A112) containing the P1 phage Cre recombinase was introduced into the strain SL7207 (ΔdapE:: PsseA-hlyA-KnaR) for recombination to remove kanamycin resistance, thereby obtaining the strain SL7207 (ΔdapE:: PsseA-hlyA).

1.5 The pSim6 plasmid was introduced into SL7207 (ΔdapE:: PsseA-hlyA) strain; SL7207 (ΔdapE:: PsseA-hlyA) (pSim6) electrotransform competent cells were prepared.

1.6 The target fragment 2 was introduced into SL7207 (ΔdapE:: PsseA-hlyA) (pSim6) competent cells by electrotransformation (E=18 KV/cm). The htrA gene (encoding a serine protease) in the SL7207 genome (ΔdapE:: PsseA-hlyA) was replaced with desired fragment 2 using λ-red homologous recombination to obtain the strain DB-ZW1, which was an SL7207 strain (ΔdapE::PsseA-hlyA;ΔhtrA::Pssbp1-dapE-Cm) deficient in htrA gene, containing an hlyA gene under the control of PsseA and a dapE gene under the control of a strictly hypoxia inducible promoter Pssbp1.

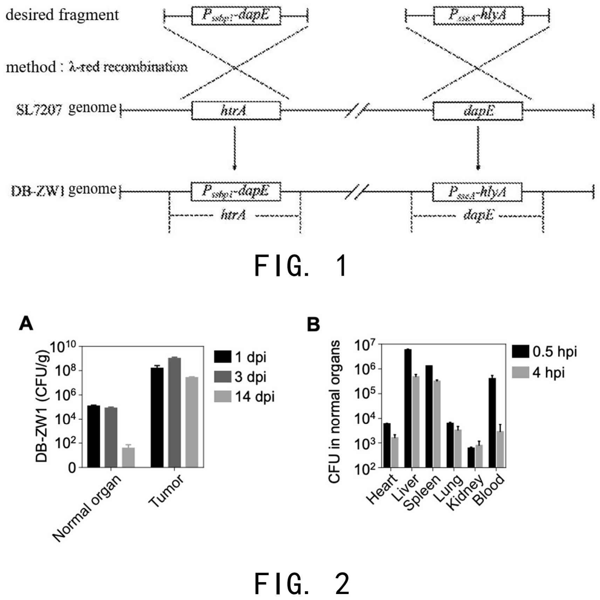

The strain construction scheme was shown in FIG.1.

### Example 2: DB-ZW1 has tumor inhibition effect on various tumor models

### 2.1 Construction of tumor models

C57BL/6 mice (purchased from Beijing Vital River Laboratory Animal Technology Co., Ltd., about 18g in weight, kept in a SPF environment) were subcutaneously inoculated with 1×10 ⁶ MB49 mouse bladder cancer cells/B16 melanoma cells (ATCC: CRL-6475) to establish a mouse subcutaneous tumor model for bladder cancer/melanoma. The experiment was carried out after 14-18 days of inoculation, when the tumor volume was about 100 mm³. In situ colon cancer model was induced by DSS/AOM: 1 week after a single intraperitoneal injection of 10 mg/kg AOM, the mice were fed with water containing 2.5% DSS for 7 days, and then, with normal drinking water for 14 days, which was considered as one cycle of DSS treatment. The DSS treatment was repeated for another 3 cycles to obtain a mouse model with colorectal cancer.

### 2.2 Changes in the distribution of DB-ZW1 in tumors and different organs

1× 10 ⁷ CFU of DB-ZW1 at a volume of 125µL was injected into tumor-bearing mice for MB49 bladder cancer via tail vein, with 3 mice in each group. Approximately 90% of DB-ZW1 in mice was observed to reside in tumors 1 day post injection (1 dpi). The density of DB-ZW1 in tumors was further elevated 100-fold (to 10 ⁸ CFU/g) within the following 2 days and maintained at a high level for the following two weeks. In contrast, the density of DB-ZW1 in normal organs steadily decreased to < 10² CFU/g over two weeks (FIG. 2A).

To further determine whether DB-ZW1 preferentially proliferated in tumors, the number of bacteria distributed in different organs was determined 24 hours after the injection of 125 µL of 1 × 10 ⁷ CFU of DB-ZW1 bacteria into tumor-bearing mice for MB49 bladder cancer via tail vein, with 3 mice in each group (FIG.2B). The results showed that DB-ZW1 spread rapidly in mice within 30 minutes after injection, and most of DB-ZW1 cells (about 99.9%) were distributed in the liver, spleen and blood. And the number of DB-ZW1 cells was significantly reduced within 4 hours after injection, and the total number of DB-ZW1 cells was reduced by about 90%. Subsequently, DB-ZW1 in tumors proliferated exponentially at a growth rate of about 0.7 h⁻¹, reaching saturation within 3 days. In contrast, in normal organs, the number of DB-ZW1 cells were all steadily reduced. This specific tumor growth profile of DB-ZW1 was key to ensuring the validity of the following findings.

### 2.3 The tumor inhibition effect of DB-ZW1 on bladder cancer, melanoma and colon cancer

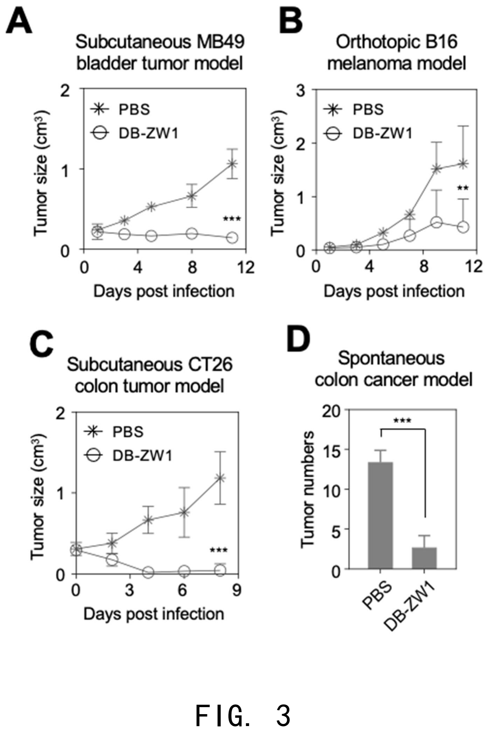

Mice with subcutaneous bladder cancer, orthotopic melanoma and subcutaneous and orthotopic colon cancer were treated with DB-ZW1, and 1×10 ⁷ CFU of DB- ZW1 at a volume of 125µL was injected into tumor-bearing mice via tail vein, with 5 mice per group. As shown in FIGs. 3A-C, DB-ZW1 significantly reduced the tumor size. In addition, the treatment of mice with orthotopic colon cancer using DB-ZW1 also significantly reduced the number of tumors in the colon of mice (FIG. 3D).

Example 3: Construction of Vectors and Strains for Expressing Nanobody/Fusion Protein Binding to CXCR2

### 3.1 Construction of Vector and Bacteria for the Inducible Expression of Nanobody

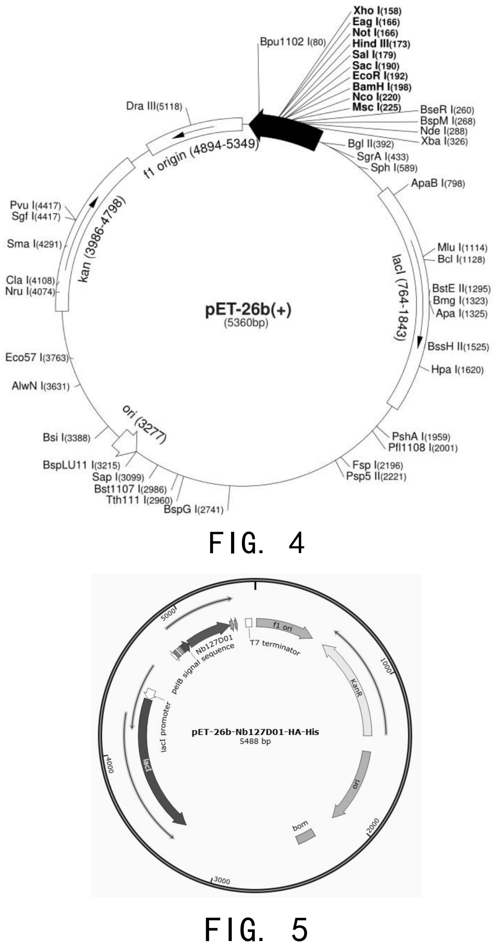

The DNA molecule encoding the fusion of the three polypeptides "Signal Peptide+Nanobody+Tag" was synthesized (Beijing Tsingke Biotech Co., Ltd.) and cloned into the p ET26b plasmid (see FIG. 4), in which the nanobody was selected from the group consisting of Nb127D01, Nb163E3 and Nb97A9 (SEQ ID NO: 19, 20 and 21), the signal peptide is a signal peptide derived from Erwinia crotovora pectin lyase (pelB signal peptide, SEQ ID NO: 28), the tag is a HA-His tag, and the above segments were directly linked. The obtained plasmids were designated as pET26b-pelB-Nb127D01-HA-his (see FIG. 5), pET26b-pelB-Nb163E3-HA-his, pET26b-pelB-Nb97A9-HA-his (SEQ ID NO: 30, 31 and 32), respectively.

In addition, plasmids pET26b-psp-Nb127D01-HA-his, pET26b-psp-Nb163E3-HA-his and pET26b-psp-Nb97A9-HA-his (SEQ ID NO: 34-36) were constructed by replacing the coding sequence of the pelB signal peptide with the coding sequence of the psp signal peptide (SEQ ID NO: 29) derived from Paenibacillus sp. EC003.

The above plasmids were transformed into competent cells of E. coli strain BL21(DE3) (purchased from Novidin) by chemical transformation to obtain strains BL21(DE3)-pET26b-pelB-Nb127D01-HA-his, BL21(DE3)-pET26b-pelB-Nb163E3-HA-his, BL21(DE3)-pET26b-pelB-Nb97A9-HA-his, BL21(DE3)-pET26b-psp-Nb127D01-HA-his, BL21(DE3)-pET26b-psp-Nb163E3-HA-his, and BL21(DE3)-pET26b-psp-Nb97A9-HA-his for the inducible expression of the nanobodies. Specifically, about 100 ng of plasmid was added to competent cells, which was left to stand on ice for 30 min, then placed in a 42°C water bath for 45s, and then placed back on ice for 2-3 min. Then 900 ul LB medium was added, incubated at 37° C for 1 h, and positive clones were screened on LB plates containing 50 mg/L kanamycin.

### 3.2 Construction of Vector for the Anaerobic Expression of Nanobody

To construct the vector for the anaerobic expression of the nanobodies, the oxygen-sensitive promoter FNRsp (SEQ ID NO: 10) was used to drive the expression of the six fusion polypeptides in Examples 3.1.

Specifically, the coding sequence fragments were amplified by designing primers Nb frag F1/R1 and Nb frag F2/R2, respectively, with the plasmid pET26b-pelB-Nb127D01-HA-his, pET26b-pelB-Nb163E3-HA-his, pET26b-pelB-Nb97A9-HA-his, pET26b-pelB-Nb127D01-HA-his, pET26b-pelB-Nb163E3-HA-his, pET26b-pelB-Nb97A9-HA-his constructed in 3.1 as templates. PCR was performed with primers linearize REV/FOR using plasmid pSC101-pFNRsp (SEQ ID NO: 33) as the template to obtain a linear backbone fragment.

### Primer Information

| Name of Primer | Purpose | Sequence |
|---|---|---|
| linearize REV | Amplifying the linear fragment of pSC101-pFNRsp | CCTTCTCCTCTTTAATCTTGCG |
| | | (SEQ ID NO: 37) |
| linearize FOR | Amplifying the linear fragment of pSC101-pFNRsp | CTGGAAGATCCGCGCGTACC |
| | | (SEQ ID NO: 38) |
| Nb frag F1 | Amplifying the linear fragment of pelB-Nb | CGCAAGATTAAAGAGGAGAAGGATGAAATA CCTGCTGCCGAC (SEQ ID NO: 39) |
| Nb frag R1 | Amplifying the linear fragment of pelB-Nb | GGTACGCGCGGATCTTCCAGAGCCGGATCTC AGTGGTGGT (SEQ ID NO: 40) |
| Nb frag F2 | Amplifying the linear fragment of psp-Nb | CCTGCCGTTCGACGATATGGGCCTGAAAATG AAAAAACG (SEQ ID NO: 41) |
| Nb frag R2 | Amplifying the linear fragment of psp-Nb | TGAGGAGACGGTGACCTG |
| | | (SEQ ID NO: 42) |

PCR amplification was performed using PrimeSTAR Max DNA Polymerase (Takara # R054A), and the PCR system (total volume of 50µL) contained 25µL of PrimeSTAR Max Premix, 0.2-0.3µM of each primer, and 200 ng of template. The PCR condition was: denaturation: 98° C 10 sec, annealing: 55° C 10 sec, extension: 72° C 5 sec/kb, 30 cycles of denaturation-anneal-extension in total.

The backbone fragments and the coding sequence fragments were subjected to homologous recombination with a Vazyme one-step cloning kit (ClonExpress ∥ One Step Cloning Kit, Vazyme # C112) to construct plasmids pSC101-pFRRsp-pelB-Nb127D01-HAhis, pSC101-pFRRsp-pelB-Nb163E3-HA-his, pSC101-pFRRsp-pelB-Nb97A9-HA-his, pSC101-pFRRsp-ps-Nb127D01-HA-his, p SC101-pFRRsp-ps-Nb163E3-HA-his and pSC101-pFRRsp-ps-Nb97A9-HA-his for the anaerobic expression of the nanobodies.

### 3.3 Construction of Vector and Bacterium for the Inducible Expression of Nanobody-PE Fusion Protein

Nucleotide fragments encoding Pseudomonas exotoxin fragments PE38 (SEQ ID NO: 26, 55 kDa) and PE [LR] (SEQ ID NO: 27, 43 kDa) were inserted into plasmids pET26b-pelB-Nb127D01-HA-his, pET26b-pelB-Nb163E3-HA-his, pET26b-pelB-Nb97A9-HA-his (at the 3' end of the coding sequence for nanobody) constructed in 3.1 to obtain plasmids pET26b-psp-Nb127D01-PE38-HA-his, pET26b-psp-Nb163E3-PE38-HA-his, pET26b-psp-Nb97A9-PE38-HA-his, pET26b-psp-Nb127D01-PE[LR]-HA-his, pET26b-psp-Nb163E3-PE[LR]-HA-his, pET26b-psp-Nb97A9-PE[LR]-HA-his (SEQ ID NO: 43-48) for the inducible expression of the nanobody-PE fusion proteins. The above constructed plasmids were transformed into competent cells of E. coli strain BL21(DE3) by chemical transformation (see Example 3.1) to obtain strain BL21(DE3)-pET26b-psp-Nb127D01-PE38-HA-his, BL21(DE3)-pET26b-psp-Nb163E3-PE38-HA-his, BL21(DE3)-pET26b-psp-Nb97A9-PE38-HA-his, BL21(DE3)-pET26b-psp-Nb127D01-PE[LR]-HA-his, BL21(DE3)-pET26b-psp-Nb163E3-PE[LR]-HA-his, BL21(DE3)-pET26b-psp-Nb97A9-PE[LR]-HA-his.

Strains for the expression of PE38 and PE [LR], BL21(DE3)-pET26b-psp-PE38-HA-his and BL21(DE3)-pET26b-psp-PE[LR]-HA-his, were constructed in a similar way.

### 3.4 Construction of Vector for the Anaerobic Expression of Nanobody-PE Fusion Protein

Plasmids pET26b-psp-Nb127D01-PE38-HA-his, pET26b-psp-Nb163E3-PE38-HA-his, pET26b-psp-Nb97A9-PE38-HA-his, pET26b-psp-Nb127D01-PE[LR]-HA-his, pET26b-psp-Nb163E3-PE[LR]-HA-his, pET26b-psp-Nb97A9-PE[LR]-HA-his constructed in Examples 3.3 were used as templates, and PCR amplification was performed with primers Fragment.FOR/REV (SEQ ID NOs: 55 and 56) to obtain coding sequence fragments (see Example 3.2 for the PCR system and conditions). The coding sequence fragments were subjected to homologous recombination with the backbone fragments prepared in Example 3.2 by the method described in Example 3.2 to obtain plasmids pSC101-pFNRsp-psp-Nb127D01-PE38-HA-his, pSC101-pFNRsp-psp-Nb163E3-PE38-HA-his, pSC101-pFNRsp-psp-Nb97A9-PE38-HA-his, pSC101-pFNRsp-psp-Nb127D01-PE[LR]-HA-his, pSC101-pFNRsp-psp-Nb163E3-PE[LR]-HA-his and pSC101-pFNRsp-psp-Nb97A9-PE[LR]-HA-his (SEQ ID NO: 49-54).

The above plasmids were transformed into competent cells of the Salmonella typhimurium strain ZW1 prepared in Example 1 by electrotransformation (see Example 1 for the electrotransformation method, the screening medium was LB+chloramphenicol 25 mg/L) to obtain strains ZW1-psc101-pFNRsp-psp-Nb127D01-PE38-HA-his, ZW1-psc101-pFNRsp-psp-Nb163E3-PE38-HA-his, ZW1-psc101-pFNRsp-psp-Nb97A9-PE38-HA-his, ZW1-psc101-pFNRsp-psp-Nb127D01-PE[LR]-HA-his, ZW1-psc101-pFNRsp-psp-Nb163E3-PE[LR]-HA-his, ZW1-psc101-pFNRsp-psp-Nb97A9-PE[LR]-HA-his, ZW1-psc101-pFNRsp-psp-PE38-HA-his and ZW1-psc101-pFNRsp-psp-PE[LR]-HA-his.

Strains ZW1-psc101-pFNRsp-psp-PE38-HA-his and ZW1-psc101-pFNRsp-psp-PE[LR]-HA-his for the expression of PE38 and PE[LR] were constructed in a similar way.

### Example 4: Inducible Expression of Nanobodies and Fusion Proteins with Bacteria

To detect the inducible expression of nanobodies and fusion proteins, the prokaryotic expression was performed with the following strains constructed in Examples 3.1 and 3.3: BL21(DE3)-pET26b-pelB-Nb127D01-HA-his, BL21(DE3)-pET26b-pelB-Nb163E3-HA-his, BL21(DE3)-pET26b-pelB-Nb97A9-HA-his, BL21(DE3)-pET26b-psp-Nb127D01-PE38-HA-his, BL21(DE3)-pET26b-psp-Nb163E3-PE38-HA-his, BL21(DE3)-pET26b-psp-Nb97A9-PE38-HA-his, BL21(DE3)-pET26b-psp-Nb127D01-PE[LR]-HA-his, BL21(DE3)-pET26b-psp-Nb163E3-PE[LR]-HA-hisBL21(DE3)-pET26b-psp-Nb97A9-PE[LR]-HA-his, and the strains expressing PE[LR] and PE38 were used as controls.

Specifically, the bacterium was inoculated into sugar free LB medium (containing kanamycin 50 mg/L) and cultured in a 37°C shaker at 220 rpm until OD600 was between 0.4-0.6. IPTG was added to the culture medium at different concentrations (final concentrations of 0 mM, 0.1 mM, 0.2 mM and 0.5 mM), which was then induced on a 16° C shaker at 220 rpm overnight, and the bacterial culture was collected. The collected bacterial culture was centrifuged, and LB medium supernatant and bacterial precipitate were collected separately. The LB medium supernatant was concentrated by centrifugation with a 3 KDa ultrafiltration tube (2,500 rpm, 2-3 h). The bacterial precipitate was lysed with a protein extraction kit from Qiagen (Qiagen, Qproteome Bacterial Protein Prep Kit # 37900, by reference to the experimental manual Qproteome Bacterial Protein Preparation Handbook, www.qiagen.com). The lysate was centrifuged and the lysed pellet and lysate supernatant were collected separately.

In addition, the strains prepared in Example 3.4 were tested on the expression under anaerobic expression condition. Similar to the above method, but the antibiotic added in the culture medium was 25 mg/L chloramphenicol, the culturing and induced expression were performed under anaerobic condition.

The sample was subjected to electrophoresis (80V) with a polyacrylamide gel of 12.5%, a Pyxis Transfer Stack kit was used for membrane transfer according to the manufacturer's instructions (purchased from Pyxis Company, catalog number SPJ- T20S), a rabbit anti-6×His antibody was used as the primary antibody (purchased from Thermo Fisher, dilution ratio 1:10000), a goat anti-rabbit antibody was used as the secondary antibody (purchased from Thermo Fisher, 1:5000), and the expression and secretion of protein by the bacterium were detected by Western bloting (developed with SuperSignalTM West Pico PLUS Chemiluminecent purchased from Thermo Fisher).

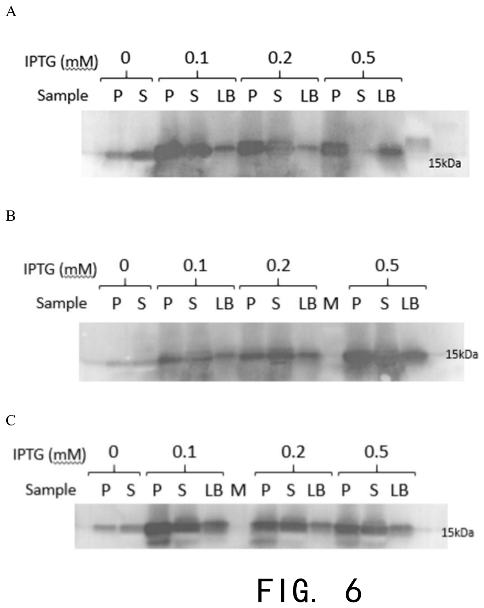

As shown in FIGS. 6A-6C, the expression of nanobodies is positively correlated with the IPTG concentration, and there were distinct bands of nanobodies in the LB supernatant, indicating that the signal peptide can direct the secretion of the expressed nanobodies out of bacterial cells.

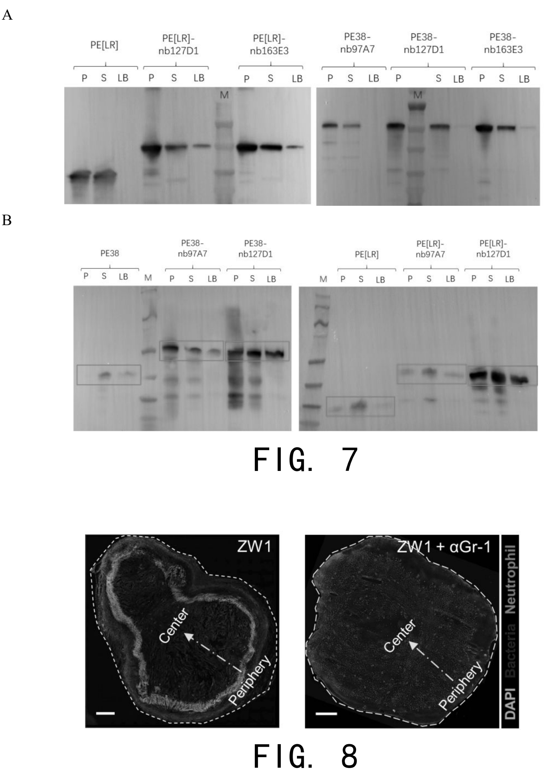

As shown in FIG. 7, during IPTG induction and hypoxia induced expression, there was an significant band for the nanobody-PE fusion protein in the LB supernatant, indicating that the signal peptide can direct the expressed nanobody-PE fusion protein to be secreted outside of the bacterial cells.

### Example 5: Neutralizing Neutrophils in Tumor Increased DB- ZW1 Level in Tumors of MB49 Bladder Cancer

C57BL/6 mice (purchased from Beijing Vital River Laboratory Animal Technology Co., Ltd., weighing about 18 g, and raised in an SPF environment) were subcutaneously inoculated with 1×10 ⁶ MB49 mouse bladder cancer cells (Merck SCC148) to establish a mouse bladder cancer subcutaneous tumor model. After 14-18 days of inoculation (tumor volume about 100 mm³), the mice were divided into PBS group, ZW1 treatment group (only DB-ZW1 was administered, 1×10 ⁷ cfu of bacteria was suspended in 125µL of PBS and administered to the mice through tail vein injection) and ZW1+αGr-1 group, three mice in each group, and the ZW1+ αGr-1 group was injected with 0.2 mg of InVivoMab anti-mouse Ly6G/Ly6c (Gr-1) antibody (purchased from BioXCell) through the tail vein of each mouse 3 days before the bacterial treatment, on the day of bacterial injection and on the next day (2 dpi) after bacterial injection. On Day 3 after the bacterial treatment (3 dpi), the mice were sacrificed, the tumors were taken out and embedded, and continuously sectioned near the center of the tumor and the sections were placed on slides. The samples were fixed for 10 min in acetone, dried in air and then washed twice with 0.01 mol/L PBS at room temperature, and blocked at 4° C for 45 min upon dropping goat serum (Normal Goat Serum purchased from Jackson ImmunoResearch) thereon. Upon the incubation with the primary antibody (the antibody for labeling bacteria pAb anti-Salmonella was purchased from Novus, diluted for 200 folds, the antibody for labeling neutrophil Purified anti-mouse Ly-6G Antibody was purchased from Biolegend, 1:500) in the dark at room temperature for 2 h, washing for 5 times with PBS, the incubation with the secondary antibody (the secondary antibody for labeling bacterium Goat Anti-Rabbit H L & 1:500; the secondary antibody for labeling neutrophil Donkey pAb to Rat IgG, 1:1000 were all purchased from Abcam) in the dark at room temperature for 1 h, and washing for 5 times with PBS, the antifade mounting agent containing DAPI (ProLongTM Gold antifade reagent with DAPI, purchased from Invitrogen) was dropped thereon. The observation was performed with fluorescence microscope upon covering the coverslip. The lipopolysaccharide O antigen and flagellin H antigen on the bacterial surface were labeled with the antibody bearing the AF555 fluorescent group to showed red fluorescence, the lymphocyte membrane surface complex Ly-6G of the neutrophil was labeled with the antibody bearing the AF488 fluorescent group to showed green fluorescence, and the nuclei stained by DAPI showed blue fluorescence. As shown in FIG. 8, after the neutrophils were cleared by αGr-1, it was beneficial for the diffusion of bacterium to the whole tumor area.

### Example 6: Neutralizing Neutrophils Improved the Therapeutic Effect of Artificial Anti-tumor Bacterium on 4T1 Breast Cancer Model

Orthotopic 4T1 breast cancer model was constructed by injecting the PBS suspension of 4T1 breast cancer cells (ATCC CRL- 2539) at the second pair of breast fat pads on the right side of Balb/c mice (purchased from Beijing Vital River Laboratory Animal Technology Co., Ltd., SPF grade). Each of the mice was injected with 10 ⁶ tumor cells/125µL. The tumor volume was about 200 mm³ 20 days post-injection. 125µL of InVivoMab anti-mouse Ly6G/Ly6c (Gr-1) antibody at 1.6 mg/mL was injected via the tail vein to neutralize neutrophils. 24 hours after antibody injection, the mouse tumors and blood were taken for flow cytometry analysis, and 10⁷ CFU DB-ZW1 was administered to mice by tail vein injection, and mice injected with PBS, antibody only and DB-ZW1 only were used as controls.

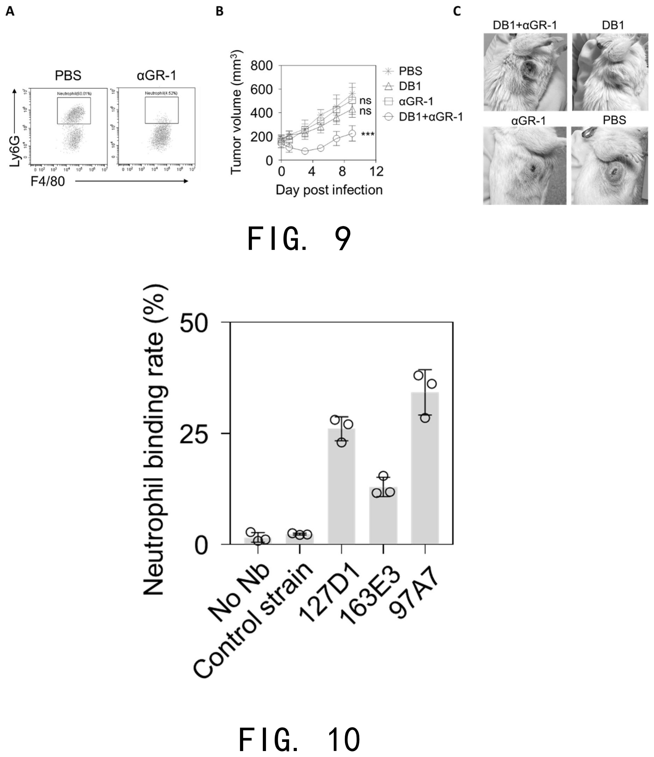

As shown in FIG. 9A, after antibody injection, more than 90% neutrophils in 4T1 tumors were cleared. As shown in FIG. 9B, the growth of 4T1 tumors was not inhibited by injecting Ly6G/Ly6c (Gr-1) antibody only or injecting DB-ZW1 only, while 4T1 tumors in mice injected with antibody and DB-ZW1 were significantly smaller, and there was a significant difference relative to the three control groups (P < 0.01). As shown in FIG. 9C, day 1 after the DB-ZW1 injection, the tumors in the mice of the experimental group showed redness and blackening, and significant scabs were formed. These results indicate that the reduction of the neutrophil content in tumors can improve the effect of the anti-tumor bacterium (DB-ZW1) on inhibiting tumor.

### Example 7: In Vitro Test of Nanobodies Expressed by Bacterium

In order to identify the binding of nanobody expressed by E. coli to neutrophils, primary neutrophils were isolated from blood of C57BL/6 mice and the binding of the expressed nanobody to CXCR2 on the surface of neutrophils was detected.

Specifically, blood was obtained from mice by retro-orbital blood sampling, red blood cell lysing solution (containing 150 mM NH₄Cl, 10mM KHCO₃ and 0.1 mM Na₂EDTA, pH 7.2-7.4) was added to the obtained blood, incubated at room temperature for 5 min to fully lyse red blood cells, and red blood cell fragments were removed by filtration to obtain purified immune cells. Purified immune cells were incubated with Zombie Aqua (purchased from Biolegend) and TruStain FcX (anti-mouse CD16/32) (purchased from Biolegend, Cat. No. 101320) in dark for 15 min at room temperature for staining to count live and dead cells and block Fc receptor, preventing the generation of non-specific signal. Then, the purified nanobody (purified by AKATA protein purifier against his-tag by a nickel column) was incubated with the purified immune cells for 1 hour, and the cells without adding nanobody were employed as control. Co-staining for CD45 (Alexa Fluor 700 anti-mouse CD45, Biolegend), His tag (PE anti-His Tag, Biolegend) and Ly6G (anti-mouse, Biolegend) were performed by flow cytometry, and the sorting purity of neutrophils and the binding of nanobody to neutrophils were determined upon counting by flow cytometry.

As shown in FIG. 10, all of the three nanobodies expressed by E. coli can bind to neutrophils, among which the binding rate of Nb97A7 was the highest, exceeding 25%.

### Example 8: Effect of DB-ZW1 Expressing Nanobodies and Fusion Proteins on Inhibiting Tumor in Tumor-Bearing Mouse Model

In this example, the strains constructed in Examples 3.4 were used: ZW1-psc101-pFNRsp- -psp-Nb127D01-PE38-HA-his, ZW1-psc101-pFNRsp-psp-Nb163E3-PE38-HA-his, ZW1-psc101-pFNRsp-psp-Nb97A9-PE38-HA-his, ZW1-psc101-pFNRsp-psp-Nb127D01-PE[LR]-HA-his, ZW1-psc101-pFNRsp-psp-Nb163E3-PE[LR]-HA-his, ZW1-psc101-pFNRsp-psp-Nb97A9-PE[LR]-HA-his, ZW1-psc101-pFNRsp-psp-PE38-HA-his, ZW1-psc101-pFNRsp-psp-PE[LR]-HA-his, ZW1-psc101-pFnr-SP-psp-Nb127D01-CM, ZW1-psc101-pFnr-SP-psp-Nb163E3-CM, ZW1-psc101-pFnr-SP-psp-Nb97A9-CM to treat MB49 subcutaneous tumor mouse model prepared in Example 5.

Mice injected with the above bacterial strains expressing nanobodies or fusion proteins were used as experimental groups, mice injected with the DB-ZW1 strain were used as control, mice injected with PBS were used as blank control, and mice injected with Gr-1 antibodies after injecting DB-ZW1 were used as positive controls. 1×10 ⁷ cfu of bacteria was suspended in 125µL of PBS, administered to mice via tail vein injection, and the body weight of mice was recorded and the tumor volume was measured every two days. All mice had comparable body weight, indicating the safety of the treatment with the anti-tumor bacteria of the present invention.

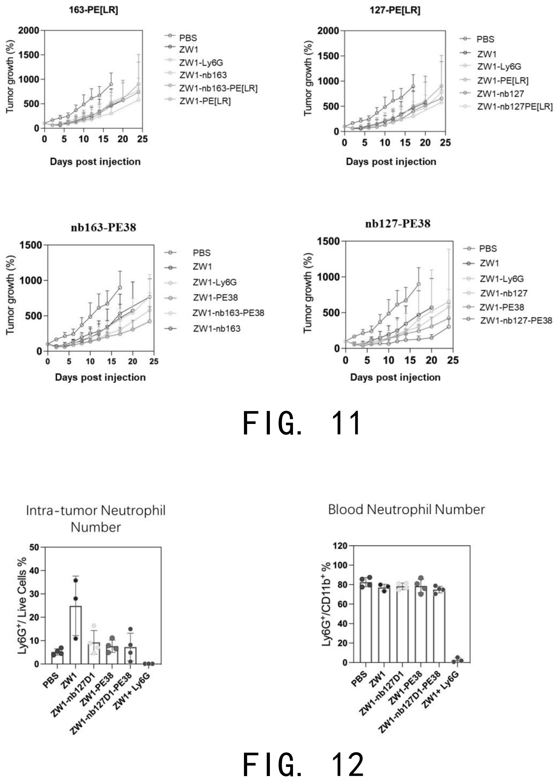

As shown in FIG. 11, ZW1-psc101-pFNRsp-psp-Nb127D01-PE38-HA-his showed the best therapeutic effect, which significantly improved the inhibition to tumor growth compared with DB-ZW1.

Meanwhile, the blood and solid tumors of mice were collected on day 4 after injection, and the blood and intratumoral neutrophils were counted by flow cytometry.

As shown in FIG. 12, compared with the mice injected with PBS, the intratumoral neutrophils in the mice injected with DB-ZW1 were significantly increased, the intratumoral neutrophils in the positive control mice were significantly decreased, and the intratumoral neutrophil levels in the mice in other groups were equivalent to those in the mice injected with PBS. In addition, except that the neutrophil level in the blood of the positive control group was significantly lower (almost completely eliminated), the neutrophil levels in the blood of the other groups were comparable. It was shown that the strain of the present invention can position-specifically inhibit neutrophils in tumors.

### Example 9: Plasmid for the IPTG Induced Expression of Nanobodies and Nanobody Fusion Toxin Protein

### 9.1 Construction of Plasmids for the IPTG Induced Expression of Nanobodies or Toxin Proteins

The purpose of this example was to construct plasmids for expressing nanobodies 238D2 (SEQ ID NO: 64), 238D4 (SEQ ID NO: 65), EGa1 (SEQ ID NO: 63), VHH1 (SEQ ID NO: 61) and VHH26 (SEQ ID NO: 62), and toxin proteins CET40, DT390 (SEQ ID NO: 80 and 85).

The coding sequence was synthesized (Beijing Tsingke Biotech Co., Ltd.) and cloned into the pET26 backbone using the same pelB signal peptide as in Example 3.1, a 6*his tag for nanobody 238D2, 238D4, EGa1 and VHH1, and the same HA-his tag as in Example 3.1 for nanobody VHH26 and toxin proteins CET40 and DT390.

The obtained recombinant plasmids were designated as follows:
pET26b-Kana-T7-pelB-238D2-his (SEQ ID NO: 92);
pET26b-Kana-T7-pelB-238D4-his (SEQ ID NO: 93);
pET26b-Kana-T7-pelB-EGa1-his (SEQ ID NO: 94);
pET26b-Kana-T7-pelB-VHH1-his (SEQ ID NO: 95);
pET26b-Kana-T7-pelB-VHH26-HA-his (SEQ ID NO: 96);
pET26b-Kana-T7-pelB-CET40-HA-his (SEQ ID NO: 97);
pET26b-Kana-T7-pelB-DT390-HA-his (SEQ ID NO: 98).

### 9.2 Construction of Plasmids for Expressing Fusion Protein of Nanobody and Toxin PE38 Under IPTG Induction

The purpose of this example was to construct plasmids for expressing nanobodies 238D2, 238D4, EGa1, VHH1 and VHH26 fused to toxin proteins.

The coding sequence of toxin protein PE38 was inserted into the nanobody-expressing plasmids constructed in Example 9.1 as described in Example 3.3. The obtained recombinant plasmids were designated as follows:
pET26b-Kana-T7-pelB-Nb238D2-PE38-his (SEQ ID NO: 99);
pET26b-Kana-T7-pelB-Nb238D4-PE38-his (SEQ ID NO: 100);
pET26b-Kana-T7-pelB-NbEGa1-PE38-his (SEQ ID NO: 101);
pET26b-Kana-T7-pelB-VHH1-PE38-his (SEQ ID NO: 102);
pET26b-Kana-T7-pelB-VHH26-PE38-his (SEQ ID NO: 103).

### 9.3 Construction of Plasmids for Expressing Fusion Protein of Nanobody Nb127D01 and Toxin CET40 or DT390 Under IPTG Induction

Nucleotide sequences encoding toxin proteins CET40 and DT390 were inserted into the plasmid expressing nanobody Nb127D01 (pET26b-pelB-Nb127D01-HA-his) as described in Example 3.3. The obtained recombinant plasmids were designated as follows:
pET26b-Kana-T7-pelB-127D01-CET40-HA-his (SEQ ID NO: 104)
pET26b-Kana-T7-pelB-127D01-DT390-HA-his (SEQ ID NO: 105).

### 9.4 Construction of Strains Expressing Nanobodies, Toxin Proteins and Nanobodies Fused to Toxin Proteins Under IPTG Induction

The recombinant plasmids were transformed into the recombinant protein expressing strain BL21(DE3) (BL21(DE3) Compound E. coli Strain, Vazyme # C504-03) by chemical transformation. Specifically, the competent cells were thawed on ice, 100 ng of the above recombinant plasmid was mixed with the competent cells, and placed on ice for 30 min. After heat shock in 42°C water bath for 45 sec, it was immediately placed on ice for 3 min. 900µL of LB medium (antibiotic free) was added, followed by shaking at 37° C for 1 h (rotation speed 200-250 rpm), and centrifuging at 5,000 rpm for 5 min. 900µL of supernatant was discarded, the bacteria were resuspended with the remaining volume of medium, and slightly spread on LB plate containing kanamycin (KanR, 100 mg/L) with a sterile spreading rod. The plates were inverted and incubated overnight in a 37°C incubator.

The obtained strains expressing nanobodies, toxin proteins and nanobodies fused to toxin proteins under IPTG induction were as follows:
BL21(DE3)-pET26b-Kana-T7-pelB-Nb238D2-his;
BL21(DE3)-pET26b-Kana-T7-pelB-Nb238D4-his;
BL21(DE3)-pET26b-Kana-T7-pelB-EGa1-his;
BL21(DE3)-pET26b-Kana-T7-pelB-VHH1-his;
BL21(DE3)-pET26b-Kana-T7-pelB-VHH26-his;
BL21(DE3)-pET26b-Kana-T7-pelB-Nb238D2-PE38-his;
BL21(DE3)-pET26b-Kana-T7-pelB-Nb238D4-PE38-his;
BL21(DE3)-pET26b-Kana-T7-pelB-NbEGa1-PE3 8-his;
BL21(DE3)-pET26b-Kana-T7-pelB-VHH1-PE38-his;
BL21(DE3)-pET26b-Kana-T7-pelB-VHH26-PE38-his;
BL21(DE3)-pET26b-Kana-T7-pelB-CET40-HA-his;
BL21(DE3)-pET26b-Kana-T7-pelB-127D01-CET40-HA-his;
BL21(DE3)-pET26b-Kana-T7-pelB-DT390-HA-his;
BL21(DE3)-pET26b-Kana-T7-pelB-127D01-DT390-HA-his.

### 9.5 Prokaryotic expression of nanobodies and nanobodies fused to toxin proteins under IPTG induction

The purpose of this example was to detect the ability of the constructed strain to express heterologous proteins.

To this end, prokaryotic expression was performed with the BL21(DE3) strains constructed in Examples 9.4. Specifically:
i) The cryopreserved bacteria were revived in liquid medium, when the culture reached OD=0.4-0.5, IPTG was added at a final concentration of 0.5 mM for the induction overnight in a shaker at 16°C (induced for 14-16h, 200-250 rpm), and the bacterial culture was taken out and placed on ice.
ii) The culture was centrifuged at 4,200 rpm and 4°C for 10 min, and the bacterial pellete and LB medium supernatant were collected separately. The LB medium supernatant was placed in an ultrafiltration tube (Amicon Ultra, # UFC901096) and centrifuged at 2,500 rpm for 2 h at 4°C, and the concentrated LB medium supernatant was collected, in which the secreted proteins was present.
iii) The lysing solution (Qiagen, Qproteome Bacterial Protein Prep Kit # 37900, referring to the experimental manual Qproteome Bacterial Protein Preparation Handbook, www.qiagen.com) was added to the bacterial pellete, placed on ice for 30 min, then centrifuged at 14,000 rpm for 30 min at 4°C, and the bacterial debris precipitate and the supernatant of the lysate were collected separately. The bacterial debris precipitate contained proteins in functional inclusion body, and the lysis supernatant contained the expressed but unsecreted soluble protein.
iv) 5×SDS loading buffer (Beyotime, #P0015) was added to the above collected samples (concentrated LB medium supernatant, bacterial debris precipitate and lysis supernatant), and the metal bath at 100°C was used for 5 min; then SDS-PAGE was performed: electrophoresis was performed at 80 V for 20 min; after the sample was transferred to the interface between the separation gel and the concentration gel, the voltage was raised to 120 V until the dye completely left the gel.
v) According to the instructions, the membrane transferring was performed using a Pyxis^{™} M Protein Transfer Stack (PVDF membrane) and a transfection instrument. After the membrane transferring, the membrane was washed with TBS buffer and TBST buffer in sequence; the PVDF membrane was blocked with 5% skim milk (dissolved in TBST buffer) at room temperature for 1 h; the primary antibody (6*his, his-tag monoclonal antibody, Proteintech, # 66005-1-lg) was diluted in 5% skim milk at a ratio of 1:10000, and incubated overnight at 4°C; the PVDF membrane incubated overnight was washed with TBST buffer for multiple times, and the antibody on the membrane surface was removed; the secondary antibody (HRP-conjugate Affinity Goat Anti-Rabbit IgG (H+L), Proteintech, # SA00001-2-100UL) was diluted in 5% skim milk at a ratio of 1:5000 for the incubation at room temperature for 1 h; the incubated PVDF membrane was washed with TBST buffer for multiple times, the unbound antibody on the membrane surface was removed, and finally the membrane was soaked in TBS. The prepared A and B solutions for development was mixed at a ratio of 1:1, the membrane was placed in a tray, the AB solution was added, and the membrane was exposed for 5 min and imaged with a multifunctional imaging device (Thermo Fisher, SuperSignal^{™} West Pico PLUS Chemiluminate Substrate).

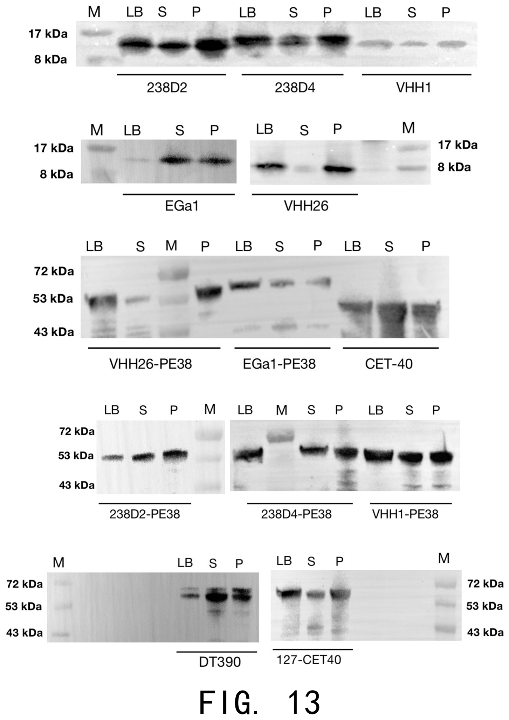

As shown in FIG. 13 below, all heterologous proteins were successfully expressed under IPTG induction.

### Example 10: Anaerobic Expression of Nanobody and Nanobody Fused to Toxin Protein

First, plasmids and strains for the anaerobic expression of nanobodies and nanobodies fused to toxin proteins were constructed.

The plasmids constructed in Example 9, pET26b-Kana-T7-pelB-238D2-his, pET26b-Kana-T7-pelB-238D4-his, pET26b-Kana-T7-pelB-EGa1-his, pET26b-Kana-T7-pelB-VHH1-his, pET26b-Kana-T7-pelB-VHH26-HA-his, pET26b-Kana-T7-pelB-Nb238D2-PE38-his, pET26b-Kana-T7-pelB-Nb238D4-PE38-his, pET26b-Kana-T7-pelB-NbEGa1-PE38-his, pET26b-Kana-T7-pelB-VHH1-PE38-his, pET26b-Kana-T7-pelB-VHH26-PE38-his, pET26b-Kana-T7-pelB-CET40-HA-his, pET26b-Kana-T7-pelB-DT390-HA-his, pET26b-Kana-T7-pelB-127D01-CET40-HA-his, and pET26b-Kana-T7-pelB-127D01-DT390-HA-his were employed as templates for the amplification of the linear fragments encoding the nanobodies, toxin proteins or nanobodies fused to toxin proteins, and pSC101-pFNRsp was employed as template for the amplification of vector backbone. The linear fragments of vector backbone were amplified by PCR. The primers as used were shown in Table 5, and the PCR system and condition were as described in Examples 3.2.

**Table 5**

| Name of Primers | Purpose | Sequence |
|---|---|---|
| Backbone FOR | Amplifying the linearfragment of vectro pSC101-pFNRsp | GGCTCTGGAAGATCCGCG |
| Backbone REV | | GGCCGCAAGTAGGCCATC |
| Nb frag F1 | Amplifying the fragments comprising target genes | GATGGCCTACTTGCGGCCTCACACAGGAAA GTACTAGATGAAATACCTGCTGCCGAC |
| Nb frag R1 | | CGCGGATCTTCCAGAGCCCCGGATCTTAAT GATGATGGTGATG |

The obtained plasmids were as follows:
pSC101-Cm-FNRSP-0033-pelB-238D2-His (SEQ ID NO: 106);
pSC101-Cm-FNRSP-0033-pelB-238D4-His (SEQ ID NO: 107);
pSC101-Cm-FNRSP-0033-pelB-EGa1-His (SEQ ID NO: 108);
pSC101-Cm-FNRSP-0033-pelB-VHH1-His (SEQ ID NO: 109);
pSC101-Cm-FNRSP-0033-pelB-VHH26-His (SEQ ID NO: 110);
pSC101-Cm-FNRSP-0033-pelB-238D2-PE38-His (SEQ ID NO: 111);
pSC101-Cm-FNRSP-0033-pelB-238D4-PE38-His (SEQ ID NO: 112);
pSC101-Cm-FNRSP-0033-pelB-EGa1-PE38-His (SEQ ID NO: 113);
pSC101-Cm-FNRSP-0033-pelB-VHH1-PE38-His (SEQ ID NO: 114);
pSC101-Cm-FNRSP-0033-pelB-VHH26-PE38-His (SEQ ID NO: 115);
pSC101-Cm-FNRSP-0033-pelB-CET40-HA-His (SEQ ID NO: 116);
pSC101-Cm-FNRSP-0033-pelB-DT390-HA-His (SEQ ID NO: 117);
pSC101-Cm-FNRSP-0033-pelB-127D01-CT390-HA-His (SEQ ID NO: 118);
pSC101-Cm-FNRSP-0033-pelB-127D01-CET40-HA-His (SEQ ID NO: 119).

The above plasmids were introduced into competent cells of strain DB-ZW1 by electrotransformation method (E=18KV/cm). The obtained strains were as follows:
ZW1-pSC101-Cm-FNRSP-0033-pelB-238D2-His;
ZW1-pSC 1 01-Cm-FNRSP-0033-pelB-238D4-His;
ZW 1-pSC 101-Cm-FNRSP-0033-pelB-EGa1-His;
ZW1-pSC101-Cm-FNRSP-0033-pelB-VHH1-His;
ZW1-pSC101-Cm-FNRSP-0033-pelB-VHH26-His;
ZW1-pSC101-Cm-FNRSP-0033-pelB-238D2-PE38-His;
ZW1-pSC101-Cm-FNRSP-0033-pelB-238D4-PE38-His;
ZW1-pSC101-Cm-FNRSP-0033-pelB-EGa1-PE38-His;
ZW1-pSC101-Cm-FNRSP-0033-pelB-VHH1-PE38-His;
ZW1-pSC101-Cm-FNRSP-0033-pelB-VHH26-PE38-His;
ZW1-pSC101-Cm-FNRSP-0033-pelB-CET40-HA-His;
ZW1-pSC101-Cm-FNRSP-0033-pelB-DT390-HA-His;
ZW1-pSC101-Cm-FNRSP-0033-pelB-127D01-CT390-HA-His;
ZW1-pSC101-Cm-FNRSP-0033-pelB-127D01-CET40-HA-His.

The anaerobic expression of the fusion protein of the above strains and the effect thereof on treating tumors were tested as described in Examples 4 and 8.

### List of Sequences

SEQ ID NO:1: PsseA-hlyA-loxp-KnaR-loxp 
TTCACAGAAAAGTGTTGCCCCCTTCCATGGCGGAAGGGGGACAAAGGTGAATTTGTCCTACTCAGGAGAGCGTTCACCGACAA
ACAACAGATAAAACGAAAGGCCCAGTCTTTCGACTGAGCCTTTCGTTTTATTTGATGCACTAGTGCCGATCATATTCAATAAC
CCTTAATATAACTTCGTATAATGTATGCTATACGAAGTTATTAGGTCTGAAGAGGAGTTTACGTCCCATATGGAGTCCCGCTC
AGAAGAACTCGTCAAGAAGGCGATAGAAGGCGATGCGCTGCGAATCGGGAGCGGCGATACCGTAAAGCACGAGGAAGCGGTCA
GCCCATTCGCCGCCAAGCTCTTCAGCAATATCACGGGTAGCCAACGCTATGTCCTGATAGCGGTCCGCCACACCCAGCCGGCC
ACAGTCGATGAATCCAGAAAAGCGGCCATTTTCCACCATGATATTCGGCAAGCAGGCATCGCCATGGGTCACGACGAGATCCT
CGCCGTCGGGCATGCGCGCCTTGAGCCTGGCGAACAGTTCGGCCGGCGCGAGCCCCTGATGCTCTTCGTCCAGATCATCCTGA
TCGACAAGACCGGCTTCCATCCGAGTACGTGCTCGCTCGATGCGATGTTTCGCTTGGTGGTCGAATGGGCAGGTAGCCGGATC
AAGCGTATGCAGCCGCCGCATTGCATCAGCCATGATGGATACTTTCTCGGCAGGAGCAAGGTGAGATGACAGGAGATCCTGCC
CCGGCACTTCGCCCAATAGCAGCCAGTCCCTTCCCGCTTCAGTGACAACGTCGAGCACAGCTGCGCAAGGAACGCCCGTCGTG
GCCAGCCACGATAGCCGCGCTGCCTCGTCCTGCAGTTCATTCAGGGCACCGGACAGGTCGGTCTTGACAAAAAGAACCGGGCG
CCCCTGCGCTGACAGCCGGAACACGGCGGCATCAGAGCAGCCGATTGTCTGTTGTGCCCAGTCATAGCCGAATAGCCTCTCCA
CCCAAGCGGCCGGAGAACCTGCGTGCAATCCATCTTGTTCAATCATGCGAAACGATCCTCATCCTGTCTCTTGATCAGATCCT
GATCCCCTGCGCCATCAGATCCTTGGCGGCAAGAAAGCCATCCAGTTTACTTTGCAGGGCTTCCCAACCTTACCAGAGGGCGC
CCCAGCTGGCAATTCCGACGTCATTGCGTTGCTAGCCAAGCTTAGGATCCGGAACCCTTAATATAACTTCGTATAATGTATGC
TATACGAAGTTATTAGGTCCCTCGAAGAGGTTCACTAGCTATCTAGAGCTTGGACTCCTGTTGATAGATCCAGTAATGACCTC
AGAACTCCATCTGGATTTGTTCAGAACGCTCGGTTGCCGCCGGGCGTTTTTTATTGGTGAGAATCCAAGCCCGGGAAACGGGA
TCCTGATCTTGATCAGGCTGTGGGTCAGGCCACTCTTGGCATCGACACCAATGTGGGCCTTCATGCCAAAGTGCCACTGATTG
CCTTTCTTGGTCTGATGCATCTCCGGATCGCGTTGCTGCTCTTTGTTCTTGGTCGAGCTGGGTGCCTCAATGATGGTGGCATC
GACCAAGGTGCCTTGAGTCATCATGACGCCTGCTTCGGCCAGCCAGCGATTGATGGTCTTGAACAATTGGCGGGCCAGTTGAT
GCTGCTCCAGCAGGTGGCGGAAATTCATGATGGTGGTGCGGTCCGGCAAGGCGCTATCCAGGGATAACCGGGCAAACAGACGC
ATGGAGGCGATTTCGTACAGAGCATCTTCCATCGCGCCATCGCTCAGGTTGTACCAATGCTGCATGCAGTGAATGCGTAGCAT
GGTTTCCAGCGGATAAGGTCGCCGGCCATTACCAGCCTTGGGGTAAAACGGCTCGATGACTTCCACCATGTTTTGCCATGGCA
GAATCTGCTCCATGCGGGACAAGAAAATCTCTTTTCTGGTCTGACGGCGCTTACTGCTGAATTCACTGTCGGCGAAGGTAAGT
TGATGACTCATGATGAACCCTGTTCTATGGCTCCAGATGACAAACATGATCTCATATCAGGGACTTGTTCGCACCTTCCTTAG
CACGTTAATTATCTATCGTGTATATGGAGGGGATCTAGAATGAAAAAAATAATGCTAGTTTTTATTACACTTATATTAGTTAG
TCTACCAATTGCGCAACAAACTGAAGCAAAGGATGCATCTGCATTCAATAAAGAAAATTCAATTTCATCCATGGCACCACCAG
CATCTCCGCCTGCAAGTCCTAAGACGCCAATCGAAAAGAAACACGCGGATGAAATCGATAAGTATATACAAGGATTGGACTAC
AATAAAAACAATGTATTAGTATACCACGGAGATGCAGTGACAAATGTGCCGCCAAGAAAAGGTTACAAAGATGGAAATGAATA
TATTGTTGTGGAGAAAAAGAAGAAATCCATCAATCAAAATAATGCAGACATTCAAGTTGTGAATGCAATTTCGAGCCTAACCT
ATCCAGGTGCTCTCGTAAAAGCGAATTCGGAATTAGTAGAAAATCAACCAGATGTTCTCCCTGTAAAACGTGATTCATTAACA
CTCAGCATTGATTTGCCAGGTATGACTAATCAAGACAATAAAATCGTTGTAAAAAATGCCACTAAATCAAACGTTAACAACGC
AGTAAATACATTAGTGGAAAGATGGAATGAAAAATATGCTCAAGCTTATCCAAATGTAAGTGCAAAAATTGATTATGATGACG
AAATGGCTTACAGTGAATCACAATTAATTGCGAAATTTGGTACAGCATTTAAAGCTGTAAATAATAGCTTGAATGTAAACTTC
GGCGCAATCAGTGAAGGGAAAATGCAAGAAGAAGTCATTAGTTTTAAACAAATTTACTATAACGTGAATGTTAATGAACCTAC
AAGACCTTCCAGATTTTTCGGCAAAGCTGTTACTAAAGAGCAGTTGCAAGCGCTTGGAGTGAATGCAGAAAATCCTCCTGCAT
ATATCTCAAGTGTGGCGTATGGCCGTCAAGTTTATTTGAAATTATCAACTAATTCCCATAGTACTAAAGTAAAAGCTGCTTTT
GATGCTGCCGTAAGCGGAAAATCTGTCTCAGGTGATGTAGAACTAACAAATATCATCAAAAATTCTTCCTTCAAAGCCGTAAT
TTACGGAGGTTCCGCAAAAGATGAAGTTCAAATCATCGACGGCAACCTCGGAGACTTACGAGATATTTTGAAAAAAGGCGCTA
CTTTTAATCGAGAAACACCAGGAGTTCCCATTGCTTATACAACAAACTTCCTAAAAGACAATGAATTAGCTGTTATTAAAAAC
AACTCAGAATATATTGAAACAACTTCAAAAGCTTATACAGATGGAAAAATTAACATCGATCACTCTGGAGGATACGTTGCTCA
ATTCAACATTTCTTGGGATGAAGTAAATTATGATCCTGAAGGTAACGAAATTGTTCAACATAAAAACTGGAGCGAAAACAATA
AAAGTAAGCTAGCTCATTTCACATCGTCCATCTATTTGCCAGGTAACGCGAGAAATATTAATGTTTACGCTAAAGAATGCACT
GGTTTAGCTTGGGAATGGTGGAGAACGGTAATTGATGACCGGAACTTACCACTTGTGAAAAATAGAAATATCTCCATCTGGGG
CACCACGCTTTATCCGAAATATAGTAATAAAGTAGATAATCCAATCGAATAAGTCGACCTCGAGGACTAGTGAACCTCTTCGA
GGGACCTAATAACTTCGTATAGCATACATTATACGAAGTTATATTAAGGGTTATTGAATATGATCGGCTCGAGATTACTTCGC
ATTGCAATCAGCTACGGACTCAAGCTTACGTCAGGTCATACGATGAAGGCGATAAGTCGCGTAAGTTGCTAGCCCACGCGGCT
CACCTTCACGGGTGGGCCTTTCTTCGGTGTTTCAATCTCGATTAACAGGTAACGCAAAATTGCTGTGTACGTCAGA
SEQ ID NO:2: Pssbp1-dapE-Cm 
TTGGTTTCAGTGAATCCCGTTATCAGCAGTTTTTTGATGAGGTGTAGTCTATTTGTCCTACTCAGGAGAGCGTTCACCGACAA
ACAACAGATAAAACGAAAGGCCCAGTCTTTCGACTGAGCCTTTCGTTTTATTTGATGCACTAGTGCCGATCATATTCAATAAC
CCTTAATATAACTTCGTATAATGTATGCTATACGAAGTTATTAGGTCTGAAGAGGAGTTTACGTCCCATATGGAGTCCCGCTT
ACGCCCCGCCCTGCCACTCATCGCAGTACTGTTGTAATTCATTAAGCATTCTGCCGACATGGAAGCCATCACAGACGGCATGA
TGAACCTGAATCGCCAGCGGCATCAGCACCTTGTCGCCTTGCGTATAATATTTGCCCATGGTGAAAACGGGGGCGAAGAAGTT
GTCCATATTGGCCACGTTTAAATCAAAACTGGTGAAACTCACCCAGGGATTGGCTGAGACGAAAAACATATTCTCAATAAACC
CTTTAGGGAAATAGGCCAGGTTTTCACCGTAACACGCCACATCTTGCGAATATATGTGTAGAAACTGCCGGAAATCGTCGTGG
TATTCACTCCAGAGCGATGAAAACGTTTCAGTTTGCTCATGGAAAACGGTGTAACAAGGGTGAACACTATCCCATATCACCAG
CTCACCGTCTTTCATTGCCATACGAAATTCCGGATGAGCATTCATCAGGCGGGCAAGAATGTGAATAAAGGCCGGATAAAACT
TGTGCTTATTTTTCTTTACGGTCTTTAAAAAGGCCGTAATATCCAGCTGAACGGTCTGGTTATAGGTACATTGAGCAACTGAC
TGAAATGCCTCAAAATGTTCTTTACGATGCCATTGGGATATATCAACGGTGGTATATCCAGTGATTTTTTTCTCCATGCGAAA
CGATCCTCATCCTGTCTCTTGATCAGATCCTGATCCCCTGCGCCATCAGATCCTTGGCGGCAAGAAAGCCATCCAGTTTACTT
TGCAGGGCTTCCCAACCTTACCAGAGGGCGCCCCAGCTGGCAATTCCGACGTCATTGCGTTGCTAGCCAAGCTTAGGATCCGG
AACCCTTAATATAACTTCGTATAATGTATGCTATACGAAGTTATTAGGTCCCTCGAAGAGGTTCACTAGCTATCTAGAGCTTG
GACTCCTGTTGATAGATCCAGTAATGACCTCAGAACTCCATCTGGATTTGTTCAGAACGCTCGGTTGCCGCCGGGCGTTTTTT
ATTGGTGAGAATCCAAGCCCGGGAAACGGGATCCTGATCTTGATCAACCGAGGTCACAACATAGTAAAAGCGCTATTGGTAAT
 
GGTACAATCGCGCGTTTACACTTATTCAGAACGATTTTTTTCAGGAGGGCCTACTTGTCACACAGGACGGTAATGTCGTGCCC
GGTTATTGAGCTGACACAGCAACTTATTCGTCGCCCCTCCCTGAGTCCGGATGACGCAGGCTGTCAGGCATTAATGATTGAAC
GCCTGCGTAAAATCGGTTTTACCATTGAGCATATGGATTTTGGCGACACGCAAAATTTTTGGGCATGGCGTGGGCGTGGCGAA
ACGCTGGCGTTCGCCGGGCATACTGACGTCGTACCGGCTGGCGATGTCGATCGCTGGATCAATCCGCCGTTTGAGCCTACGAT
TCGCGACGGAATGTTGTTCGGTCGCGGCGCGGCGGATATGAAAGGTTCACTGGCGGCCATGGTGGTGGCTGCTGAACGTTTTG
TTGCGCAGCATCCTCATCACCGGGGCCGCCTCGCATTTTTGATTACCTCTGATGAGGAAGCCAGCGCAAAAAATGGCACCGTA
AAGGTGGTTGAAGCGCTGATGGCCCGTAACGAGCGGCTGGATTACTGTCTGGTCGGCGAACCTTCCAGTACTGAAATTGTGGG
CGATGTCGTCAAAAACGGGCGTCGCGGTTCTCTCACCTGTAACCTCACTATCCATGGCGTTCAGGGGCACGTCGCCTATCCGC
ATCTGGCGGATAACCCTGTTCATCGCGCCGCGCCTTTTCTTAATGAACTGGTCGCTATCGAGTGGGATCGGGGCAATGACTTT
TTCCCGGCCACCAGTATGCAGGTAGCCAATATCCAGGCAGGCACCGGCAGCAATAATGTCATTCCCGGCGAACTGTTCGTGCA
GTTTAACTTCCGTTTCAGCACGGAACTGACCGATGAGATGATCAAAGAGCGGGTACATGCGCTGCTTGAAAAGCATCAGCTAC
GCTATACCGTAGACTGGTGGCTTTCCGGGCAGCCGTTCCTGACCGCACGCGGCAAGCTGGTGGACGCGGTGGTAAACGCCATT
GAGCACTATAATGAAATTAAACCGCAATTACTGACGACGGGCGGTACGTCCGACGGACGTTTTATCGCCCGTATGGGAGCGCA
GGTGGTAGAACTGGGGCCGGTGAACGCCACCATTCATAAAATTAATGAATGTGTGAATGCCGCCGATCTCCAGCTACTGGCCC
GTATGTATCAACGCATTATGGAACAGCTCGTCGCCCATCACCATCACCATCACTAAGATTACTTCGCATTGCAATCAGCTACG
GACTCAAGCTTACGTCAGGTCATACGATGAAGGCGATAAGTCGCGTAAGTTGCTAGCCCACGCGGCTCACCTTCACGGGTGGG
CCTTTCTTCGTGAGCGTTCCGGTAAGAGGAAATAAGCATGGACTGGCTGGCAAAATATTG
SEQ ID NO:3: Plasmid PsseA-hlyA-loxp-KnaR-loxp 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCGACTTCCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTACTGTCTAGGGCGGCGGATTTGTCCTACTCAGGAGAGCGTTCACCGACAAACAACAGATA
AAACGAAAGGCCCAGTCTTTCGACTGAGCCTTTCGTTTTATTTGATGCACTAGTGCCGATCATATTCAATAACCCTTAATATA
ACTTCGTATAATGTATGCTATACGAAGTTATTAGGTCTGAAGAGGAGTTTACGTCCCATATGGAGTCCCGCTCAGAAGAACTC
GTCAAGAAGGCGATAGAAGGCGATGCGCTGCGAATCGGGAGCGGCGATACCGTAAAGCACGAGGAAGCGGTCAGCCCATTCGC
CGCCAAGCTCTTCAGCAATATCACGGGTAGCCAACGCTATGTCCTGATAGCGGTCCGCCACACCCAGCCGGCCACAGTCGATG
AATCCAGAAAAGCGGCCATTTTCCACCATGATATTCGGCAAGCAGGCATCGCCATGGGTCACGACGAGATCCTCGCCGTCGGG
CATGCGCGCCTTGAGCCTGGCGAACAGTTCGGCCGGCGCGAGCCCCTGATGCTCTTCGTCCAGATCATCCTGATCGACAAGAC
CGGCTTCCATCCGAGTACGTGCTCGCTCGATGCGATGTTTCGCTTGGTGGTCGAATGGGCAGGTAGCCGGATCAAGCGTATGC
AGCCGCCGCATTGCATCAGCCATGATGGATACTTTCTCGGCAGGAGCAAGGTGAGATGACAGGAGATCCTGCCCCGGCACTTC
GCCCAATAGCAGCCAGTCCCTTCCCGCTTCAGTGACAACGTCGAGCACAGCTGCGCAAGGAACGCCCGTCGTGGCCAGCCACG
ATAGCCGCGCTGCCTCGTCCTGCAGTTCATTCAGGGCACCGGACAGGTCGGTCTTGACAAAAAGAACCGGGCGCCCCTGCGCT
GACAGCCGGAACACGGCGGCATCAGAGCAGCCGATTGTCTGTTGTGCCCAGTCATAGCCGAATAGCCTCTCCACCCAAGCGGC
CGGAGAACCTGCGTGCAATCCATCTTGTTCAATCATGCGAAACGATCCTCATCCTGTCTCTTGATCAGATCCTGATCCCCTGC
GCCATCAGATCCTTGGCGGCAAGAAAGCCATCCAGTTTACTTTGCAGGGCTTCCCAACCTTACCAGAGGGCGCCCCAGCTGGC
AATTCCGACGTCATTGCGTTGCTAGCCAAGCTTAGGATCCGGAACCCTTAATATAACTTCGTATAATGTATGCTATACGAAGT
TATTAGGTCCCTCGAAGAGGTTCACTAGCTATCTAGAGCTTGGACTCCTGTTGATAGATCCAGTAATGACCTCAGAACTCCAT
CTGGATTTGTTCAGAACGCTCGGTTGCCGCCGGGCGTTTTTTATTGGTGAGAATCCAAGCCCGGGAAACGGGATCCTGATCTT
GATCAGGCTGTGGGTCAGGCCACTCTTGGCATCGACACCAATGTGGGCCTTCATGCCAAAGTGCCACTGATTGCCTTTCTTGG
 
TCTGATGCATCTCCGGATCGCGTTGCTGCTCTTTGTTCTTGGTCGAGCTGGGTGCCTCAATGATGGTGGCATCGACCAAGGTG
CCTTGAGTCATCATGACGCCTGCTTCGGCCAGCCAGCGATTGATGGTCTTGAACAATTGGCGGGCCAGTTGATGCTGCTCCAG
CAGGTGGCGGAAATTCATGATGGTGGTGCGGTCCGGCAAGGCGCTATCCAGGGATAACCGGGCAAACAGACGCATGGAGGCGA
TTTCGTACAGAGCATCTTCCATCGCGCCATCGCTCAGGTTGTACCAATGCTGCATGCAGTGAATGCGTAGCATGGTTTCCAGC
GGATAAGGTCGCCGGCCATTACCAGCCTTGGGGTAAAACGGCTCGATGACTTCCACCATGTTTTGCCATGGCAGAATCTGCTC
CATGCGGGACAAGAAAATCTCTTTTCTGGTCTGACGGCGCTTACTGCTGAATTCACTGTCGGCGAAGGTAAGTTGATGACTCA
TGATGAACCCTGTTCTATGGCTCCAGATGACAAACATGATCTCATATCAGGGACTTGTTCGCACCTTCCTTAGCACGTTAATT
ATCTATCGTGTATATGGAGGGGATCTAGAATGAAAAAAATAATGCTAGTTTTTATTACACTTATATTAGTTAGTCTACCAATT
GCGCAACAAACTGAAGCAAAGGATGCATCTGCATTCAATAAAGAAAATTCAATTTCATCCATGGCACCACCAGCATCTCCGCC
TGCAAGTCCTAAGACGCCAATCGAAAAGAAACACGCGGATGAAATCGATAAGTATATACAAGGATTGGACTACAATAAAAACA
ATGTATTAGTATACCACGGAGATGCAGTGACAAATGTGCCGCCAAGAAAAGGTTACAAAGATGGAAATGAATATATTGTTGTG
GAGAAAAAGAAGAAATCCATCAATCAAAATAATGCAGACATTCAAGTTGTGAATGCAATTTCGAGCCTAACCTATCCAGGTGC
TCTCGTAAAAGCGAATTCGGAATTAGTAGAAAATCAACCAGATGTTCTCCCTGTAAAACGTGATTCATTAACACTCAGCATTG
ATTTGCCAGGTATGACTAATCAAGACAATAAAATCGTTGTAAAAAATGCCACTAAATCAAACGTTAACAACGCAGTAAATACA
TTAGTGGAAAGATGGAATGAAAAATATGCTCAAGCTTATCCAAATGTAAGTGCAAAAATTGATTATGATGACGAAATGGCTTA
CAGTGAATCACAATTAATTGCGAAATTTGGTACAGCATTTAAAGCTGTAAATAATAGCTTGAATGTAAACTTCGGCGCAATCA
GTGAAGGGAAAATGCAAGAAGAAGTCATTAGTTTTAAACAAATTTACTATAACGTGAATGTTAATGAACCTACAAGACCTTCC
AGATTTTTCGGCAAAGCTGTTACTAAAGAGCAGTTGCAAGCGCTTGGAGTGAATGCAGAAAATCCTCCTGCATATATCTCAAG
TGTGGCGTATGGCCGTCAAGTTTATTTGAAATTATCAACTAATTCCCATAGTACTAAAGTAAAAGCTGCTTTTGATGCTGCCG
TAAGCGGAAAATCTGTCTCAGGTGATGTAGAACTAACAAATATCATCAAAAATTCTTCCTTCAAAGCCGTAATTTACGGAGGT
TCCGCAAAAGATGAAGTTCAAATCATCGACGGCAACCTCGGAGACTTACGAGATATTTTGAAAAAAGGCGCTACTTTTAATCG
AGAAACACCAGGAGTTCCCATTGCTTATACAACAAACTTCCTAAAAGACAATGAATTAGCTGTTATTAAAAACAACTCAGAAT
ATATTGAAACAACTTCAAAAGCTTATACAGATGGAAAAATTAACATCGATCACTCTGGAGGATACGTTGCTCAATTCAACATT
TCTTGGGATGAAGTAAATTATGATCCTGAAGGTAACGAAATTGTTCAACATAAAAACTGGAGCGAAAACAATAAAAGTAAGCT
AGCTCATTTCACATCGTCCATCTATTTGCCAGGTAACGCGAGAAATATTAATGTTTACGCTAAAGAATGCACTGGTTTAGCTT
GGGAATGGTGGAGAACGGTAATTGATGACCGGAACTTACCACTTGTGAAAAATAGAAATATCTCCATCTGGGGCACCACGCTT
TATCCGAAATATAGTAATAAAGTAGATAATCCAATCGAATAAGTCGACCTCGAGGACTAGTGAACCTCTTCGAGGGACCTAAT
AACTTCGTATAGCATACATTATACGAAGTTATATTAAGGGTTATTGAATATGATCGGCTCGAGATTACTTCGCATTGCAATCA
GCTACGGACTCAAGCTTACGTCAGGTCATACGATGAAGGCGATAAGTCGCGTAAGTTGCTAGCCCACGCGGCTCACCTTCACG
GGTGGGCCTTTCTTCGCGATCCACATG
SEQ ID NO:4: Plasmid Pssbp1-dapE-Cm 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCGACTTCCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTACTGTCTAGGGCGGCGGATTTGTCCTACTCAGGAGAGCGTTCACCGACAAACAACAGATA
AAACGAAAGGCCCAGTCTTTCGACTGAGCCTTTCGTTTTATTTGATGCACTAGTGCCGATCATATTCAATAACCCTTAATATA
ACTTCGTATAATGTATGCTATACGAAGTTATTAGGTCTGAAGAGGAGTTTACGTCCCATATGGAGTCCCGCTTACGCCCCGCC
CTGCCACTCATCGCAGTACTGTTGTAATTCATTAAGCATTCTGCCGACATGGAAGCCATCACAGACGGCATGATGAACCTGAA
TCGCCAGCGGCATCAGCACCTTGTCGCCTTGCGTATAATATTTGCCCATGGTGAAAACGGGGGCGAAGAAGTTGTCCATATTG
 
GCCACGTTTAAATCAAAACTGGTGAAACTCACCCAGGGATTGGCTGAGACGAAAAACATATTCTCAATAAACCCTTTAGGGAA
ATAGGCCAGGTTTTCACCGTAACACGCCACATCTTGCGAATATATGTGTAGAAACTGCCGGAAATCGTCGTGGTATTCACTCC
AGAGCGATGAAAACGTTTCAGTTTGCTCATGGAAAACGGTGTAACAAGGGTGAACACTATCCCATATCACCAGCTCACCGTCT
TTCATTGCCATACGAAATTCCGGATGAGCATTCATCAGGCGGGCAAGAATGTGAATAAAGGCCGGATAAAACTTGTGCTTATT
TTTCTTTACGGTCTTTAAAAAGGCCGTAATATCCAGCTGAACGGTCTGGTTATAGGTACATTGAGCAACTGACTGAAATGCCT
CAAAATGTTCTTTACGATGCCATTGGGATATATCAACGGTGGTATATCCAGTGATTTTTTTCTCCATTTTAGCTTCCTTAGCT
CCTGAAAATCTCGATAACTCAAAAAATACGCCCGGTAGTGATCTTATTTCATTATGGTGAAAGTTGGAACCTCTTACGTGCCG
ATCAGACGTCATTGCGTTGCTAGCCAAGCTTAGGATCCGGAACCCTTAATATAACTTCGTATAATGTATGCTATACGAAGTTA
TTAGGTCCCTCGAAGAGGTTCACTAGCTATCTAGAGCTTGGACTCCTGTTGATAGATCCAGTAATGACCTCAGAACTCCATCT
CGATTTCTTCACAACCCTCGGTTCCCCCCGGGCCCTTTTTATTGCTCACAATCCAACCCCCGCAAACCGGATCCTGATCTTGA
TCAACCGAGGTCACAACATAGTAAAAGCGCTATTGGTAATGGTACAATCGCGCGTTTACACTTATTCAGAACGATTTTTTTCA
GGAGGGCCTACTTGTCACACAGGACGGTAATGTCGTGCCCGGTTATTGAGCTGACACAGCAACTTATTCGTCGCCCCTCCCTG
AGTCCGGATGACGCAGGCTGTCAGGCATTAATGATTGAACGCCTGCGTAAAATCGGTTTTACCATTGAGCATATGGATTTTGG
CGACACGCAAAATTTTTGGGCATGGCGTGGGCGTGGCGAAACGCTGGCGTTCGCCGGGCATACTGACGTCGTACCGGCTGGCG
ATGTCGATCGCTGGATCAATCCGCCGTTTGAGCCTACGATTCGCGACGGAATGTTGTTCGGTCGCGGCGCGGCGGATATGAAA
GGTTCACTGGCGGCCATGGTGGTGGCTGCTGAACGTTTTGTTGCGCAGCATCCTCATCACCGGGGCCGCCTCGCATTTTTGAT
TACCTCTGATGAGGAAGCCAGCGCAAAAAATGGCACCGTAAAGGTGGTTGAAGCGCTGATGGCCCGTAACGAGCGGCTGGATT
ACTGTCTGGTCGGCGAACCTTCCAGTACTGAAATTGTGGGCGATGTCGTCAAAAACGGGCGTCGCGGTTCTCTCACCTGTAAC
CTCACTATCCATGGCGTTCAGGGGCACGTCGCCTATCCGCATCTGGCGGATAACCCTGTTCATCGCGCCGCGCCTTTTCTTAA
TGAACTGGTCGCTATCGAGTGGGATCGGGGCAATGACTTTTTCCCGGCCACCAGTATGCAGGTAGCCAATATCCAGGCAGGCA
CCGGCAGCAATAATGTCATTCCCGGCGAACTGTTCGTGCAGTTTAACTTCCGTTTCAGCACGGAACTGACCGATGAGATGATC
AAAGAGCGGGTACATGCGCTGCTTGAAAAGCATCAGCTACGCTATACCGTAGACTGGTGGCTTTCCGGGCAGCCGTTCCTGAC
CGCACGCGGCAAGCTGGTGGACGCGGTGGTAAACGCCATTGAGCACTATAATGAAATTAAACCGCAATTACTGACGACGGGCG
GTACGTCCGACGGACGTTTTATCGCCCGTATGGGAGCGCAGGTGGTAGAACTGGGGCCGGTGAACGCCACCATTCATAAAATT
AATGAATGTGTGAATGCCGCCGATCTCCAGCTACTGGCCCGTATGTATCAACGCATTATGGAACAGCTCGTCGCCCATCACCA
TCACCATCACTAAGATTACTTCGCATTGCAATCAGCTACGGACTCAAGCTTACGTCAGGTCATACGATGAAGGCGATAAGTCG
CGTAAGTTGCTAGCCCACGCGGCTCACCTTCACGGGTGGGCCTTTCTTCGCGATCCACATG
SEQ ID NO:5: PsseA-hlyA-loxp-KnaR-loxp 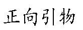
   TTCACAGAAAAGTGTTGCCCCCTTCCATGGCGGAAGGGGGACAAAGGTGAATTTGTCCTACTCAGGAGAGCGTTCA
SEQ ID NO:6: PsseA-hlyA-loxp-KnaR-loxp 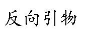
   TCTGACGTACACAGCAATTTTGCGTTACCTGTTAATCGAGATTGAAACACCGAAGAAAGGCCCACCCG
SEQ ID NO:7: Pssbp1-dapE-Cm 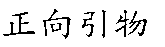
   TTGGTTTCAGTGAATCCCGTTATCAGCAGTTTTTTGATGAGGTGTAGTCTATTTGTCCTACTCAGGAGAGCG
SEQ ID NO:8: Pssbp1-dapE-Cm 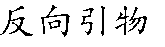
   CAATATTTTGCCAGCCAGTCCATGCTTATTTCCTCTTACCGGAACGCTCACGAAGAAAGGCCCACCCG
SEQ ID NO: 9: 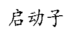 pepTp 
GCAGGGGTAAAAGTGACCTGACGCAATATTTGTCTTTTCTTGCTTCTTAATAATGTTGTCACAAAAAGTGAGGGTGACTACAT
GG
SEQ ID NO:10: 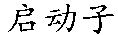 fnrSp 
CGCCGCAAAGTTTGAGCGAAGTCAATAAACTCTCTACCCATTCAGGGCAATATCTCTCTTGCAGGTGAATGCAACGTCAAGCG
AT
SEQ ID NO:11: 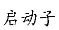 ysgAp 
TCAGAAGAAGCAAAAAGACACTTTACCGAAGGGTTTAACATTTTTTCGTGATACTCATCACCATGACGCAAATGCGTTGCATA
AA
SEQ ID NO:12: 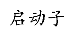 ssbp1 
AACCGAGGTCACAACATAGTAAAAGCGCTATTGGTAATGGTACAATCGCGCGTTTACACTTATTCAGAACGATTTTTTTCAGG
AG
SEQ ID NO:13: 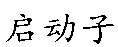 Hip1
   GGATAAAAGTGACCTGACGCAATATTTGTCTTTTCTTGCTTAATAATGTTGTCA
SEQ ID NO:14: 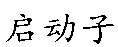 BBa_114018
   TGTAAGTTTATACATAGGCGAGTACTCTGTTATGG
SEQ ID NO:15: 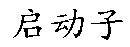 BBa_R1074
   TTAAATTTCCTCTCGTCAGGCCGGAATAACTCCCTATAATGCGCCACCACACTGATAGTGCTAGTGTAGATCAC
SEQ ID NO:16: 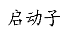 Ptet-Fnr
   AAAATTGATCTGAATCAATATTTTGACAAAAATTGATCTGAATCAATATTTACTGAGC
SEQ ID NO:17: 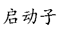 Ptet-arcA
   GTTAATAAAATGTTATTGACAGTTAATAAAATGTTATACTGAGC
SEQ ID NO: 18: 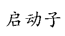 PsseA
   TTAGCACGTTAATTATCTATCGTGTATATG
SEQ ID NO: 19: Nb127D01 (CDR 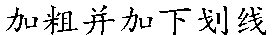 ) 
EVQLVESGGGLVQAGESLRLSCAASGSTFDFKVMGWYRQPPGKQREGVAAIRLSGNMHYAESVKGRFAISKANAKNTVYLQMN
SLRPEDTAVYYCKVNIRGQDYWGQGTQVTVSSGSG
SEQ ID NO:20 Nb163E3 (CDR 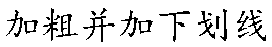 ) 
EVQLVESGGGLVQPGGSVRLSCVASGRIFSSNAMGWFRQAPGKEREFVAAITWRSGGSAYYADSAKGRFTISRDNAKNTVYLQ
MNSLKPEDTAVYYCAAGGSSWLSFPPDYWGQGTQVTVSS
SEQ ID NO:21 Nb97A9(CDR 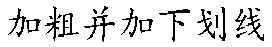 ) 
EVQLVESGGGLVQPGGSLRLSCVASGSIVRINTMGWYRQTPGKQRELVADITSGGNINYIDAVKGRFTISRDNTKKRVYLQMN
SLKPEDTAVYYCNAEIVVLVGVWTQRARTGNYWGQGTQVTVSS
SEQ ID NO:22 Nb127D01 
GAGGTTCAATTAGTGGAATCTGGCGGGGGATTGGTTCAAGCCGGTGAGTCCTTGCGTTTGAGCTGCGCCGCATCTGGGAGCAC
ATTTGATTTCAAAGTGATGGGCTGGTATCGTCAGCCGCCCGGAAAACAGCGCGAGGGTGTAGCAGCCATCCGCTTGAGCGGAA
ATATGCACTACGCCGAGTCTGTAAAAGGGCGCTTTGCTATTTCCAAAGCGAACGCTAAGAACACAGTGTATCTTCAGATGAAC
TCCTTGCGTCCCGAAGACACTGCCGTTTATTATTGCAAAGTAAACATCCGTGGGCAAGACTACTGGGGTCAAGGAACGCAGGT
CACAGTCTCTTCTGGCTCTGGC
SEQ ID NO:23 Nb163E3 
GAGGTGCAGCTGGTGGAGTCTGGGGGAGGATTGGTGCAGCCTGGGGGCTCTGTGAGACTCTCCTGTGTAGCCTCTGGACGCAT
CTTCAGTAGCAATGCCATGGGCTGGTTCCGCCAGGCTCCAGGGAAGGAGCGTGAGTTTGTAGCGGCCATTACCTGGAGGAGTG
GCGGTAGCGCGTACTATGCAGACTCCGCGAAGGGCCGATTCACCATCTCCAGAGACAACGCCAAGAACACGGTGTATTTGCAA
ATGAACAGCCTGAAACCTGAGGACACGGCCGTTTATTATTGTGCAGCTGGTGGTAGTTCCTGGTTAAGTTTTCCGCCGGACTA
CTGGGGCCAGGGGACCCAGGTCACCGTCTCCTCA
SEQ ID NO:24 Nb97A9 
GAGGTGCAGCTGGTGGAGTCTGGGGGAGGCTTGGTGCAGCCTGGGGGGTCTCTGAGACTCTCCTGTGTAGCCTCTGGAAGCAT
CGTCAGAATTAATACCATGGGCTGGTACCGCCAGACTCCAGGGAAGCAGCGCGAGTTGGTCGCAGATATTACCAGTGGTGGTA
ACATAAACTATATAGACGCCGTGAAGGGCCGATTCACCATCTCCAGAGACAACACCAAGAAGAGGGTGTATCTGCAAATGAAC
AGCCTGAAACCTGAGGACACGGCCGTCTATTACTGTAATGCAGAGATCGTTGTTCTGGTGGGAGTTTGGACCCAGCGTGCGCG
GACCGGCAACTACTGGGGCCAGGGGACCCAGGTCACCGTCTCCTCA
SEQ ID NO: 25 PE 
AEEAFDLWNECAKACVLDLKDGVRSSRMSVDPAIADTNGQGVLHYSMVLEGGNDALKLAIDNALSITSDGLTIRLEGGVEPNK
PVRYSYTRQARGSWSLNWLVPIGHEKPSNIKVFIHELNAGNQLSHMSPIYTIEMGDELLAKLARDATFFVRAHESNEMQPTLA
ISHAGVSVVMAQTQPRREKRWSEWASGKVLCLLDPLDGVYNYLAQQRCNLDDTWEGKIYRVLAGNPAKHDLDIKPTVISHRLH
FPEGGSLAALTAHQACHLPLETFTRHRQPRGWEQLEQCGYPVQRLVALYLAARLSWNQVDQVIRNALASPGSGGDLGEAIREQ
PEQARLALTLAAAESERFVRQGTGNDEAGAANADVVSLTCPVAAGECAGPADSGDALLERNYPTGAEFLGDGGDVSFSTRGTQ
NWTVERLLQAHRQLEERGYVFVGYHGTFLEAAQSIVFGGVRARSQDLDAIWRGFYIAGDPALAYGYAQDQEPDARGRIRNGAL
LRVYVPRSSLPGFYRTSLTLAAPEAAGEVERLIGHPLPLRLDAITGPEEEGGRLETILGWPLAERTVVIPSAIPTDPRNVGGD
LDPSSIPDKEQAISALPDYASQPGKPPREDLK
SEQ ID NO: 26 PE38 
PEGGSLAALTAHQACHLPLETFTRHRQPRGWEQLEQCGYPVQRLVALYLAARLSWNQVDQVIRNALASPGSGGDLGEAIREQP
EQARLALTLAAAESERFVRQGTGNDEAGAANGPADSGDALLERNYPTGAEFLGDGGDVSFSTRGTQNWTVERLLQAHRQLEER
GYVFVGYHGTFLEAAQSIVFGGVRARSQDLDAIWRGFYIAGDPALAYGYAQDQEPDARGRIRNGALLRVYVPRSSLPGFYRTS
LTLAAPEAAGEVERLIGHPLPLRLDAITGPEEEGGRLETILGWPLAERTVVIPSAIPTDPRNVGGDLDPSSIPDKEQAISALP
DYASQPGKPPREDL
SEQ ID NO: 27 PE[LR] 
RHRQPRGWEQLPTGAEFLGDGGDVSFSTRGTQNWTVERLLQAHRQLEERGYVFVGYHGTFLEAAQSIVFGGVRARSQDLDAIW
RGFYIAGDPALAYGYAQDQEPDARGRIRNGALLRVYVPRSSLPGFYRTSLTLAAPEAAGEVERLIGHPLPLRLDAITGPEEEG
GRLETILGWPLAERTVVIPSAIPTDPRNVGGDLDPSSIPDKEQAISALPDYASQPGKPPREDLK
SEQ ID NO: 28 pelB 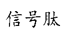
   MKYLLPTAAAGLLLLAAQPAMA
SEQ ID NO: 29 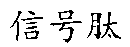 psp
   MGLKMKKRSGKKAWMLLVMSLLIAAVPITASA
SEQ ID NO:30 pET26b-pelB-Nb127D01-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
 
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAGGTGCAGCTGGTGGAGTCTGGGGGAGGCTTGGTGCAG
CCTGGGGGGTCTCTGAGACTCTCCTGTGTAGCCTCTGGAAGCATCGTCAGAATTAATACCATGGGCTGGTACCGCCAGACTCC
AGGGAAGCAGCGCGAGTTGGTCGCAGATATTACCAGTGGTGGTAACATAAACTATATAGACGCCGTGAAGGGCCGATTCACCA
TCTCCAGAGACAACACCAAGAAGAGGGTGTATCTGCAAATGAACAGCCTGAAACCTGAGGACACGGCCGTCTATTACTGTAAT
GCAGAGATCGTTGTTCTGGTGGGAGTTTGGACCCAGCGTGCGCGGACCGGCAACTACTGGGGCCAGGGGACCCAGGTCACCGT
CTCCTCAtacccctatgacgtgcctgattacgccggctctggccaccaccaccaccaccactgagatccggctgctaacaaag
cccgaaaggaagctgagttggctgctgccaccgctgagcaataactagcataaccccttggggcctctaaacgggtcttgagg
ggttttttgctgaaaggaggaactatatccggat
SEQ ID NO:31 pET26b-pelB-Nb163E3-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
 
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAGGTGCAGCTGGTGGAGTCTGGGGGAGGATTGGTGCAG
CCTGGGGGCTCTGTGAGACTCTCCTGTGTAGCCTCTGGACGCATCTTCAGTAGCAATGCCATGGGCTGGTTCCGCCAGGCTCC
AGGGAAGGAGCGTGAGTTTGTAGCGGCCATTACCTGGAGGAGTGGCGGTAGCGCGTACTATGCAGACTCCGCGAAGGGCCGAT
TCACCATCTCCAGAGACAACGCCAAGAACACGGTGTATTTGCAAATGAACAGCCTGAAACCTGAGGACACGGCCGTTTATTAT
TGTGCAGCTGGTGGTAGTTCCTGGTTAAGTTTTCCGCCGGACTACTGGGGCCAGGGGACCCAGGTCACCGTCTCCTCAtaccc
ctatgacgtgcctgattacgccggctctggccaccaccaccaccaccactgagatccggctgctaacaaagcccgaaaggaag
ctgagttggctgctgccaccgctgagcaataactagcataaccccttggggcctctaaacgggtcttgaggggttttttgctg
aaaggaggaactatatccggat
SEQ ID NO:32 pET26b-pelB-Nb97A9-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
 
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAGGTGCAGCTGGTGGAGTCTGGGGGAGGCTTGGTGCAG
CCTGGGGGGTCTCTGAGACTCTCCTGTGTAGCCTCTGGAAGCATCGTCAGAATTAATACCATGGGCTGGTACCGCCAGACTCC
AGGGAAGCAGCGCGAGTTGGTCGCAGATATTACCAGTGGTGGTAACATAAACTATATAGACGCCGTGAAGGGCCGATTCACCA
TCTCCAGAGACAACACCAAGAAGAGGGTGTATCTGCAAATGAACAGCCTGAAACCTGAGGACACGGCCGTCTATTACTGTAAT
GCAGAGATCGTTGTTCTGGTGGGAGTTTGGACCCAGCGTGCGCGGACCGGCAACTACTGGGGCCAGGGGACCCAGGTCACCGT
CTCCTCAtacccctatgacgtgcctgattacgccggctctggccaccaccaccaccaccactgagatccggctgctaacaaag
cccgaaaggaagctgagttggctgctgccaccgctgagcaataactagcataaccccttggggcctctaaacgggtcttgagg
ggttttttgctgaaaggaggaactatatccggat
SEQ ID NO:33 pSC101-pFNRsp 
ctggaagatccgcgcgtaccgagttctaattcactggccgtcgttttacaacatacgatgaaggcgataagtcgcgtaagTTG
CTAGCccacgcGGCTCACCTTCACGGGTGGGCCTTTCTTCGCGATCCAcatgCATGGTCATAGCTGTTTCCTTTAATGCCCAT
GGCGTTTACCAATGCTTAATCAGTGAGGCACCTATCTCAGCGATCTGTCTATTTCGTTCATCCATAGTTGCCTGACTCCCCGT
CGTGTAGATAACTACGATACGGGAGGGCTTACCATCTGGCCCCAGTGCTGCAATGATACCGCgacttcCACGCTCACCGGCTC
CAGATTTATCAGCAATAAACCAGCCAGCCGGAAGGGCCGAGCGCAGAAGTGGTCCTGCAACTTTATCCGCCTCCATCCAGTCT
ATTAATTGTTGCCGGGAAGCTAGAGTAAGTAGTTCGCCAGTTAATAGTTTGCGCAACGTTGTTGCCATTGCTACAGGCATCGT
GGTGTCACGCTCGTCGTTTGGTATGGCTTCATTCAGCTCCGGTTCCCAACGATCAAGGCGAGTTACATGATCCCCCATGTTGT
GCAAAAAAGCGGTTAGCTCCTTCGGTCCTCCGATCGTTGTCAGAAGTAAGTTGGCCGCAGTGTTATCACTCATGGTTATGGCA
GCACTGCATAATTCTCTTACTGTCATGCCATCCGTAAGATGCTTTTCTGTGACTGGTGAGTACTCAACCAAGTCATTCTGAGA
ATAGTGTATGCGGCGACCGAGTTGCTCTTGCCCGGCGTCAATACGGGATAATACCGCGCCACATAGCAGAACTTTAAAAGTGC
TCATCATTGGAAAACGTTCTTCGGGGCGAAAACTCTCAAGGATCTTACCGCTGTTGAGATCCAGTTCGATGTAACCCACTCGT
GCACCCAACTGATCTTCAGCATCTTTTACTTTCACCAGCGTTTCTGGGTGAGCAAAAACAGGAAGGCAAAATGCCGCAAAAAA
 
GGGAATAAGGGCGACACGGAAATGTTGAATACTCATACTCTTCCTTTTTCAATATTATTGAAGCATTTATCAGGGTTATTGTC
TCATGAGCGGATACATATTTGAATGTATTTAGAAAAATAAACAAATAGGGGTTCCGCGCACATTTCCCCGAAAAGTGCCACCT
GACGTCTAAGAAACCATTATTATCATGACATTAACCTATAAAAATAGGCGTATCACGAGGCCCTTAGGCCAAATAGGCCGTTC
AGATCCTTCCGTATTTAGCCAGTATGTTCTCTAGTGTGGTTCGTTGTTTTTGCGTGAGCCATGAGAACGAACCATTGAGATCA
TGCTTACTTTGCATGTCACTCAAAAATTTTGCCTCAAAACTGGTGAGCTGAATTTTTGCAGTTAAAGCATCGTGTAGTGTTTT
TCTTAGTCCGTTACGTAGGTAGGAATCTGATGTAATGGTTGTTGGTATTTTGTCACCATTCATTTTTATCTGGTTGTTCTCAA
GTTCGGTTACGAGATCCATTTGTCTATCTAGTTCAACTTGGAAAATCAACGTATCAGTCGGGCGGCCTCGCTTATCAACCACC
AATTTCATATTGCTGTAAGTGTTTAAATCTTTACTTATTGGTTTCAAAACCCATTGGTTAAGCCTTTTAAACTCATGGTAGTT
ATTTTCAAGCATTAACATGAACTTAAATTCATCAAGGCTAATCTCTATATTTGCCTTGTGAGTTTTCTTTTGTGTTAGTTCTT
TTAATAACCACTCATAAATCCTCATAGAGTATTTGTTTTCAAAAGACTTAACATGTTCCAGATTATATTTTATGAATTTTTTT
AACTGGAAAAGATAAGGCAATATCTCTTCACTAAAAACTAATTCTAATTTTTCGCTTGAGAACTTGGCATAGTTTGTCCACTG
GAAAATCTCAAAGCCTTTAACCAAAGGATTCCTGATTTCCACAGTTCTCGTCATCAGCTCTCTGGTTGCTTTAGCTAATACAC
CATAAGCATTTTCCCTACTGATGTTCATCATCTGAGCGTATTGGTTATAAGTGAACGATACCGTCCGTTCTTTCCTTGTAGGG
TTTTCAATCGTGGGGTTGAGTAGTGCCACACAGCATAAAATTAGCTTGGTTTCATGCTCCGTTAAGTCATAGCGACTAATCGC
TAGTTCATTTGCTTTGAAAACAACTAATTCAGACATACATCTCAATTGGTCTAGGTGATTTTAATCACTATACCAATTGAGAT
GGGCTAGTCAATGATAATTACTAGTCCTTTTCCTTTGAGTTGTGGGTATCTGTAAATTCTGCTAGACCTTTGCTGGAAAACTT
GTAAATTCTGCTAGACCCTCTGTAAATTCCGCTAGACCTTTGTGTGTTTTTTTTGTTTATATTCAAGTGGTTATAATTTATAG
AATAAAGAAAGAATAAAAAAAGATAAAAAGAATAGATCCCAGCCCTGTGTATAACTCACTACTTTAGTCAGTTCCGCAGTATT
ACAAAAGGATGTCGCAAACGCTGTTTGCTCCTCTACAAAACAGACCTTAAAACCCTAAAGGCTTAAGTAGCACCCTCGCAAGC
TCGGGCAAATCGCTGAATATTCCTTTTGTCTCCGACCATCAGGCACCTGAGTCGCTGTCTTTTTCGTGACATTCAGTTCGCTG
CGCTCACGGCTCTGGCAGTGAATGGGGGTAAATGGCACTACAGGCGCCTTTTATGGATTCATGCAAGGAAACTACCCATAATA
CAAGAAAAGCCCGTCACGGGCTTCTCAGGGCGTTTTATGGCGGGTCTGCTATGTGGTGCTATCTGACTTTTTGCTGTTCAGCA
GTTCCTGCCCTCTGATTTTCCAGTCTGACCACTTCGGATTATCCCGTGACAGGTCATTCAGACTGGCTAATGCACCCAGTAAG
GCAGCGGTATCATCAACAGGCTTACCCGTCTTACTGTCAAGAGGACATCCGGTCAGATAACCGTCATCTTCGGCTactgtcta
gggcggcggatttgtcctactcaggagagcgttcaccgacaaacaacagataaaacgaaaggcccagtctttcgactgagcct
ttcgttttatttgatgcaCTAGTgccgatcatattcaataacccttaatataacttcgtataatgtatgctatacgaagttat
tagGTCTGAAGAGGAGTTTACGTCCCATATGGAGTCCCGCTCAGAAGAACTcgtcaagaaggcgatagaaggcgatgcgctgc
gaatcgggagcggcgataccgtaaagcacgaggaagcggtcagcccattcgccgccaagctcttcagcaatatcacgggtagc
caacgctatgtcctgatagcggtccgccacacccagccggccacagtcgatgaatccagaaaagcggccattttccaccatga
tattcggcaagcaggcatcgccatgggtcacgacgagatcctcgccgtcgggcatgcgcgccttgagcctggcgaacagttcg
gcCggcgcgagcccctgatgctcttcgtccagatcatcctgatcgacaagaccggcttccatccgagtacgtgctcgctcgat
gcgatgtttcgcttggtggtcgaatgggcaggtagccggatcaagcgtatgcagccgccgcattgcatcagccatgatggata
ctttctcggcaggagcaaggtgagatgacaggagatcctgccccggcacttcgcccaatagcagccagtcccttcccgcttca
gtgacaacgtcgagcacagctgcgcaaggaacgcccgtcgtggccagccacgatagccgcgctgcctcgtcctgcagttcatt
cagggcaccggacaggtcggtcttgacaaaaagaaccgggcgcccctgcgctgacagccggaacacggcggcatcagagcagc
cgattgtctgttgtgcccagtcatagccgaatagcctctccacccaagcggccggagaacctgcgtgcaatccatcttgttca
atcatgcgaaacgatcctcatcctgtctcttgatcagatcCtgatcccctgcgccatcagatccttggcggcaagaaagccat
ccagtttactttgcagggcttcccaaccttaccagagggcgccccagctggcaattccgACGTCATTGCGTTGCTAGCCAAGC
TTAGGATCCGGAACccttaatataacttcgtataatgtatgctatacgaagttattaggtccctcgaagaggttcactagcta
Tctagagcttggactcctgttgatagatccagtaatgacctcagaactccatctggatttgttcagaacgctcggttgccgcc
gggcgttttttattggtgagaatccaagcccgGGAAacgGGATCCtGatCTTGATCCGCCGCAAAGTTTGAGCGAAGTCAATA
AACTCTCTACCCATTCAGGGCAATATCTCTCTTGCAGGTGAATGCAACGTCAAGCGATGGCCtacttGCGGCCGCaagattAA
AGAGGAGAAGG
SEQ ID NO:34 pET26b-psp-Nb127D01-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
 
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCgaggttcaa
ttagtggaatctggcgggggattggttcaagccggtgagtccttgcgtttgagctgcgccgcatctgggagcacatttgattt
caaagtgatgggctggtatcgtcagccgcccggaaaacagcgcgagggtgtagcagccatccgcttgagcggaaatatgcact
acgccgagtctgtaaaagggcgctttgctatttccaaagcgaacgctaagaacacagtgtatcttcagatgaactccttgcgt
cccgaagacactgccgtttattattgcaaagtaaacatccgtgggcaagactactggggtcaaggaacgcaggtcacagtctc
ttctggctctggctacccctatgacgtgcctgattacgccggctctggccaccaccaccaccaccactgagatccggctgcta
acaaagcccgaaaggaagctgagttggctgctgccaccgctgagcaataactagcataaccccttggggcctctaaacgggtc
ttgaggggttttttgctgaaaggaggaactatatccggat
SEQ ID NO:35 pET26b-psp-Nb163E3-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
 
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGAAAAAGCGTGGATGCTGCTGGTTATGTCGCTGCTGATTGCGGCGGTTCCGATTACGGCGAGCGCCGAGGTGCAG
CTGGTGGAGTCTGGGGGAGGATTGGTGCAGCCTGGGGGCTCTGTGAGACTCTCCTGTGTAGCCTCTGGACGCATCTTCAGTAG
CAATGCCATGGGCTGGTTCCGCCAGGCTCCAGGGAAGGAGCGTGAGTTTGTAGCGGCCATTACCTGGAGGAGTGGCGGTAGCG
CGTACTATGCAGACTCCGCGAAGGGCCGATTCACCATCTCCAGAGACAACGCCAAGAACACGGTGTATTTGCAAATGAACAGC
CTGAAACCTGAGGACACGGCCGTTTATTATTGTGCAGCTGGTGGTAGTTCCTGGTTAAGTTTTCCGCCGGACTACTGGGGCCA
GGGGACCCAGGTCACCGTCTCCTCAtacccctatgacgtgcctgattacgccggctctggccaccaccaccaccaccactgag
atccggctgctaacaaagcccgaaaggaagctgagttggctgctgccaccgctgagcaataactagcataaccccttggggcc
tctaaacgggtcttgaggggttttttgctgaaaggaggaactatatccggat
SEQ ID NO:36 pET26b-psp-Nb97A9-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
 
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCGAGGTGCAG
CTGGTGGAGTCTGGGGGAGGCTTGGTGCAGCCTGGGGGGTCTCTGAGACTCTCCTGTGTAGCCTCTGGAAGCATCGTCAGAAT
TAATACCATGGGCTGGTACCGCCAGACTCCAGGGAAGCAGCGCGAGTTGGTCGCAGATATTACCAGTGGTGGTAACATAAACT
ATATAGACGCCGTGAAGGGCCGATTCACCATCTCCAGAGACAACACCAAGAAGAGGGTGTATCTGCAAATGAACAGCCTGAAA
CCTGAGGACACGGCCGTCTATTACTGTAATGCAGAGATCGTTGTTCTGGTGGGAGTTTGGACCCAGCGTGCGCGGACCGGCAA
CTACTGGGGCCAGGGGACCCAGGTCACCGTCTCCTCAtacccctatgacgtgcctgattacgccggctctggccaccaccacc
accaccactgagatccggctgctaacaaagcccgaaaggaagctgagttggctgctgccaccgctgagcaataactagcataa
ccccttggggcctctaaacgggtcttgaggggttttttgctgaaaggaggaactatatccggat
SEQ ID NO: 43 pET26b-psp-Nb127D01-PE38-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
 
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCgaggttcaa
ttagtggaatctggcgggggattggttcaagccggtgagtccttgcgtttgagctgcgccgcatctgggagcacatttgattt
caaagtgatgggctggtatcgtcagccgcccggaaaacagcgcgagggtgtagcagccatccgcttgagcggaaatatgcact
acgccgagtctgtaaaagggcgctttgctatttccaaagcgaacgctaagaacacagtgtatcttcagatgaactccttgcgt
cccgaagacactgccgtttattattgcaaagtaaacatccgtgggcaagactactggggtcaaggaacgcaggtcacagtctc
ttctggctctggcCCTGAAGGTGGTAGCCTGGCGGCACTGACCGCACACCAAGCATGTCATCTGCCACTGGAAACCTTTACCC
GTCATCGTCAGCCGCGTGGCTGGGAACAACTGGAACAATGTGGATATCCGGTGCAGAGACTGGTCGCATTATATCTGGCAGCA
CGTCTGAGTTGGAATCAGGTTGATCAAGTTATTCGTAACGCACTTGCCTCACCGGGTAGCGGTGGTGATTTAGGTGAGGCAAT
TCGTGAACAACCTGAACAGGCACGCTTAGCATTAACCCTTGCAGCCGCAGAAAGTGAAAGATTTGTGCGTCAGGGTACTGGTA
ATGATGAGGCCGGTGCAGCAAATGGGCCGGCAGACAGCGGAGATGCATTATTAGAGAGAAATTATCCTACCGGCGCAGAATTT
CTGGGTGATGGGGGTGATGTTAGTTTTTCAACACGCGGGACACAGAATTGGACCGTGGAACGTTTACTGCAGGCACATCGTCA
GCTGGAAGAACGCGGTTATGTTTTTGTTGGTTATCATGGGACGTTTCTGGAGGCAGCACAGAGCATTGTCTTTGGCGGTGTGC
GGGCGCGTAGCCAGGATCTTGATGCAATTTGGCGTGGTTTTTATATTGCGGGTGATCCTGCGCTGGCGTATGGATATGCGCAG
GATCAGGAGCCGGATGCGCGTGGTCGTATTCGTAATGGTGCCCTGCTGCGGGTTTATGTTCCGCGTAGCTCACTGCCGGGGTT
TTATAGAACAAGCTTAACACTGGCTGCGCCGGAAGCAGCAGGTGAAGTTGAACGTCTGATTGGCCATCCGCTGCCGTTACGTT
TAGATGCAATTACTGGTCCGGAAGAAGAAGGTGGTCGCCTGGAAACCATTCTGGGTTGGCCGCTGGCAGAAAGAACCGTGGTT
ATACCTTCCGCAATACCGACCGATCCTCGGAATGTGGGGGGTGATTTAGATCCTAGCAGCATTCCTGATAAAGAACAGGCAAT
TTCTGCTCTGCCTGATTATGCATCACAGCCGGGTAAACCGCCGCGTGAAGATTTAAAGCATCATCACCATCATCATTAAgatc
cggctgctaacaaagcccgaaaggaagctgagttggctgctgccaccgctgagcaataactagcataaccccttggggcctct
aaacgggtcttgaggggttttttgctgaaaggaggaactatatccggat
SEQ ID NO: 44 pET26b-psp-Nb163E3-PE38-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
 
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCGAGGTGCAG
CTGGTGGAGTCTGGGGGAGGATTGGTGCAGCCTGGGGGCTCTGTGAGACTCTCCTGTGTAGCCTCTGGACGCATCTTCAGTAG
CAATGCCATGGGCTGGTTCCGCCAGGCTCCAGGGAAGGAGCGTGAGTTTGTAGCGGCCATTACCTGGAGGAGTGGCGGTAGCG
CGTACTATGCAGACTCCGCGAAGGGCCGATTCACCATCTCCAGAGACAACGCCAAGAACACGGTGTATTTGCAAATGAACAGC
CTGAAACCTGAGGACACGGCCGTTTATTATTGTGCAGCTGGTGGTAGTTCCTGGTTAAGTTTTCCGCCGGACTACTGGGGCCA
GGGGACCCAGGTCACCGTCTCCTCACCTGAAGGTGGTAGCCTGGCGGCACTGACCGCACACCAAGCATGTCATCTGCCACTGG
AAACCTTTACCCGTCATCGTCAGCCGCGTGGCTGGGAACAACTGGAACAATGTGGATATCCGGTGCAGAGACTGGTCGCATTA
TATCTGGCAGCACGTCTGAGTTGGAATCAGGTTGATCAAGTTATTCGTAACGCACTTGCCTCACCGGGTAGCGGTGGTGATTT
AGGTGAGGCAATTCGTGAACAACCTGAACAGGCACGCTTAGCATTAACCCTTGCAGCCGCAGAAAGTGAAAGATTTGTGCGTC
AGGGTACTGGTAATGATGAGGCCGGTGCAGCAAATGGGCCGGCAGACAGCGGAGATGCATTATTAGAGAGAAATTATCCTACC
GGCGCAGAATTTCTGGGTGATGGGGGTGATGTTAGTTTTTCAACACGCGGGACACAGAATTGGACCGTGGAACGTTTACTGCA
GGCACATCGTCAGCTGGAAGAACGCGGTTATGTTTTTGTTGGTTATCATGGGACGTTTCTGGAGGCAGCACAGAGCATTGTCT
TTGGCGGTGTGCGGGCGCGTAGCCAGGATCTTGATGCAATTTGGCGTGGTTTTTATATTGCGGGTGATCCTGCGCTGGCGTAT
GGATATGCGCAGGATCAGGAGCCGGATGCGCGTGGTCGTATTCGTAATGGTGCCCTGCTGCGGGTTTATGTTCCGCGTAGCTC
ACTGCCGGGGTTTTATAGAACAAGCTTAACACTGGCTGCGCCGGAAGCAGCAGGTGAAGTTGAACGTCTGATTGGCCATCCGC
TGCCGTTACGTTTAGATGCAATTACTGGTCCGGAAGAAGAAGGTGGTCGCCTGGAAACCATTCTGGGTTGGCCGCTGGCAGAA
AGAACCGTGGTTATACCTTCCGCAATACCGACCGATCCTCGGAATGTGGGGGGTGATTTAGATCCTAGCAGCATTCCTGATAA
AGAACAGGCAATTTCTGCTCTGCCTGATTATGCATCACAGCCGGGTAAACCGCCGCGTGAAGATTTAAAGCATCATCACCATC
ATCATTAAgatccggctgctaacaaagcccgaaaggaagctgagttggctgctgccaccgctgagcaataactagcataaccc
cttggggcctctaaacgggtcttgaggggttttttgctgaaaggaggaactatatccggat
SEQ ID NO: 45 pET26b-psp-Nb97A9-PE38-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
 
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCGAGGTGCAG
CTGGTGGAGTCTGGGGGAGGCTTGGTGCAGCCTGGGGGGTCTCTGAGACTCTCCTGTGTAGCCTCTGGAAGCATCGTCAGAAT
TAATACCATGGGCTGGTACCGCCAGACTCCAGGGAAGCAGCGCGAGTTGGTCGCAGATATTACCAGTGGTGGTAACATAAACT
ATATAGACGCCGTGAAGGGCCGATTCACCATCTCCAGAGACAACACCAAGAAGAGGGTGTATCTGCAAATGAACAGCCTGAAA
CCTGAGGACACGGCCGTCTATTACTGTAATGCAGAGATCGTTGTTCTGGTGGGAGTTTGGACCCAGCGTGCGCGGACCGGCAA
CTACTGGGGCCAGGGGACCCAGGTCACCGTCTCCTCACCTGAAGGTGGTAGCCTGGCGGCACTGACCGCACACCAAGCATGTC
ATCTGCCACTGGAAACCTTTACCCGTCATCGTCAGCCGCGTGGCTGGGAACAACTGGAACAATGTGGATATCCGGTGCAGAGA
CTGGTCGCATTATATCTGGCAGCACGTCTGAGTTGGAATCAGGTTGATCAAGTTATTCGTAACGCACTTGCCTCACCGGGTAG
CGGTGGTGATTTAGGTGAGGCAATTCGTGAACAACCTGAACAGGCACGCTTAGCATTAACCCTTGCAGCCGCAGAAAGTGAAA
GATTTGTGCGTCAGGGTACTGGTAATGATGAGGCCGGTGCAGCAAATGGGCCGGCAGACAGCGGAGATGCATTATTAGAGAGA
AATTATCCTACCGGCGCAGAATTTCTGGGTGATGGGGGTGATGTTAGTTTTTCAACACGCGGGACACAGAATTGGACCGTGGA
ACGTTTACTGCAGGCACATCGTCAGCTGGAAGAACGCGGTTATGTTTTTGTTGGTTATCATGGGACGTTTCTGGAGGCAGCAC
AGAGCATTGTCTTTGGCGGTGTGCGGGCGCGTAGCCAGGATCTTGATGCAATTTGGCGTGGTTTTTATATTGCGGGTGATCCT
GCGCTGGCGTATGGATATGCGCAGGATCAGGAGCCGGATGCGCGTGGTCGTATTCGTAATGGTGCCCTGCTGCGGGTTTATGT
TCCGCGTAGCTCACTGCCGGGGTTTTATAGAACAAGCTTAACACTGGCTGCGCCGGAAGCAGCAGGTGAAGTTGAACGTCTGA
TTGGCCATCCGCTGCCGTTACGTTTAGATGCAATTACTGGTCCGGAAGAAGAAGGTGGTCGCCTGGAAACCATTCTGGGTTGG
CCGCTGGCAGAAAGAACCGTGGTTATACCTTCCGCAATACCGACCGATCCTCGGAATGTGGGGGGTGATTTAGATCCTAGCAG
CATTCCTGATAAAGAACAGGCAATTTCTGCTCTGCCTGATTATGCATCACAGCCGGGTAAACCGCCGCGTGAAGATTTAAAGC
ATCATCACCATCATCATTAAgatccggctgctaacaaagcccgaaaggaagctgagttggctgctgccaccgctgagcaataa
ctagcataaccccttggggcctctaaacgggtcttgaggggttttttgctgaaaggaggaactatatccggat
SEQ ID NO: 46 pET26b-psp-Nb127D01-PE[LR]-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
 
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCgaggttcaa
ttagtggaatctggcgggggattggttcaagccggtgagtccttgcgtttgagctgcgccgcatctgggagcacatttgattt
caaagtgatgggctggtatcgtcagccgcccggaaaacagcgcgagggtgtagcagccatccgcttgagcggaaatatgcact
acgccgagtctgtaaaagggcgctttgctatttccaaagcgaacgctaagaacacagtgtatcttcagatgaactccttgcgt
cccgaagacactgccgtttattattgcaaagtaaacatccgtgggcaagactactggggtcaaggaacgcaggtcacagtctc
ttctggctctggcAGACACCGCCAGCCGAGAGGTTGGGAACAGCTGCCGACCGGAGCTGAATTTCTGGGCGATGGTGGGGATG
TTAGTTTTTCTACCCGTGGCACACAGAATTGGACCGTTGAACGGTTATTACAAGCACATCGTCAGCTGGAAGAAAGAGGTTAT
GTGTTTGTTGGGTATCATGGTACATTTTTAGAAGCAGCCCAGTCTATTGTTTTTGGCGGTGTTCGTGCCCGTAGCCAGGATCT
GGATGCAATTTGGAGAGGTTTTTATATTGCGGGTGATCCGGCGCTGGCATATGGTTACGCACAGGATCAGGAACCAGATGCAC
GTGGTCGTATTCGTAATGGTGCCCTGTTACGGGTGTATGTTCCCCGTTCCAGCCTGCCGGGTTTTTATCGTACCTCTTTAACA
CTGGCAGCCCCGGAAGCAGCAGGTGAAGTGGAACGTCTGATTGGCCATCCGCTGCCGCTGCGCTTAGATGCAATTACCGGTCC
GGAAGAAGAAGGTGGGCGGTTAGAAACCATTCTGGGTTGGCCGCTGGCAGAAAGAACAGTGGTTATACCGAGCGCGATTCCTA
CCGATCCTCGTAATGTGGGGGGTGATTTAGATCCGAGCAGCATTCCGGATAAAGAGCAGGCGATTAGCGCGCTGCCGGATTAT
GCAAGCCAACCGGGGAAACCGCCGCGTGAAGACTTAAAACATCATCACCATCATCATTAAgatccggctgctaacaaagcccg
 
aaaggaagctgagttggctgctgccaccgctgagcaataactagcataaccccttggggcctctaaacgggtcttgaggggtt
ttttgctgaaaggaggaactatatccggat
SEQ ID NO: 47 pET26b-psp-Nb163E3-PE[LR]-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCGAGGTGCAG
CTGGTGGAGTCTGGGGGAGGATTGGTGCAGCCTGGGGGCTCTGTGAGACTCTCCTGTGTAGCCTCTGGACGCATCTTCAGTAG
CAATGCCATGGGCTGGTTCCGCCAGGCTCCAGGGAAGGAGCGTGAGTTTGTAGCGGCCATTACCTGGAGGAGTGGCGGTAGCG
CGTACTATGCAGACTCCGCGAAGGGCCGATTCACCATCTCCAGAGACAACGCCAAGAACACGGTGTATTTGCAAATGAACAGC
CTGAAACCTGAGGACACGGCCGTTTATTATTGTGCAGCTGGTGGTAGTTCCTGGTTAAGTTTTCCGCCGGACTACTGGGGCCA
GGGGACCCAGGTCACCGTCTCCTCAAGACACCGCCAGCCGAGAGGTTGGGAACAGCTGCCGACCGGAGCTGAATTTCTGGGCG
ATGGTGGGGATGTTAGTTTTTCTACCCGTGGCACACAGAATTGGACCGTTGAACGGTTATTACAAGCACATCGTCAGCTGGAA
GAAAGAGGTTATGTGTTTGTTGGGTATCATGGTACATTTTTAGAAGCAGCCCAGTCTATTGTTTTTGGCGGTGTTCGTGCCCG
 
TAGCCAGGATCTGGATGCAATTTGGAGAGGTTTTTATATTGCGGGTGATCCGGCGCTGGCATATGGTTACGCACAGGATCAGG
AACCAGATGCACGTGGTCGTATTCGTAATGGTGCCCTGTTACGGGTGTATGTTCCCCGTTCCAGCCTGCCGGGTTTTTATCGT
ACCTCTTTAACACTGGCAGCCCCGGAAGCAGCAGGTGAAGTGGAACGTCTGATTGGCCATCCGCTGCCGCTGCGCTTAGATGC
AATTACCGGTCCGGAAGAAGAAGGTGGGCGGTTAGAAACCATTCTGGGTTGGCCGCTGGCAGAAAGAACAGTGGTTATACCGA
GCGCGATTCCTACCGATCCTCGTAATGTGGGGGGTGATTTAGATCCGAGCAGCATTCCGGATAAAGAGCAGGCGATTAGCGCG
CTGCCGGATTATGCAAGCCAACCGGGGAAACCGCCGCGTGAAGACTTAAAACATCATCACCATCATCATTAAgatccggctgc
taacaaagcccgaaaggaagctgagttggctgctgccaccgctgagcaataactagcataaccccttggggcctctaaacggg
tcttgaggggttttttgctgaaaggaggaactatatccggat
SEQ ID NO: 48 pET26b-psp-Nb97A9-PE[LR]-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatATGGGCCTGAAAATGAAAAAAC
GTAGCGGGAAAAAAGCGTGGATGCTGCTGGTTATGTCGCTGCTGATTGCGGCGGTTCCGATTACGGCGAGCGCCGAGGTGCAG
CTGGTGGAGTCTGGGGGAGGCTTGGTGCAGCCTGGGGGGTCTCTGAGACTCTCCTGTGTAGCCTCTGGAAGCATCGTCAGAAT
 
TAATACCATGGGCTGGTACCGCCAGACTCCAGGGAAGCAGCGCGAGTTGGTCGCAGATATTACCAGTGGTGGTAACATAAACT
ATATAGACGCCGTGAAGGGCCGATTCACCATCTCCAGAGACAACACCAAGAAGAGGGTGTATCTGCAAATGAACAGCCTGAAA
CCTGAGGACACGGCCGTCTATTACTGTAATGCAGAGATCGTTGTTCTGGTGGGAGTTTGGACCCAGCGTGCGCGGACCGGCAA
CTACTGGGGCCAGGGGACCCAGGTCACCGTCTCCTCAAGACACCGCCAGCCGAGAGGTTGGGAACAGCTGCCGACCGGAGCTG
AATTTCTGGGCGATGGTGGGGATGTTAGTTTTTCTACCCGTGGCACACAGAATTGGACCGTTGAACGGTTATTACAAGCACAT
CGTCAGCTGGAAGAAAGAGGTTATGTGTTTGTTGGGTATCATGGTACATTTTTAGAAGCAGCCCAGTCTATTGTTTTTGGCGG
TGTTCGTGCCCGTAGCCAGGATCTGGATGCAATTTGGAGAGGTTTTTATATTGCGGGTGATCCGGCGCTGGCATATGGTTACG
CACAGGATCAGGAACCAGATGCACGTGGTCGTATTCGTAATGGTGCCCTGTTACGGGTGTATGTTCCCCGTTCCAGCCTGCCG
GGTTTTTATCGTACCTCTTTAACACTGGCAGCCCCGGAAGCAGCAGGTGAAGTGGAACGTCTGATTGGCCATCCGCTGCCGCT
GCGCTTAGATGCAATTACCGGTCCGGAAGAAGAAGGTGGGCGGTTAGAAACCATTCTGGGTTGGCCGCTGGCAGAAAGAACAG
TGGTTATACCGAGCGCGATTCCTACCGATCCTCGTAATGTGGGGGGTGATTTAGATCCGAGCAGCATTCCGGATAAAGAGCAG
GCGATTAGCGCGCTGCCGGATTATGCAAGCCAACCGGGGAAACCGCCGCGTGAAGACTTAAAACATCATCACCATCATCATTA
Agatccggctgctaacaaagcccgaaaggaagctgagttggctgctgccaccgctgagcaataactagcataaccccttgggg
cctctaaacgggtcttgaggggttttttgctgaaaggaggaactatatccggat
SEQ ID NO: 49 pSC101-pFNRsp-psp-Nb127D01-PE38-HA-his 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCGCaagattAAAGAGGAGAAGGATGGGCCTGAAAATGAAAAAACGTAGCGG
GAAAAAAGCGTGGATGCTGCTGGTTATGTCGCTGCTGATTGCGGCGGTTCCGATTACGGCGAGCGCCgaggttcaattagtgg
aatctggcgggggattggttcaagccggtgagtccttgcgtttgagctgcgccgcatctgggagcacatttgatttcaaagtg
atgggctggtatcgtcagccgcccggaaaacagcgcgagggtgtagcagccatccgcttgagcggaaatatgcactacgccga
gtctgtaaaagggcgctttgctatttccaaagcgaacgctaagaacacagtgtatcttcagatgaactccttgcgtcccgaag
 
acactgccgtttattattgcaaagtaaacatccgtgggcaagactactggggtcaaggaacgcaggtcacagtctcttctggc
tctggcCCTGAAGGTGGTAGCCTGGCGGCACTGACCGCACACCAAGCATGTCATCTGCCACTGGAAACCTTTACCCGTCATCG
TCAGCCGCGTGGCTGGGAACAACTGGAACAATGTGGATATCCGGTGCAGAGACTGGTCGCATTATATCTGGCAGCACGTCTGA
GTTGGAATCAGGTTGATCAAGTTATTCGTAACGCACTTGCCTCACCGGGTAGCGGTGGTGATTTAGGTGAGGCAATTCGTGAA
CAACCTGAACAGGCACGCTTAGCATTAACCCTTGCAGCCGCAGAAAGTGAAAGATTTGTGCGTCAGGGTACTGGTAATGATGA
GGCCGGTGCAGCAAATGGGCCGGCAGACAGCGGAGATGCATTATTAGAGAGAAATTATCCTACCGGCGCAGAATTTCTGGGTG
ATGGGGGTGATGTTAGTTTTTCAACACGCGGGACACAGAATTGGACCGTGGAACGTTTACTGCAGGCACATCGTCAGCTGGAA
GAACGCGGTTATGTTTTTGTTGGTTATCATGGGACGTTTCTGGAGGCAGCACAGAGCATTGTCTTTGGCGGTGTGCGGGCGCG
TAGCCAGGATCTTGATGCAATTTGGCGTGGTTTTTATATTGCGGGTGATCCTGCGCTGGCGTATGGATATGCGCAGGATCAGG
AGCCGGATGCGCGTGGTCGTATTCGTAATGGTGCCCTGCTGCGGGTTTATGTTCCGCGTAGCTCACTGCCGGGGTTTTATAGA
ACAAGCTTAACACTGGCTGCGCCGGAAGCAGCAGGTGAAGTTGAACGTCTGATTGGCCATCCGCTGCCGTTACGTTTAGATGC
AATTACTGGTCCGGAAGAAGAAGGTGGTCGCCTGGAAACCATTCTGGGTTGGCCGCTGGCAGAAAGAACCGTGGTTATACCTT
CCGCAATACCGACCGATCCTCGGAATGTGGGGGGTGATTTAGATCCTAGCAGCATTCCTGATAAAGAACAGGCAATTTCTGCT
CTGCCTGATTATGCATCACAGCCGGGTAAACCGCCGCGTGAAGATTTAAAGCATCATCACCATCATCATTAAgatccggggctct
ggaagatccgcgcgtaccgagttctaattcactggccgtcgttttacaacatacgatgaaggcgataagtcgcgtaagTTGCT
AGCccacgcGGCTCACCTTCACGGGTGGGCCTTTCTTCGCGATCCAcatg
SEQ ID NO: 50 pSC101-pFNRsp-psp-Nb163E3-PE38-HA-his 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCGCaagattAAAGAGGAGAAGGATGGGCCTGAAAATGAAAAAACGTAGCGG
GAAAAAAGCGTGGATGCTGCTGGTTATGTCGCTGCTGATTGCGGCGGTTCCGATTACGGCGAGCGCCGAGGTGCAGCTGGTGG
AGTCTGGGGGAGGATTGGTGCAGCCTGGGGGCTCTGTGAGACTCTCCTGTGTAGCCTCTGGACGCATCTTCAGTAGCAATGCC
 
ATGGGCTGGTTCCGCCAGGCTCCAGGGAAGGAGCGTGAGTTTGTAGCGGCCATTACCTGGAGGAGTGGCGGTAGCGCGTACTA
TGCAGACTCCGCGAAGGGCCGATTCACCATCTCCAGAGACAACGCCAAGAACACGGTGTATTTGCAAATGAACAGCCTGAAAC
CTGAGGACACGGCCGTTTATTATTGTGCAGCTGGTGGTAGTTCCTGGTTAAGTTTTCCGCCGGACTACTGGGGCCAGGGGACC
CAGGTCACCGTCTCCTCACCTGAAGGTGGTAGCCTGGCGGCACTGACCGCACACCAAGCATGTCATCTGCCACTGGAAACCTT
TACCCGTCATCGTCAGCCGCGTGGCTGGGAACAACTGGAACAATGTGGATATCCGGTGCAGAGACTGGTCGCATTATATCTGG
CAGCACGTCTGAGTTGGAATCAGGTTGATCAAGTTATTCGTAACGCACTTGCCTCACCGGGTAGCGGTGGTGATTTAGGTGAG
GCAATTCGTGAACAACCTGAACAGGCACGCTTAGCATTAACCCTTGCAGCCGCAGAAAGTGAAAGATTTGTGCGTCAGGGTAC
TGGTAATGATGAGGCCGGTGCAGCAAATGGGCCGGCAGACAGCGGAGATGCATTATTAGAGAGAAATTATCCTACCGGCGCAG
AATTTCTGGGTGATGGGGGTGATGTTAGTTTTTCAACACGCGGGACACAGAATTGGACCGTGGAACGTTTACTGCAGGCACAT
CGTCAGCTGGAAGAACGCGGTTATGTTTTTGTTGGTTATCATGGGACGTTTCTGGAGGCAGCACAGAGCATTGTCTTTGGCGG
TGTGCGGGCGCGTAGCCAGGATCTTGATGCAATTTGGCGTGGTTTTTATATTGCGGGTGATCCTGCGCTGGCGTATGGATATG
CGCAGGATCAGGAGCCGGATGCGCGTGGTCGTATTCGTAATGGTGCCCTGCTGCGGGTTTATGTTCCGCGTAGCTCACTGCCG
GGGTTTTATAGAACAAGCTTAACACTGGCTGCGCCGGAAGCAGCAGGTGAAGTTGAACGTCTGATTGGCCATCCGCTGCCGTT
ACGTTTAGATGCAATTACTGGTCCGGAAGAAGAAGGTGGTCGCCTGGAAACCATTCTGGGTTGGCCGCTGGCAGAAAGAACCG
TGGTTATACCTTCCGCAATACCGACCGATCCTCGGAATGTGGGGGGTGATTTAGATCCTAGCAGCATTCCTGATAAAGAACAG
GCAATTTCTGCTCTGCCTGATTATGCATCACAGCCGGGTAAACCGCCGCGTGAAGATTTAAAGCATCATCACCATCATCATTA
Agatccggctctggaagatccgcgcgtaccgagttctaattcactggccgtcgttttacaacatacgatgaaggcgataagtc
gcgtaagTTGCTAGCccacgcGGCTCACCTTCACGGGTGGGCCTTTCTTCGCGATCCAcatg
SEQ ID NO: 51 pSC101-pFNRsp-psp-Nb97A9-PE38-HA-his 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCGCaagattAAAGAGGAGAAGGATGGGCCTGAAAATGAAAAAACGTAGCGG
 
GAAAAAAGCGTGGATGCTGCTGGTTATGTCGCTGCTGATTGCGGCGGTTCCGATTACGGCGAGCGCCGAGGTGCAGCTGGTGG
AGTCTGGGGGAGGCTTGGTGCAGCCTGGGGGGTCTCTGAGACTCTCCTGTGTAGCCTCTGGAAGCATCGTCAGAATTAATACC
ATGGGCTGGTACCGCCAGACTCCAGGGAAGCAGCGCGAGTTGGTCGCAGATATTACCAGTGGTGGTAACATAAACTATATAGA
CGCCGTGAAGGGCCGATTCACCATCTCCAGAGACAACACCAAGAAGAGGGTGTATCTGCAAATGAACAGCCTGAAACCTGAGG
ACACGGCCGTCTATTACTGTAATGCAGAGATCGTTGTTCTGGTGGGAGTTTGGACCCAGCGTGCGCGGACCGGCAACTACTGG
GGCCAGGGGACCCAGGTCACCGTCTCCTCCCTGAAGGTGGTAGCCTGGCGGCACTGACCGCACACCAAGCATGTCATCTGCCA
CTGGAAACCTTTACCCGTCATCGTCAGCCGCGTGGCTGGGAACAACTGGAACAATGTGGATATCCGGTGCAGAGACTGGTCGC
ATTATATCTGGCAGCACGTCTGAGTTGGAATCAGGTTGATCAAGTTATTCGTAACGCACTTGCCTCACCGGGTAGCGGTGGTG
ATTTAGGTGAGGCAATTCGTGAACAACCTGAACAGGCACGCTTAGCATTAACCCTTGCAGCCGCAGAAAGTGAAAGATTTGTG
CGTCAGGGTACTGGTAATGATGAGGCCGGTGCAGCAAATGGGCCGGCAGACAGCGGAGATGCATTATTAGAGAGAAATTATCC
TACCGGCGCAGAATTTCTGGGTGATGGGGGTGATGTTAGTTTTTCAACACGCGGGACACAGAATTGGACCGTGGAACGTTTAC
TGCAGGCACATCGTCAGCTGGAAGAACGCGGTTATGTTTTTGTTGGTTATCATGGGACGTTTCTGGAGGCAGCACAGAGCATT
GTCTTTGGCGGTGTGCGGGCGCGTAGCCAGGATCTTGATGCAATTTGGCGTGGTTTTTATATTGCGGGTGATCCTGCGCTGGC
GTATGGATATGCGCAGGATCAGGAGCCGGATGCGCGTGGTCGTATTCGTAATGGTGCCCTGCTGCGGGTTTATGTTCCGCGTA
GCTCACTGCCGGGGTTTTATAGAACAAGCTTAACACTGGCTGCGCCGGAAGCAGCAGGTGAAGTTGAACGTCTGATTGGCCAT
CCGCTGCCGTTACGTTTAGATGCAATTACTGGTCCGGAAGAAGAAGGTGGTCGCCTGGAAACCATTCTGGGTTGGCCGCTGGC
AGAAAGAACCGTGGTTATACCTTCCGCAATACCGACCGATCCTCGGAATGTGGGGGGTGATTTAGATCCTAGCAGCATTCCTG
ATAAAGAACAGGCAATTTCTGCTCTGCCTGATTATGCATCACAGCCGGGTAAACCGCCGCGTGAAGATTTAAAGCATCATCAC
CATCATCATTAAgatccggctctggaagatccgcgcgtaccgagttctaattcactggccgtcgttttacaacatacgatgaa
ggcgataagtcgcgtaagTTGCTAGCccacgcGGCTCACCTTCACGGGTGGGCCTTTCTTCGCGATCCAcatg
SEQ ID NO: 52 pSC101-pFNRsp-psp-Nb127D01-PE[LR]-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
 
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccgaggttcaattagtggaatctggcgggggattggttcaa
gccggtgagtccttgcgtttgagctgcgccgcatctgggagcacatttgatttcaaagtgatgggctggtatcgtcagccgcc
cggaaaacagcgcgagggtgtagcagccatccgcttgagcggaaatatgcactacgccgagtctgtaaaagggcgctttgcta
tttccaaagcgaacgctaagaacacagtgtatcttcagatgaactccttgcgtcccgaagacactgccgtttattattgcaaa
gtaaacatccgtgggcaagactactggggtcaaggaacgcaggtcacagtctcttctggctctggcAGACACCGCCAGCCGAG
AGGTTGGGAACAGCTGCCGACCGGAGCTGAATTTCTGGGCGATGGTGGGGATGTTAGTTTTTCTACCCGTGGCACACAGAATT
GGACCGTTGAACGGTTATTACAAGCACATCGTCAGCTGGAAGAAAGAGGTTATGTGTTTGTTGGGTATCATGGTACATTTTTA
GAAGCAGCCCAGTCTATTGTTTTTGGCGGTGTTCGTGCCCGTAGCCAGGATCTGGATGCAATTTGGAGAGGTTTTTATATTGC
GGGTGATCCGGCGCTGGCATATGGTTACGCACAGGATCAGGAACCAGATGCACGTGGTCGTATTCGTAATGGTGCCCTGTTAC
GGGTGTATGTTCCCCGTTCCAGCCTGCCGGGTTTTTATCGTACCTCTTTAACACTGGCAGCCCCGGAAGCAGCAGGTGAAGTG
GAACGTCTGATTGGCCATCCGCTGCCGCTGCGCTTAGATGCAATTACCGGTCCGGAAGAAGAAGGTGGGCGGTTAGAAACCAT
TCTGGGTTGGCCGCTGGCAGAAAGAACAGTGGTTATACCGAGCGCGATTCCTACCGATCCTCGTAATGTGGGGGGTGATTTAG
ATCCGACCACCATTCCCGATAAACAGCAGGCCATTACCGCCCTGCCCGATTATGCAAGCCAACCGGCAAACCGCCCCGTGAA
GACTTAAAACATCATCACCATCATCATTAAgatccggctgctaacaaagcccgaaaggaagctgagttggctgctgccaccgc
tgagcaataactagcataaccccttggggcctctaaacgggtcttgaggggttttttgctgaaaggaggaactatatccggat
SEQ ID NO: 53 pSC101-pFNRsp-psp-Nb163E3-PE[LR]-HA-his 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
 
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCGCaagattAAAGAGGAGAAGGATGGGCCTGAAAATGAAAAAACGTAGCGG
GAAAAAAGCGTGGATGCTGCTGGTTATGTCGCTGCTGATTGCGGCGGTTCCGATTACGGCGAGCGCCGAGGTGCAGCTGGTGG
AGTCTGGGGGAGGATTGGTGCAGCCTGGGGGCTCTGTGAGACTCTCCTGTGTAGCCTCTGGACGCATCTTCAGTAGCAATGCC
ATGGGCTGGTTCCGCCAGGCTCCAGGGAAGGAGCGTGAGTTTGTAGCGGCCATTACCTGGAGGAGTGGCGGTAGCGCGTACTA
TGCAGACTCCGCGAAGGGCCGATTCACCATCTCCAGAGACAACGCCAAGAACACGGTGTATTTGCAAATGAACAGCCTGAAAC
CTGAGGACACGGCCGTTTATTATTGTGCAGCTGGTGGTAGTTCCTGGTTAAGTTTTCCGCCGGACTACTGGGGCCAGGGGACC
CAGGTCACCGTCTCCTCAAGACACCGCCAGCCGAGAGGTTGGGAACAGCTGCCGACCGGAGCTGAATTTCTGGGCGATGGTGG
GGATGTTAGTTTTTCTACCCGTGGCACACAGAATTGGACCGTTGAACGGTTATTACAAGCACATCGTCAGCTGGAAGAAAGAG
GTTATGTGTTTGTTGGGTATCATGGTACATTTTTAGAAGCAGCCCAGTCTATTGTTTTTGGCGGTGTTCGTGCCCGTAGCCAG
GATCTGGATGCAATTTGGAGAGGTTTTTATATTGCGGGTGATCCGGCGCTGGCATATGGTTACGCACAGGATCAGGAACCAGA
TGCACGTGGTCGTATTCGTAATGGTGCCCTGTTACGGGTGTATGTTCCCCGTTCCAGCCTGCCGGGTTTTTATCGTACCTCTT
TAACACTGGCAGCCCCGGAAGCAGCAGGTGAAGTGGAACGTCTGATTGGCCATCCGCTGCCGCTGCGCTTAGATGCAATTACC
GGTCCGGAAGAAGAAGGTGGGCGGTTAGAAACCATTCTGGGTTGGCCGCTGGCAGAAAGAACAGTGGTTATACCGAGCGCGAT
TCCTACCGATCCTCGTAATGTGGGGGGTGATTTAGATCCGAGCAGCATTCCGGATAAAGAGCAGGCGATTAGCGCGCTGCCGG
ATTATGCAAGCCAACCGGGGAAACCGCCGCGTGAAGACTTAAAACATCATCACCATCATCATTAAgatccggctctggaagat
ccgcgcgtaccgagttctaattcactggccgtcgttttacaacatacgatgaaggcgataagtcgcgtaagTTGCTAGCccac
gcGGCTCACCTTCACGGGTGGGCCTTTCTTCGCGATCCAcatg
SEQ ID NO: 54 pSC101-pFNRsp-psp-Nb97A9-PE[LR]-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
 
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAGGTGCAGCTGGTGGAGTCTGGGGGAGGCTTGGTGCAG
CCTGGGGGGTCTCTGAGACTCTCCTGTGTAGCCTCTGGAAGCATCGTCAGAATTAATACCATGGGCTGGTACCGCCAGACTCC
AGGGAAGCAGCGCGAGTTGGTCGCAGATATTACCAGTGGTGGTAACATAAACTATATAGACGCCGTGAAGGGCCGATTCACCA
TCTCCAGAGACAACACCAAGAAGAGGGTGTATCTGCAAATGAACAGCCTGAAACCTGAGGACACGGCCGTCTATTACTGTAAT
GCAGAGATCGTTGTTCTGGTGGGAGTTTGGACCCAGCGTGCGCGGACCGGCAACTACTGGGGCCAGGGGACCCAGGTCACCGT
CTCCTCAAGACACCGCCAGCCGAGAGGTTGGGAACAGCTGCCGACCGGAGCTGAATTTCTGGGCGATGGTGGGGATGTTAGTT
TTTCTACCCGTGGCACACAGAATTGGACCGTTGAACGGTTATTACAAGCACATCGTCAGCTGGAAGAAAGAGGTTATGTGTTT
GTTGGGTATCATGGTACATTTTTAGAAGCAGCCCAGTCTATTGTTTTTGGCGGTGTTCGTGCCCGTAGCCAGGATCTGGATGC
AATTTGGAGAGGTTTTTATATTGCGGGTGATCCGGCGCTGGCATATGGTTACGCACAGGATCAGGAACCAGATGCACGTGGTC
GTATTCGTAATGGTGCCCTGTTACGGGTGTATGTTCCCCGTTCCAGCCTGCCGGGTTTTTATCGTACCTCTTTAACACTGGCA
GCCCCGGAAGCAGCAGGTGAAGTGGAACGTCTGATTGGCCATCCGCTGCCGCTGCGCTTAGATGCAATTACCGGTCCGGAAGA
AGAAGGTGGGCGGTTAGAAACCATTCTGGGTTGGCCGCTGGCAGAAAGAACAGTGGTTATACCGAGCGCGATTCCTACCGATC
CTCGTAATGTGGGGGGTGATTTAGATCCGAGCAGCATTCCGGATAAAGAGCAGGCGATTAGCGCGCTGCCGGATTATGCAAGC
CAACCGGGGAAACCGCCGCGTGAAGACTTAAAACATCATCACCATCATCATTAAgatccggctgctaacaaagcccgaaagga
agctgagttggctgctgccaccgctgagcaataactagcataaccccttggggcctctaaacgggtcttgaggggttttttgc
tgaaaggaggaactatatccggat
SEQ ID NO: 55 Fragment.FOR
   GATTACGGCGAGCGCCGAGGTGCAGCTGGTGGAG
SEQ ID NO: 56 Fragment.REV
   GCCAGGCTACCACCTTCAGGTGAGGAGACGGTGACCTGG
SEQ ID NO: 57: dapE (encoding succinyl-diaminoheptanedioate desuccinylase, Gene ID: 1254005) 
ATGTCGTGCCCGGTTATTGAGCTGACACAGCAACTTATTCGTCGCCCCTCCCTGAGTCCGGATGACGCAGGCTGTCAGGCATT
AATGATTGAACGCCTGCGTAAAATCGGTTTTACCATTGAGCATATGGATTTTGGCGACACGCAAAATTTTTGGGCATGGCGTG
GGCGTGGCGAAACGCTGGCGTTCGCCGGGCATACTGACGTCGTACCGGCTGGCGATGTCGATCGCTGGATCAATCCGCCGTTT
GAGCCTACGATTCGCGACGGAATGTTGTTCGGTCGCGGCGCGGCGGATATGAAAGGTTCACTGGCGGCCATGGTGGTGGCTGC
TGAACGTTTTGTTGCGCAGCATCCTCATCACCGGGGCCGCCTCGCATTTTTGATTACCTCTGATGAGGAAGCCAGCGCAAAAA
ATGGCACCGTAAAGGTGGTTGAAGCGCTGATGGCCCGTAACGAGCGGCTGGATTACTGTCTGGTCGGCGAACCTTCCAGTACT
GAAATTGTGGGCGATGTCGTCAAAAACGGGCGTCGCGGTTCTCTCACCTGTAACCTCACTATCCATGGCGTTCAGGGGCACGT
CGCCTATCCGCATCTGGCGGATAACCCTGTTCATCGCGCCGCGCCTTTTCTTAATGAACTGGTCGCTATCGAGTGGGATCGGG
GCAATGACTTTTTCCCGGCCACCAGTATGCAGGTAGCCAATATCCAGGCAGGCACCGGCAGCAATAATGTCATTCCCGGCGAA
CTGTTCGTGCAGTTTAACTTCCGTTTCAGCACGGAACTGACCGATGAGATGATCAAAGAGCGGGTACATGCGCTGCTTGAAAA
GCATCAGCTACGCTATACCGTAGACTGGTGGCTTTCCGGGCAGCCGTTCCTGACCGCACGCGGCAAGCTGGTGGACGCGGTGG
TAAACGCCATTGAGCACTATAATGAAATTAAACCGCAATTACTGACGACGGGCGGTACGTCCGACGGACGTTTTATCGCCCGT
ATGGGAGCGCAGGTGGTAGAACTGGGGCCGGTGAACGCCACCATTCATAAAATTAATGAATGTGTGAATGCCGCCGATCTCCA
GCTACTGGCCCGTATGTATCAACGCATTATGGAACAGCTCGTCGCCTGA
SEQ ID NO: 58: htrA (encoding serine protease, Gene ID: 1251727) 
ATGAAAAAAACCACATTAGCAATGAGTGCACTGGCTCTGAGTTTAGGTTTGGCATTGTCGCCTCTGTCTGCCACGGCGGCTGA
AACGTCCTCTTCAGCAATGACTGCCCAGCAGATGCCAAGCCTGGCACCGATGCTCGAAAAAGTGATGCCATCGGTGGTCAGTA
TTAATGTTGAAGGTAGCACCACGGTGAATACGCCGCGTATGCCGCGTAATTTCCAGCAGTTCTTTGGCGATGACTCCCCGTTC
TGCCAGGACGGTTCTCCGTTCCAGAATTCTCCGTTCTGCCAGGGCGGCGGTAACGGCGGCAACGGCGGTCAACAACAGAAATT
CATGGCGCTGGGCTCCGGCGTAATTATTGACGCCGCGAAGGGCTACGTCGTCACCAACAACCACGTGGTTGATAACGCCAGCG
TGATTAAAGTACAGCTTAGCGATGGGCGTAAATTCGATGCTAAAGTGGTGGGCAAAGATCCGCGTTCTGATATCGCGCTGATT
CAAATTCAGAATCCGAAGAACCTGACGGCGATTAAGCTGGCGGACTCCGACGCGCTGCGCGTGGGGGATTATACCGTCGCTAT
TGGTAACCCGTTTGGTCTGGGCGAAACGGTGACGTCAGGTATCGTTTCGGCGCTGGGGCGTAGCGGCCTGAACGTAGAAAATT
ACGAGAACTTTATTCAGACCGACGCCGCGATTAACCGTGGTAACTCCGGCGGCGCGCTGGTGAACCTGAACGGTGAGCTGATC
GGTATTAACACCGCGATTCTGGCGCCGGACGGCGGCAACATCGGTATCGGCTTCGCTATCCCCAGTAACATGGTGAAAAACCT
GACGTCGCAGATGGTGGAATACGGCCAGGTGAAACGCGGCGAACTGGGGATCATGGGGACTGAGCTGAATTCCGAATTGGCGA
AAGCGATGAAAGTCGACGCCCAGCGAGGCGCGTTCGTCAGCCAGGTGATGCCGAATTCGTCCGCGGCGAAAGCGGGTATCAAA
GCCGGGGATGTCATTACCTCGCTGAACGGTAAACCGATCAGCAGCTTTGCGGCGCTGCGCGCTCAGGTCGGCACTATGCCGGT
CGGCAGCAAAATCAGCCTCGGTCTGCTGCGTGAAGGTAAAGCGATTACGGTGAATCTGGAACTGCAGCAGAGCAGCCAGAGTC
AGGTTGATTCCAGCACCATCTTCAGCGGGATTGAAGGCGCTGAAATGAGCAATAAAGGCCAGGATAAAGGCGTTGTGGTGAGC
AGCGTGAAAGCGAACTCACCCGCCGCGCAAATTGGCCTCAAAAAAGGCGATGTGATTATCGGCGCTAACCAGCAGCCGGTGAA
AAATATCGCCGAGCTGCGTAAGATTCTCGACAGCAAGCCGTCGGTTCTGGCGCTGAATATTCAGCGTGGTGATAGTTCTATTT
 ATTTGCTGATGCAGTAA
SEQ ID NO: 59: hlyA (encoding Listeria hemolysin, Gene ID:987033) 
ATGAAAAAAATAATGCTAGTTTTTATTACACTTATATTAGTTAGTCTACCAATTGCGCAACAAACTGAAGCAAAGGATGCATC
TGCATTCAATAAAGAAAATTCAATTTCATCCATGGCACCACCAGCATCTCCGCCTGCAAGTCCTAAGACGCCAATCGAAAAGA
AACACGCGGATGAAATCGATAAGTATATACAAGGATTGGACTACAATAAAAACAATGTATTAGTATACCACGGAGATGCAGTG
ACAAATGTGCCGCCAAGAAAAGGTTACAAAGATGGAAATGAATATATTGTTGTGGAGAAAAAGAAGAAATCCATCAATCAAAA
TAATGCAGACATTCAAGTTGTGAATGCAATTTCGAGCCTAACCTATCCAGGTGCTCTCGTAAAAGCGAATTCGGAATTAGTAG
AAAATCAACCAGATGTTCTCCCTGTAAAACGTGATTCATTAACACTCAGCATTGATTTGCCAGGTATGACTAATCAAGACAAT
AAAATCGTTGTAAAAAATGCCACTAAATCAAACGTTAACAACGCAGTAAATACATTAGTGGAAAGATGGAATGAAAAATATGC
TCAAGCTTATCCAAATGTAAGTGCAAAAATTGATTATGATGACGAAATGGCTTACAGTGAATCACAATTAATTGCGAAATTTG
GTACAGCATTTAAAGCTGTAAATAATAGCTTGAATGTAAACTTCGGCGCAATCAGTGAAGGGAAAATGCAAGAAGAAGTCATT
AGTTTTAAACAAATTTACTATAACGTGAATGTTAATGAACCTACAAGACCTTCCAGATTTTTCGGCAAAGCTGTTACTAAAGA
GCAGTTGCAAGCGCTTGGAGTGAATGCAGAAAATCCTCCTGCATATATCTCAAGTGTGGCGTATGGCCGTCAAGTTTATTTGA
AATTATCAACTAATTCCCATAGTACTAAAGTAAAAGCTGCTTTTGATGCTGCCGTAAGCGGAAAATCTGTCTCAGGTGATGTA
GAACTAACAAATATCATCAAAAATTCTTCCTTCAAAGCCGTAATTTACGGAGGTTCCGCAAAAGATGAAGTTCAAATCATCGA
CGGCAACCTCGGAGACTTACGAGATATTTTGAAAAAAGGCGCTACTTTTAATCGAGAAACACCAGGAGTTCCCATTGCTTATA
CAACAAACTTCCTAAAAGACAATGAATTAGCTGTTATTAAAAACAACTCAGAATATATTGAAACAACTTCAAAAGCTTATACA
GATGGAAAAATTAACATCGATCACTCTGGAGGATACGTTGCTCAATTCAACATTTCTTGGGATGAAGTAAATTATGATCCTGA
AGGTAACGAAATTGTTCAACATAAAAACTGGAGCGAAAACAATAAAAGTAAGCTAGCTCATTTCACATCGTCCATCTATTTGC
CAGGTAACGCGAGAAATATTAATGTTTACGCTAAAGAATGCACTGGTTTAGCTTGGGAATGGTGGAGAACGGTAATTGATGAC
CGGAACTTACCACTTGTGAAAAATAGAAATATCTCCATCTGGGGCACCACGCTTTATCCGAAATATAGTAATAAAGTAGATAA
TCCAATCGAATAA
SEQ ID NO: 60 coding sequence for HA-his tag
   Tacccctatgacgtgcctgattacgccggctctggccaccaccaccaccaccac
SEQ ID NO: 61 G-CSF-R nanobody 1 (with CDRs bold and underlined) (VHH1) 
GGGSVQAGGSLKLSCEASLPSTTTPYSMAWFRQAPGREREAVATISARGGTKLYSDSVRERFTISRDNRKNTVYLKMDHLNTD
DTAIYYCAATGSILFNSRPFAESTYPFWGQGTLVTVSS
SEQ ID NO: 62 G-CSF-R nanobody 2 (with CDRs bold and underlined) (VHH26) 
GGGTVQAGGSLKLSCAARGWRFSGYCVAWFRQAPGKEREAVAIIPGAGVMYTDDSVKGRFTYSRDNVKNTVSLQMNSLEPEDT
ATYYCAVDWAYSHSCPKNTEDYRYWGQGTQVTVSS
SEQ ID NO: 63 EGFR nanobody 1 (with CDRs bold and underlined) (EGa1) 
MKYLLPTAAAGLLLLAAQPAMAQVQLQESGGGLVQPGGSLRLSCAASGRTFSSYAMGWFRQAPGKEREFVAAIRWSGGYTYYT
DSVKGRFTISRDNAKTTVYLQMNSLKPEDTAVYYCAATYLSSDYSRYALPQRPLDYDYWGQGTQVTVSSAAAEQKLISEEDLN
GLE
SEQ ID NO: 64 CXCR4 nanobody 1 (with CDRs bold and underlined) (238D2) 
EVQLVESGGGLVQTGGSLRLSCAASGFTFSSYAMSWVRQAPGKGLEWVSGIKSSGDSTRYAGSVKGRFTISRDNAKNMLYLQM
YSLKPEDTAVYYCAKSRVSRTGLYTYDNRGQGTQVTVSS
SEQ ID NO: 65 CXCR4 nanobody 2 (with CDRs bold and underlined) (238D4) 
EVQLMESGGGLVQAGGSLRLSCAASGRTFNNYAMGWFRRAPGKEREFVAAITRSGVRSGVSAIYGDSVKDRFTISRDNAKNTL
YLQMNSLKPEDTAVYTCAASAIGSGALRRFEYDYSGQGTQVTVS
SEQ ID NO: 66 IL6R nanobody 1 (with CDRs bold and underlined) 
EVQLVESGGGLVQAGDSLRLSCLASGRSFKDDAMGWFRQAPGKEREFVSGIDWRGNIVDAESVKGRFTISRENAKNTLYLQMN
HLKPEDTAVYYCAASSVFSDLRYRKNPKYWGQGTQVTVSS
SEQ ID NO: 67 IL6R nanobody 2 (with CDRs bold and underlined) 
EVQLVESGGGLVQAGGSLRRLSCAASGSIFRINAMGWYRQAPGKQRELVAAAISGGSTNYADFVKGRFTISRDNAKNTVYLQM
NSLKPEDTAVYYCNALVVSHNYSDYVPFPDDYWGQGTQVTVSS
SEQ ID NO: 68 IL6R nanobody 3 (with CDRs bold and underlined) 
EVQLVESGGGLVQPGGSLRLSCAASGFTLDYYAIGWFRQAPGKEREGVSCISSTDGSTYYADSVKGRFTISRDNAKNTVYLQM
NSLNPEDTGVYTCAAVLTTAQAMGVPTRSYEYDYWGQGTQVTVSS
SEQ ID NO: 69 IL6R nanobody 4 (with CDRs bold and underlined) 
EVQLVESGGGLVEAGGSLRLSCAAAGRTLSSYSMAWFRQAPGKEREFVAIIRGNPSRTYHSDSVKGRFTASRDNAKNTVYLQM
NRLEPEDTAVYYCAADPPPFASDYSAPQSYDYWGQGTQVTVSS
SEQ ID NO: 70 IL6R nanobody 5 (with CDRs bold and underlined) 
EVQLVESGGGLVQAGGSLNLSCNASGDIGSINAMGWHRQAPGQQREWVAVITDTDSTIYPDSVKGRFAISRDGAKNTVHLRMS
SLKPEDTAVYYCNAYQAGWGDYWGQGTQVTVSS
SEQ ID NO: 71 hTNFR1 nanobody 1 (with CDRs bold and underlined) 
QVQLQESCCCVVQPCCSLTLSCTRTGFTASTNAYGWYRQCPCKKCEWVSYMTIPSGRTTYADAVKGRFAMSRDKAKSTVLLQM
NSLKPEDTAVYYCGDVPFSTLPAMCTNDGPWGQGTQVTVSS
SEQ ID NO: 72 hTNFR1 nanobody 2 (with CDRs bold and underlined) 
QVQLQESGGGVVQPGGSLTLSCTRTGFTASTNAYGWYRQGPGKKCEWVSYMTIPSGRTTYADAVKGRFAMSRDKAKSTVFLQM
NSLKPEDTAVYYCGDVPFSTLPAMCTNDGPWGQGTQVTVSS
SEQ ID NO: 73 hTNFR1 nanobody 3 (with CDRs bold and underlined) 
QVQLQESGGGLVQAGGPLRLSCAASWRTFSSYAMGWFRQAPGKEREFVAAISWSGGSTYYADSVKGRFTISRDNAKNTVYLQM
NSLKPEDTAVYYCAVLHEDLYEYDYWGQGTQVTVSS
SEQ ID NO: 74 hTNFR1 nanobody 4 (with CDRs bold and underlined) 
QVQLQESGGGVVQPGGSLTLSCTRTGLTPSTGAYGWYRQAPGKKCELVSYITIPSGRTTYTDSVKGRFAISRDKAKNTVFLQM
NSLKPEDTALYYCGDVPYSTIQAMCTDDGPWGQGTQVTVSS
SEQ ID NO: 75 hTNFR1 nanobody 5 (with CDRs bold and underlined) 
QVQLQESGGGVVQPGGSLTLSCTRTGLTASTNAYGWYRQAPGKKCELVSYITIPSGRTTYADSVKGRFAISRDKAKNTVFLQM
NSLKPEDTALYYCGDVPYSTIQAMCTDDGPWGQGTQVTVSS
SEQ ID NO: 76 hTNFR1 nanobody 6 (with CDRs bold and underlined) 
QVQLQESGGGLVQSGGSLRLSCRASGFTFSHYPMSWYRQAPGKKRELVAGIYTDGSTNYADSVKGRFTISRDNAKNTVYLQMS
SLKPEDTAVYYCRRSMVSVYWGQGTQVTVSS
SEQ ID NO: 77 hTNFR1 nanobody 7 (with CDRs bold and underlined) 
QVQLQESGGGLVQPGGSLRLSCAASGRTFSYYTMAWFRQAPGKEREFVASISWSGGITYYADSVKGRFTISRDNAKNTVYLQM
NSLKPEDTAVYYCAARKEPGSAYFGSWGQGTQVTVSS
SEQ ID NO: 78 hTNFR1 nanobody 8 (with CDRs bold and underlined) 
QVQLQESGGGVVQPGGSLTLSCTRTGFTASTNAYGWYRQAPGKKCELVSYITIPSGRTTYADAVKGRFAISRDKAKNTVFLQM
NSLKPEDTAVYYCGDVPFSTLPAMCTNDGPWGQGTQVTVSS
SEQ ID NO: 79 Cholix toxin 
LYLTFYLEKVMKKMLLIAGATVISSMAHPTFAVEDELNIFDECRSPCSLTPELGKPIQSKLSIPSDVVLDEGVLYYSMTINDE
QNDIKDEDKGESIITIGEFATVRATRHYVNQDAPFGVINLDITTENGTKTYSYNRKEGEFAINWLVPIGEDSPASIKISVDEL
DQQRNIIEVPKLYSIDLDNQTLEQWKTQGNVSFSVTRPEHNIAISWPSVSYKAAQKEGSRHKRWAHWHTGLALCWLVPIDAIY
NYITQQNCTLGDNWFGGSYETVAGTPKAITVKQGIEQKPVEQRIHFSKKNAMEALAAHRVCGVPLETLARSRKPRDLPDDLSC
AYQAQNIVSLFVATRILFSHLDSVFTLNLDEQEPEVAERLSALRQINENNPGMVTQVLTVARQIYNDYVTHHPGLTPEQTSAG
AQAADILSLFCPDADKPCVASNNDQANINIESRSGRSYLPENRAVITPQGVTNWTYQELEATHQALTREGYVFVGYHGTNHVA
AQTIVNRIAPVPRGNNTENEEKWGGLYVATHAEVAHGYARIKEGTGEYGLPTRAERDARGVMLRVYIPRASLERFYRTNTPLE
NAEEHITQVIGHSLPLRNEAFTGPESAGGEDETVIGWDMAIHAVAIPSTIPGNAYEELAIDEEAVAKEQSISTKPPYKERKDE
LK
SEQ ID NO: 80 CET40 
EALAAHRVCGVPLETLARSRKPRDLPDDLSCAYQAQNIVSLFVATRILFSHLDSVFTLNLDEQEPEVAERLSALRQINENNPG
MVTQVLTVARQIYNDYVTHHPGLTPEQTSAGAQAADILSLFCPDADKPCVASNNDQANINIESRSGRSYLPENRAVITPQGVT
NWTYQELEATHQALTREGYVFVGYHGTNHVAAQTIVNRIAPVPRGNNTENEEKWGGLYVATHAEVAHGYARIKEGTGEYGLPT
RAERDARGVMLRVYIPRASLERFYRTNTPLENAEEHITQVIGHSLPLRNEAFTGPESAGGEDETVIGWDMAIHAVAIPSTIPG
NAYEELAIDEEAVAKEQSISTKPPYKERKDELK
SEQ ID NO: 81 Cholera toxin 
MVKIIFVFFIFLSSFSYANDDKLYRADSRPPDEIKQSGGLMPRGQSEYFDRGTQMNINLYDHARGTQTGFVRHDDGYVSTSIS
LRSAHLVGQTILSGHSTYYIYVIATAPNMFNVNDVLGAYSPHPDEQEVSALGGIPYSQIYGWYRVHFGVLDEQLHRNRGYRDR
YYSNLDIAPAADCYCLACFPPEHRAWREEPWIHHAPPCCGNAPRSSMSNTCDEKTQSLCVKFLDEYQSKVKRQIFSCYQSDID
THNRIKDELMIKLKFGVFFTVLLSSAYAHGTPQNITDLCAESHNTQIYTLNDKIFSYTESLAGKREMAIITFKNGAIFQVEVP
SSQHIDSQKKAIERMKDTLRIAYLTEAKVEKLCVWNNKTPHAIAAISMAN
SEQ ID NO: 82 Heat-Labile Enterotoxin SUBUNIT A 
MKNITFIFFILLASPLYANGDRLYRADSRPPDEIKRSGGLMPRGHNEYFDRGTQMNINLYDHARGTQTGFVRYDDGYVSTSLS
LRSAHLAGQSILSGYSTYYIYVIATAPNMFNVNDVLGVYSPHPYEQEVSALGGIPYSQIYGWYRVNFGVIDERLHRNREYRDR
YYRNLNIAPAEDGYRLAGFPPDHQAWREEPWIHHAPQGCGNSSRTITGDTCNEETQNLSTIYLREYQSKVKRQIFSDYQSEVD
IYNRIRDEL
SEQ ID NO: 83 Heat-Labile Enterotoxin SUBUNIT B 
APQTITELCSEYRNTQIYTINDKILSYTESMAGKREMVIITFKSGETFQVEVPGSQHIDSQKKAIERMKDTLRITYLTETKID
KLCVWNNKTPNSIAAISMKN
SEQ ID NO: 84 Diphtheria Toxin 
GADDVVDSSKSFVMENFSSYHGTKPGYVDSIQKGIQKPKSGTQGNYDDDWKGFYSTDNKYDAAGYSVDNENPLSGKAGGVVKV
TYPGLTKVLALKVDNAETIKKELGLSLTEPLMEQVGTEEFIKRFGDGASRVVLSLPFAEGSSSVEYINNWEQAKALSVELEIN
FETRGKRGQDAMYEYMAQACAGNRVRRSVGSSLSCINLDWDVIRDKTKTKIESLKEHGPIKNKMSESPNKTVSEEKAKQYLEE
FHQTALEHPELSELKTVTGTNPVFAGANYAAWAVNVAQVIDSETADNLEKTTAALSILPGIGSVMGIADGAVHHNTEEIVAQS
IALSSLMVAQAIPLVGELVDIGFAAYNFVESIINLFQVVHNSYNRPAYSPGHKTQPFLHDGYAVSWNTVEDSIIRTGFQGESG
HDIKITAENTPLPIAGVLLPTIPGKLDVNKSKTHISVNGRKIRMRCRAIDGDVTFCRPKSPVYVGNGVHANLHVAFHRSSSEK
 IHSNEISSDSIGVLGYQKTVDHTKVNSKLSLFFEIKS
SEQ ID NO: 85 DT390 
GADDVVDSSKSFVMENFSSYHGTKPGYVDSIQKGIQKPKSGTQGNYDDDWKGFYSTDNKYDAAGYSVDNENPLSGKAGGVVKV
TYPGLTKVLALKVDNAETIKKELGLSLTEPLMEQVGTEEFIKRFGDGASRVVLSLPFAEGSSSVEYINNWEQAKALSVELEIN
FETRGKRGQDAMYEYMAQACAGNRVRRSVGSSLSCINLDWDVIRDKTKTKIESLKEHGPIKNKMSESPNKTVSEEKAKQYLEE
FHQTALEHPELSELKTVTGTNPVFAGANYAAWAVNVAQVIDSETADNLEKTTAALSILPGIGSVMGIADGAVHHNTEEIVAQS
IALSSLMVAQAIPLVGELVDIGFAAYNFVESIINLFQVVHNSYNRPAYSPGHKTQPFL
SEQ ID NO: 86 Shigga toxin Chain A 
KEFTLDFSTAKTYVDSLNVIRSAIGTPLQTISSGGTSLLMIDSGTGDNLFAVDVRGIDPEEGRFNNLRLIVERNNLYVTGFVN
RTNNVFYRFADFSHVTFPGTTAVTLSGDSSYTTLQRVAGISRTGMQINRHSLTTSYLDLMSHSGTSLTQSVARAMLRFVTVTA
EALRFRQIQRGFRTTLDDLSGRSYVMTAEDVDLTLNWGRLSSVLPDYHGQDSVRVGRISFGSINAILGSVALILNCHHHASRV
ARMASDEFPSMCPADGRVRGITHNKILWDSSTLGAILM
SEQ ID NO: 87 Shigga toxin Chain B
   TPDCVTGKVEYTKYNDDDTFTVKVGDKELFTNRWNLQSLLLSAQITGMTVTIKTNACHNGGGFSEVIFR
SEQ ID NO: 88 Anthrax toxin Edema factor [Bacillus anthracis] 
MTRNKFIPNKFSIISFSVLLFAISSSQAIEVNAMNEHYTESDIKRNHKTEKNKTEKEKFKDSINNLVKTEFTNETLDKIQQTQ
DLLKKIPKDVLEIYSELGGEIYFTDIDLVEHKELQDLSEEEKNSMNSRGEKVPFASRFVFEKKRETPKLIINIKDYAINSEQS
KEVYYEIGKGISLDIISKDKSLDPEFLNLIKSLSDDSDSSDLLFSQKFKEKLELNNKSIDINFIKENLTEFQHAFSLAFSYYF
APDHRTVLELYAPDMFEYMNKLEKGGFEKISESLKKEGVEKDRIDVLKGEKALKASGLVPEHADAFKKIARELNTYILFRPVN
KLATNLIKSGVATKGLNVHGKSSDWGPVAGYIPFDQDLSKKHGQQLAVEKGNLENKKSITEHEGEIGKIPLKLDHLRIEELKE
NGIILKGKKEIDNGKKYYLLESNNQVYEFRISDENNEVQYKTKEGKITVLGEKFNWRNIEVMAKNVEGVLKPLTADYDLFALA
PSLTEIKKQIPQKEWDKVVNTPNSLEKQKGVTNLLIKYGIERKPDSTKGTLSNWQKQMLDRLNEAVKYTGYTGGDVVNHGTEQ
DNEEFPEKDNEIFIINPEGEFILTKNWEMTGRFIEKNITGKDYLYYFNRSYNKIAPGNKAYIEWTDPITKAKINTIPTSAEFI
KNLSSIRRSSNVGVYKDSGDKDEFAKKESVKKIAGYLSDYYNSANHIFSQEKKRKISIFRGIQAYNEIENVLKSKQIAPEYKN
YFQYLKERITNQVQLLLTHQKSNIEFKLLYKQLNFTENETDNFEVFQKIIDEK
SEQ ID NO: 89 Anthrax toxin lethal factor [Bacillus anthracis] 
MNIKKEFIKVISMSCLVTAITLSGPVFIPLVQGAGGHGDVGMHVKEKEKNKDENKRKDEERNKTQEEHLKEIMKHIVKIEVKG
EEAVKKEAAEKLLEKVPSDVLEMYKAIGGKIYIVDGDITKHISLEALSEDKKKIKDIYGKDALLHEHYVYAKEGYEPVLVIQS
SEDYVENTEKALNVYYEIGKILSRDILSKINQPYQKFLDVLNTIKNASDSDGQDLLFTNQLKEHPTDFSVEFLEQNSNEVQEV
FAKAFAYYIEPQHRDVLQLYAPEAFNYMDKFNEQEINLSLEELKDQRMLARYEKWEKIKQHYQHWSDSLSEEGRGLLKKLQIP
IEPKKDDIIHSLSQEEKELLKRIQIDSSDFLSTEEKEFLKKLQIDIRDSLSEEEKELLNRIQVDSSNPLSEKEKEFLKKLKLD
IQPYDINQRLQDTGGLIDSPSINLDVRKQYKRDIQNIDALLHQSIGSTLYNKIYLYENMNINNLTATLGADLVDSTDNTKINR
GIFNEFKKNFKYSISSNYMIVDINERPALDNERLKWRIQLSPDTRAGYLENGKLILQRNIGLEIKDVQIIKQSEKEYIRIDAK
VVPKSKIDTKIQEAQLNINQEWNKALGLPKYTKLITFNVHNRYASNIVESAYLILNEWKNNIQSDLIKKVTNYLVDGNGRFVF
TDITLPNIAEQYTHQDEIYEQVHSKGLYVPESRSILLHGPSKGVELRNDSEGFIHEFGHAVDDYAGYLLDKNQSDLVTNSKKF
IDIFKEEGSNLTSYGRTNEAEFFAEAFRLMHSTDHAERLKVQKNAPKTFQFINDQIKFIINS
SEQ ID NO: 90 Anthrax toxin protective antigen [Bacillus anthracis] 
MKKRKVLIPLMALSTILVSSTGNLEVIQAEVKQENRLLNESESSSQGLLGYYFSDLNFQAPMVVTSSTTGDLSIPSSELENIP
SENQYFQSAIWSGFIKVKKSDEYTFATSADNHVTMWVDDQEVINKASNSNKIRLEKGRLYQIKIQYQRENPTEKGLDFKLYWT
DSQNKKEVISSDNLQLPELKQKSSNSRKKRSTSAGPTVPDRDNDGIPDSLEVEGYTVDVKNKRTFLSPWISNIHEKKGLTKYK
SSPEKWSTASDPYSDFEKVTGRIDKNVSPEARHPLVAAYPIVHVDMENIILSKNEDQSTQNTDSQTRTISKNTSTSRTHTSEV
HGNAEVHASFFDIGGSVSAGFSNSNSSTVAIDHSLSLAGERTWAETMGLNTADTARLNANIRYVNTGTAPIYNVLPTTSLVLG
KNQTLATIKAKENQLSQILAPNNYYPSKNLAPIALNAQDDFSSTPITMNYNQFLELEKTKQLRLDTDQVYGNIATYNFENGRV
RVDTGSNWSEVLPQIQETTARIIFNGKDLNLVERRIAAVNPSDPLETTKPDMTLKEALKIAFGFNEPNGNLQYQGKDITEFDF
NFDQQTSQNIKNQLAELNATNIYTVLDKIKLNAKMNILIRDKRFHYDRNNIAVGADESVVKEAHREVINSSTEGLLLNIDKDI
RKILSGYIVEIEDTEGLKEVINDRYDMLNISSLRQDGKTFIDFKKYNDKLPLYISNPNYKVNVYAVTKENTIINPSENGDTST
NGIKKILIFSKKGYEIG
SEQ ID NO: 91 Ricin chain A 
SNAMIFPKQYPIINFTTAGATVQSYTNFIRAVRGRLTTGADVRHEIPVLPNRVGLPINQRFILVELSNHAELSVTLALDVTNA
YVVGYRAGNSAYFFHPDNQEDAEAITHLFTDVQNRYTFAFGGNYDRLEQLAGNLRENIELGNGPLEEAISALYYYSTGGTQLP
TLARSFIICIQMISEAARFQYIEGEMRTRIRYNRRSAPDPSVITLENSWGRLSTAIQESNQGAFASPIQLQRRNGSKFSVYDV
SILIPIIALMVYRCAPPPSSQF
SEQ ID NO: 92 pET26b-Kana-T7-pelB-238D2-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
 
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAAGTGCAACTGGTGGAAAGCGGTGGAGGCCTTGTTCAG
ACCGGCGGCAGCCTGCGTCTGTCATGCGCTGCAAGCGGCTTTACGTTCAGCAGCTACGCGATGAGCTGGGTGCGCCAGGCACC
GGGCAAAGGCTTGGAATGGGTTAGCGGCATCAAGTCAAGCGGCGATAGCACCAGATATGCCGGAAGCGTTAAAGGCCGCTTTA
CCATTTCACGCGATAACGCCAAGAACATGCTGTATCTGCAAATGTATAGCCTGAAACCGGAAGATACCGCAGTGTATTACTGC
GCCAAGAGCCGCGTGAGTCGCACGGGTCTGTATACCTATGACAACCGTGGTCAGGGCACGCAGGTGACTGTGTCAAGCCATCA
TCACCATCATCATTAAgatccggctgctaacaaagcccgaaaggaagctgagttggctgctgccaccgctgagcaataactag
cataaccccttggggcctctaaacgggtcttgaggggttttttgctgaaaggaggaactatatccggat
SEQ ID NO: 93 pET26b-Kana-T7-pelB-238D4-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
 
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAAGTCCAGCTGGTTGAAAGCGGTGGAGGCCTGGTTCAG
ACCGGCGGAAGCCTGCGTCTGAGCTGTGCAGCCTCTGGCCGCACGTTTAACAACTACGCGATGGGCTGGTTTCGCCGTGCACC
GGGCAAAGAACGCGAATTTGTGGCGGCAATCACTCGTAGCGGTGTGAGAAGCGGAGTGTCAGCGATCTATGGCGATAGCGTGA
AAGATCGCTTCACCATCAGCCGCGATAACGCCAAGAACACCTTGTACTTGCAGATGAACAGCCTGAAACCGGAAGATACTGCG
GTGTATACCTGCGCCGCAAGTGCGATCGGCAGCGGTGCACTGCGTCGTTTCGAATACGACTATAGCGGTCAGGGCACACAGGT
GACCGTGAGCAGCCATCATCACCATCATCATTAAgatccggctgctaacaaagcccgaaaggaagctgagttggctgctgcca
ccgctgagcaataactagcataaccccttggggcctctaaacgggtcttgaggggttttttgctgaaaggaggaactatatcc
ggat
SEQ ID NO: 94 pET26b-Kana-T7-pelB-EGa1-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
 
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccCAGGTTCAGCTGCAAGAAAGCGGAGGTGGTCTGGTTCAG
CCGGGAGGTAGCTTACGCCTGTCATGCGCAGCAAGCGGTCGTACCTTTAGCAGCTATGCGATGGGCTGGTTTCGTCAGGCACC
TGGCAAAGAACGCGAATTTGTGGCGGCAATTCGCTGGTCAGGCGGCTATACCTACTACACCGATAGCGTGAAAGGCCGCTTTA
CCATTAGCCGCGATAACGCCAAGACCACCGTGTACCTGCAAATGAACAGCCTGAAACCGGAAGATACCGCAGTGTACTACTGC
GCAGCGACCTATCTGAGCAGCGATTATAGCCGCTATGCCTTACCGCAGCGTCCGCTGGATTACGACTATTGGGGCCAAGGTAC
GCAGGTTACCGTTAGCAGCGCAGCAGCGCATCATCACCATCATCATTAAgatccggctgctaacaaagcccgaaaggaagctg
agttggctgctgccaccgctgagcaataactagcataaccccttggggcctctaaacgggtcttgaggggttttttgctgaaa
ggaggaactatatccggat
SEQ ID NO: 95 pET26b-Kana-T7-pelB-VHH1-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
 
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGGCGGTGGCAGCGTTCAGGCAGGTGGCAGCCTGAAACTG
AGCTGCGAAGCCAGCCTGCCTAGCACGACCACGCCGTATAGCATGGCCTGGTTTCGTCAGGCGCCTGGACGTGAAAGAGAAGC
GGTGGCCACCATCAGCGCAAGAGGTGGCACTAAACTGTACAGCGATTCTGTGCGCGAACGCTTCACGATTAGCCGTGATAACC
GCAAGAACACGGTCTACCTGAAGATGGATCATCTGAACACCGATGATACCGCCATCTACTATTGCGCCGCAACGGGCAGCATT
CTGTTTAATAGCCGTCCATTCGCGGAAAGCACGTATCCGTTCTGGGGTCAGGGTACTCTGGTCACGGTGAGCAGCCATCATCA
CCATCATCATTAAgatccggctgctaacaaagcccgaaaggaagctgagttggctgctgccaccgctgagcaataactagcat
aaccccttggggcctctaaacgggtcttgaggggttttttgctgaaaggaggaactatatccggat
SEQ ID NO: 96 pET26b-Kana-T7-pelB-VHH26-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
 
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGGCGGTGGCACGGTACAGGCTGGCGGTAGCCTGAAACTG
AGCTGCGCCGCGAGAGGTTGGCGCTTTAGCGGCTATTGCGTAGCCTGGTTTCGTCAAGCACCGGGCAAAGAACGCGAAGCGGT
AGCGATCATTCCGGGTGCTGGCGTGATGTATACCGATGATTCTGTGAAAGGCCGTTTCACCTATTCGCGCGATAATGTGAAGA
ACACCGTTAGTCTGCAAATGAACAGCCTGGAACCGGAAGATACCGCAACGTACTACTGCGCTGTGGATTGGGCATACAGCCAT
AGCTGTCCGAAGAACACGGAAGATTACCGTTATTGGGGCCAGGGCACGCAGGTGACCGTGTCATCAtacccctatgacgtgcc
tgattacgccggctctggccaccaccaccaccaccactgagatccggctgctaacaaagcccgaaaggaagctgagttggctg
ctgccaccgctgagcaataactagcataaccccttggggcctctaaacgggtcttgaggggttttttgctgaaaggaggaact
atatccggat
SEQ ID NO: 97 pET26b-Kana-T7-pelB-CET40-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
 
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAAGCACTGGCAGCACATCGTGTTTGCGGAGTTCCACTG
GAAACGCTGGCACGTAGCCGTAAACCGCGTGATCTTCCGGATGATCTGTCATGCGCCTATCAGGCACAGAACATCGTTAGCCT
GTTTGTGGCAACACGCATCTTGTTCAGCCATCTGGATAGCGTGTTTACGCTGAACCTGGATGAACAAGAACCGGAAGTGGCAG
AACGCCTTAGCGCACTGAGACAGATCAACGAGAACAATCCGGGCATGGTGACACAGGTGCTGACCGTTGCACGCCAGATTTAC
AACGATTACGTGACGCATCATCCGGGCTTAACTCCGGAACAGACTAGCGCGGGTGCACAAGCCGCAGATATCTTGAGCCTGTT
TTGTCCGGATGCAGATAAACCGTGCGTTGCGAGCAACAACGATCAGGCGAACATCAACATCGAGTCACGTAGCGGACGCAGCT
ATCTGCCGGAGAATCGCGCCGTGATTACGCCTCAGGGTGTGACCAACTGGACCTATCAAGAACTGGAAGCAACGCATCAGGCA
CTGACACGCGAAGGTTATGTGTTTGTTGGCTATCATGGCACCAACCATGTTGCGGCACAGACCATTGTGAACCGCATTGCACC
GGTTCCACGTGGTAACAACACCGAAAACGAAGAGAAATGGGGCGGTCTGTATGTTGCCACGCACGCAGAAGTTGCTCATGGCT
ATGCACGCATCAAAGAAGGTACGGGCGAATATGGCTTACCGACGCGTGCAGAACGTGATGCCAGAGGCGTTATGCTGCGTGTG
TACATTCCGAGAGCGAGCTTAGAACGCTTCTATCGCACCAACACACCGCTGGAAAACGCGGAAGAACACATTACTCAGGTGAT
TGGCCATAGCCTGCCGTTACGCAACGAAGCCTTTACCGGTCCGGAAAGCGCAGGTGGTGAAGATGAAACCGTGATTGGCTGGG
ATATGGCGATTCATGCCGTTGCCATACCGAGCACCATACCGGGCAATGCGTATGAAGAGCTGGCAATTGACGAAGAAGCAGTT
GCCAAAGAACAGAGCATTAGCACCAAACCGCCGTATAAAGAGCGCAAAGACGAACTGAAGtacccctatgacgtgcctgatta
cgccggctctggccaccaccaccaccaccactgagatccggctgctaacaaagcccgaaaggaagctgagttggctgctgcca
ccgctgagcaataactagcataaccccttggggcctctaaacgggtcttgaggggttttttgctgaaaggaggaactatatcc
ggat
SEQ ID NO: 98 pET26b-Kana-T7-pelB-DT390-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
 
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGGCGCGGATGATGTCGTGGATTCAAGCAAATCATTTGTC
ATGGAGAACTTCAGCAGCTATCACGGCACCAAACCTGGCTATGTGGATTCGATTCAGAAAGGCATTCAGAAACCGAAATCCGG
TACGCAGGGCAACTATGACGATGATTGGAAAGGCTTCTACAGCACCGATAACAAGTACGATGCGGCGGGTTACTCCGTGGATA
ATGAGAATCCACTGAGCGGTAAAGCTGGCGGTGTGGTGAAAGTCACCTATCCGGGCCTGACGAAAGTGCTGGCACTGAAGGTG
GATAATGCCGAAACCATTAAGAAAGAACTGGGCCTGAGCCTGACCGAACCTCTGATGGAACAGGTTGGCACAGAAGAGTTTAT
CAAACGCTTTGGCGATGGTGCGTCAAGAGTTGTGCTGAGCCTTCCGTTTGCCGAAGGTAGCAGCAGCGTTGAGTACATCAACA
ATTGGGAACAGGCCAAAGCACTGTCAGTGGAACTGGAAATCAACTTTGAGACACGTGGTAAGAGAGGCCAAGATGCCATGTAC
GAGTACATGGCGCAAGCGTGCGCAGGCAACCGCGTTCGTAGAAGCGTTGGCAGCAGCCTGAGCTGCATCAACCTGGACTGGGA
TGTGATTCGCGATAAGACCAAGACGAAGATCGAAAGCCTGAAAGAACACGGACCAATCAAGAACAAGATGAGCGAAAGTCCGA
ACAAGACGGTGAGCGAAGAGAAAGCGAAACAGTATCTGGAAGAGTTTCACCAGACCGCACTGGAACATCCGGAACTGAGCGAG
CTTAAGACCGTGACGGGCACAAATCCGGTGTTTGCAGGCGCAAACTATGCGGCATGGGCGGTGAACGTGGCACAGGTCATTGA
CAGCGAAACTGCGGATAATCTGGAGAAAACGACCGCTGCACTGAGCATACTGCCGGGAATTGGCAGCGTAATGGGCATCGCGG
ATGGTGCTGTTCACCACAACACCGAAGAGATTGTTGCGCAGAGCATCGCACTGAGCAGCCTGATGGTGGCACAAGCGATTCCG
CTGGTGGGCGAATTGGTCGATATCGGCTTTGCGGCCTATAACTTCGTCGAAAGCATTATCAACCTGTTTCAGGTGGTTCACAA
CAGCTACAATCGTCCGGCCTATAGTCCGGGACACAAGACGCAGCCGTTTCTGtacccctatgacgtgcctgattacgccggct
ctggccaccaccaccaccaccactgagatccggctgctaacaaagcccgaaaggaagctgagttggctgctgccaccgctgag
caataactagcataaccccttggggcctctaaacgggtcttgaggggttttttgctgaaaggaggaactatatccggat
SEQ ID NO: 99 pET26b-Kana-T7-pelB-Nb238D2-PE38-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
 
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAAGTGCAACTGGTGGAAAGCGGTGGAGGCCTTGTTCAG
ACCGGCGGCAGCCTGCGTCTGTCATGCGCTGCAAGCGGCTTTACGTTCAGCAGCTACGCGATGAGCTGGGTGCGCCAGGCACC
GGGCAAAGGCTTGGAATGGGTTAGCGGCATCAAGTCAAGCGGCGATAGCACCAGATATGCCGGAAGCGTTAAAGGCCGCTTTA
CCATTTCACGCGATAACGCCAAGAACATGCTGTATCTGCAAATGTATAGCCTGAAACCGGAAGATACCGCAGTGTATTACTGC
GCCAAGAGCCGCGTGAGTCGCACGGGTCTGTATACCTATGACAACCGTGGTCAGGGCACGCAGGTGACTGTGTCAAGCCCTGA
AGGTGGTAGCCTGGCGGCACTGACCGCACACCAAGCATGTCATCTGCCACTGGAAACCTTTACCCGTCATCGTCAGCCGCGTG
GCTGGGAACAACTGGAACAATGTGGATATCCGGTGCAGAGACTGGTCGCATTATATCTGGCAGCACGTCTGAGTTGGAATCAG
GTTGATCAAGTTATTCGTAACGCACTTGCCTCACCGGGTAGCGGTGGTGATTTAGGTGAGGCAATTCGTGAACAACCTGAACA
GGCACGCTTAGCATTAACCCTTGCAGCCGCAGAAAGTGAAAGATTTGTGCGTCAGGGTACTGGTAATGATGAGGCCGGTGCAG
CAAATGGCCCGGCAGACAGCGGAGATGCATTATTAGAGAGAAATTATCCTACCGGCGCAGAATTTCTGGGTGATGGGGGTGAT
GTTAGTTTTTCAACACGCGGGACACAGAATTGGACCGTGGAACGTTTACTGCAGGCACATCGTCAGCTGGAAGAACGCGGTTA
TGTTTTTGTTGGTTATCATGGGACGTTTCTGGAGGCAGCACAGAGCATTGTCTTTGGCGGTGTGCGGGCGCGTAGCCAGGATC
TTGATGCAATTTGGCGTGGTTTTTATATTGCGGGTGATCCTGCGCTGGCGTATGGATATGCGCAGGATCAGGAGCCGGATGCG
CGTGGTCGTATTCGTAATGGTGCCCTGCTGCGGGTTTATGTTCCGCGTAGCTCACTGCCGGGGTTTTATAGAACAAGCTTAAC
ACTGGCTGCGCCGGAAGCAGCAGGTGAAGTTGAACGTCTGATTGGCCATCCGCTGCCGTTACGTTTAGATGCAATTACTGGTC
CGGAAGAAGAAGGTGGTCGCCTGGAAACCATTCTGGGTTGGCCGCTGGCAGAAAGAACCGTGGTTATACCTTCCGCAATACCG
ACCGATCCTCGGAATGTGGGGGGTGATTTAGATCCTAGCAGCATTCCTGATAAAGAACAGGCAATTTCTGCTCTGCCTGATTA
TGCATCACAGCCGGGTAAACCGCCGCGTGAAGATTTAAAGCATCATCACCATCATCATTAAgatccggctgctaacaaagccc
gaaaggaagctgagttggctgctgccaccgctgagcaataactagcataaccccttggggcctctaaacgggtcttgaggggt
tttttgctgaaaggaggaactatatccggat
SEQ ID NO: 100 pET26b-Kana-T7-pelB-Nb238D4-PE38-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGAAGTCCAGCTGGTTGAAAGCGGTGGAGGCCTGGTTCAG
ACCGGCGGAAGCCTGCGTCTGAGCTGTGCAGCCTCTGGCCGCACGTTTAACAACTACGCGATGGGCTGGTTTCGCCGTGCACC
GGGCAAAGAACGCGAATTTGTGGCGGCAATCACTCGTAGCGGTGTGAGAAGCGGAGTGTCAGCGATCTATGGCGATAGCGTGA
AAGATCGCTTCACCATCAGCCGCGATAACGCCAAGAACACCTTGTACTTGCAGATGAACAGCCTGAAACCGGAAGATACTGCG
GTGTATACCTGCGCCGCAAGTGCGATCGGCAGCGGTGCACTGCGTCGTTTCGAATACGACTATAGCGGTCAGGGCACACAGGT
GACCGTGAGCAGCCCTGAAGGTGGTAGCCTGGCGGCACTGACCGCACACCAAGCATGTCATCTGCCACTGGAAACCTTTACCC
GTCATCGTCAGCCGCGTGGCTGGGAACAACTGGAACAATGTGGATATCCGGTGCAGAGACTGGTCGCATTATATCTGGCAGCA
CGTCTGAGTTGGAATCAGGTTGATCAAGTTATTCGTAACGCACTTGCCTCACCGGGTAGCGGTGGTGATTTAGGTGAGGCAAT
TCGTGAACAACCTGAACAGGCACGCTTAGCATTAACCCTTGCAGCCGCAGAAAGTGAAAGATTTGTGCGTCAGGGTACTGGTA
ATGATGAGGCCGGTGCAGCAAATGGGCCGGCAGACAGCGGAGATGCATTATTAGAGAGAAATTATCCTACCGGCGCAGAATTT
 
CTGGGTGATGGGGGTGATGTTAGTTTTTCAACACGCGGGACACAGAATTGGACCGTGGAACGTTTACTGCAGGCACATCGTCA
GCTGGAAGAACGCGGTTATGTTTTTGTTGGTTATCATGGGACGTTTCTGGAGGCAGCACAGAGCATTGTCTTTGGCGGTGTGC
GGGCGCGTAGCCAGGATCTTGATGCAATTTGGCGTGGTTTTTATATTGCGGGTGATCCTGCGCTGGCGTATGGATATGCGCAG
GATCAGGAGCCGGATGCGCGTGGTCGTATTCGTAATGGTGCCCTGCTGCGGGTTTATGTTCCGCGTAGCTCACTGCCGGGGTT
TTATAGAACAAGCTTAACACTGGCTGCGCCGGAAGCAGCAGGTGAAGTTGAACGTCTGATTGGCCATCCGCTGCCGTTACGTT
TAGATGCAATTACTGGTCCGGAAGAAGAAGGTGGTCGCCTGGAAACCATTCTGGGTTGGCCGCTGGCAGAAAGAACCGTGGTT
ATACCTTCCGCAATACCGACCGATCCTCGGAATGTGGGGGGTGATTTAGATCCTAGCAGCATTCCTGATAAAGAACAGGCAAT
TTCTGCTCTGCCTGATTATGCATCACAGCCGGGTAAACCGCCGCGTGAAGATTTAAAGCATCATCACCATCATCATTAAgatc
cggctgctaacaaagcccgaaaggaagctgagttggctgctgccaccgctgagcaataactagcataaccccttggggcctct
aaacgggtcttgaggggttttttgctgaaaggaggaactatatccggat
SEQ ID NO: 101 pET26b-Kana-T7-pelB-NbEGa1-PE38-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
 
ctgctgctggtctgctgctcctcgctgcccagccggcgatggccCAGGTTCAGCTGCAAGAAAGCGGAGGTGGTCTGGTTCAG
CCGGGAGGTAGCTTACGCCTGTCATGCGCAGCAAGCGGTCGTACCTTTAGCAGCTATGCGATGGGCTGGTTTCGTCAGGCACC
TGGCAAAGAACGCGAATTTGTGGCGGCAATTCGCTGGTCAGGCGGCTATACCTACTACACCGATAGCGTGAAAGGCCGCTTTA
CCATTAGCCGCGATAACGCCAAGACCACCGTGTACCTGCAAATGAACAGCCTGAAACCGGAAGATACCGCAGTGTACTACTGC
GCAGCGACCTATCTGAGCAGCGATTATAGCCGCTATGCCTTACCGCAGCGTCCGCTGGATTACGACTATTGGGGCCAAGGTAC
GCAGGTTACCGTTAGCAGCGCAGCAGCGCCTGAAGGTGGTAGCCTGGCGGCACTGACCGCACACCAAGCATGTCATCTGCCAC
TGGAAACCTTTACCCGTCATCGTCAGCCGCGTGGCTGGGAACAACTGGAACAATGTGGATATCCGGTGCAGAGACTGGTCGCA
TTATATCTGGCAGCACGTCTGAGTTGGAATCAGGTTGATCAAGTTATTCGTAACGCACTTGCCTCACCGGGTAGCGGTGGTGA
TTTAGGTGAGGCAATTCGTGAACAACCTGAACAGGCACGCTTAGCATTAACCCTTGCAGCCGCAGAAAGTGAAAGATTTGTGC
GTCAGGGTACTGGTAATGATGAGGCCGGTGCAGCAAATGGCCGGCAGACAGCGGAGATGCATTATTAGAGAGAAATTATCCT
ACCGGCGCAGAATTTCTGGGTGATGGGGGTGATGTTAGTTTTCAACACGCGGGACACAGAATTGGACCGTGGAACGTTTACT
GCAGGCACATCGTCAGCTGGAAGAACGCGGTTATGTTTTTGTTGGTTATCATGGACGTTTCTGGAGGCAGCACAGAGCATTG
TCTTTGGCGGTGTGCGGGCGCGTAGCCAGGATCTTGATGCAATTTGGCGTGGTTTTTATATTGCGGGTGATCCTGCGCTGGCG
TATGGATATGCGCAGGATCAGGAGCCGGATGCGCGTGGTCGTATTCGTAATGGTGCCCTGCTGCGGGTTTATGTTCCGCGTAG
CTCACTGCCGGGGTTTTATAGAACAAGCTTAACACTGGCTGCGCCGGAAGCAGCAGGTGAAGTTGAACGTCTGATTGGCCATC
CGCTGCCGTTACGTTTAGATGCAATTACTGGTCCGGAAGAAGAAGGTGGTCGCCTGGAAACCATTCTGGGTTGGCCGCTGGCA
GAAAGAACCGTGGTTATACCTTCCGCAATACCGACCGATCCTCGGAATGTGGGGGGTGATTTAGATCCTAGCAGCATTCCTGA
TAAAGAACAGGCAATTTCTGCTCTGCCTGATTATGCATCACAGCCGGGTAAACCGCCGCGTGAAGATTTAAAGCATCATCACC
ATCATCATTAAgatccggctgctaacaaagcccgaaaggaagctgagttggctgctgccaccgctgagcaataactagcataa
ccccttggggcctctaaacgggtcttgaggggttttttgctgaaaggaggaactatatccggat
SEQ ID NO: 102 pET26b-Kana-T7-pelB-VHH1-PE38-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
 
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGGCGGTGGCAGCGTTCAGGCAGGTGGCAGCCTGAAACTG
AGCTGCGAAGCCAGCCTGCCTAGCACGACCACGCCGTATAGCATGGCCTGGTTTCGTCAGGCGCCTGGACGTGAAAGAGAAGC
GGTGGCCACCATCAGCGCAAGAGGTGGCACTAAACTGTACAGCGATTCTGTGCGCGAACGCTTCACGATTAGCCGTGATAACC
GCAAGAACACGGTCTACCTGAAGATGGATCATCTGAACACCGATGATACCGCCATCTACTATTGCGCCGCAACGGGCAGCATT
CTGTTTAATAGCCGTCCATTCGCGGAAAGCACGTATCCGTTCTGGGGTCAGGGTACTCTGGTCACGGTGAGCAGCCCTGAAGG
TGGTAGCCTGGCGGCACTGACCGCACACCAAGCATGTCATCTGCCACTGGAAACCTTTACCCGTCATCGTCAGCCGCGTGGCT
GGGAACAACTGGAACAATGTGGATATCCGGTGCAGAGACTGGTCGCATTATATCTGGCAGCACGTCTGAGTTGGAATCAGGTT
GATCAAGTTATTCGTAACGCACTTGCCTCACCGGGTAGCGGTGGTGATTTAGGTGAGGCAATTCGTGAACAACCTGAACAGGC
ACGCTTAGCATTAACCCTTGCAGCCGCAGAAAGTGAAAGATTTGTGCGTCAGGGTACTGGTAATGATGAGGCCGGTGCAGCAA
ATGGGCCGGCAGACAGCGGAGATGCATTATTAGAGAGAAATTATCCTACCGGCGCAGAATTTCTGGGTGATGGGGGTGATGTT
AGTTTTTCAACACGCGGGACACAGAATTGGACCGTGGAACGTTTACTGCAGGCACATCGTCAGCTGGAAGAACGCGGTTATGT
TTTTGTTGGTTATCATGGGACGTTTCTGGAGGCAGCACAGAGCATTGTCTTTGGCGGTGTGCGGGCGCGTAGCCAGGATCTTG
ATGCAATTTGGCGTGGTTTTTATATTGCGGGTGATCCTGCGCTGGCGTATGGATATGCGCAGGATCAGGAGCCGGATGCGCGT
GGTCGTATTCGTAATGGTGCCCTGCTGCGGGTTTATGTTCCGCGTAGCTCACTGCCGGGGTTTTATAGAACAAGCTTAACACT
GGCTGCGCCGGAAGCAGCAGGTGAAGTTGAACGTCTGATTGGCCATCCGCTGCCGTTACGTTTAGATGCAATTACTGGTCCGG
AAGAAGAAGGTGGTCGCCTGGAAACCATTCTGGGTTGGCCGCTGGCAGAAAGAACCGTGGTTATACCTTCCGCAATACCGACC
GATCCTCGGAATGTGGGGGGTGATTTAGATCCTAGCAGCATTCCTGATAAAGAACAGGCAATTTCTGCTCTGCCTGATTATGC
ATCACAGCCGGGTAAACCGCCGCGTGAAGATTTAAAGCATCATCACCATCATCATTAAgatccggctgctaacaaagcccgaa
aggaagctgagttggctgctgccaccgctgagcaataactagcataaccccttggggcctctaaacgggtcttgaggggtttt
ttgctgaaaggaggaactatatccggat
SEQ ID NO: 103 pET26b-Kana-T7-pelB-VHH26-PE38-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
 
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccGGCGGTGGCACGGTACAGGCTGGCGGTAGCCTGAAACTG
AGCTGCGCCGCGAGAGGTTGGCGCTTTAGCGGCTATTGCGTAGCCTGGTTTCGTCAAGCACCGGGCAAAGAACGCGAAGCGGT
AGCGATCATTCCGGGTGCTGGCGTGATGTATACCGATGATTCTGTGAAAGGCCGTTTCACCTATTCGCGCGATAATGTGAAGA
ACACCGTTAGTCTGCAAATGAACAGCCTGGAACCGGAAGATACCGCAACGTACTACTGCGCTGTGGATTGGGCATACAGCCAT
AGCTGTCCGAAGAACACGGAAGATTACCGTTATTGGGGCCAGGGCACGCAGGTGACCGTGTCATCACCTGAAGGTGGTAGCCT
GGCGGCACTGACCGCACACCAAGCATGTCATCTGCCACTGGAAACCTTTACCCGTCATCGTCAGCCGCGTGGCTGGGAACAAC
TGGAACAATGTGGATATCCGGTGCAGAGACTGGTCGCATTATATCTGGCAGCACGTCTGAGTTGGAATCAGGTTGATCAAGTT
ATTCGTAACGCACTTGCCTCACCGGGTAGCGGTGGTGATTTAGGTGAGGCAATTCGTGAACAACCTGAACAGGCACGCTTAGC
ATTAACCCTTGCAGCCGCAGAAAGTGAAAGATTTGTGCGTCAGGGTACTGGTAATGATGAGGCCGGTGCAGCAAATGGGCCGG
CAGACAGCGGAGATGCATTATTAGAGAGAAATTATCCTACCGGCGCAGAATTTCTGGGTGATGGGGGTGATGTTAGTTTTTCA
ACACGCGGGACACAGAATTGGACCGTGGAACGTTTACTGCAGGCACATCGTCAGCTGGAAGAACGCGGTTATGTTTTTGTTGG
TTATCATGGGACGTTTCTGGAGGCAGCACAGAGCATTGTCTTTGGCGGTGTGCGGGCGCGTAGCCAGGATCTTGATGCAATTT
GGCGTGGTTTTTATATTGCGGGTGATCCTGCGCTGGCGTATGGATATGCGCAGGATCAGGAGCCGGATGCGCGTGGTCGTATT
CGTAATGGTGCCCTGCTGCGGGTTTATGTTCCGCGTAGCTCACTGCCGGGGTTTTATAGAACAAGCTTAACACTGGCTGCGCC
GGAAGCAGCAGGTGAAGTTGAACGTCTGATTGGCCATCCGCTGCCGTTACGTTTAGATGCAATTACTGGTCCGGAAGAAGAAG
GTGGTCGCCTGGAAACCATTCTGGGTTGGCCGCTGGCAGAAAGAACCGTGGTTATACCTTCCGCAATACCGACCGATCCTCGG
AATGTGGGGGGTGATTTAGATCCTAGCAGCATTCCTGATAAAGAACAGGCAATTTCTGCTCTGCCTGATTATGCATCACAGCC
GGGTAAACCGCCGCGTGAAGATTTAAAGCATCATCACCATCATCATTAAgatccggctgctaacaaagcccgaaaggaagctg
agttggctgctgccaccgctgagcaataactagcataaccccttggggcctctaaacgggtcttgaggggttttttgctgaaa
ggaggaactatatccggat
SEQ ID NO: 104 pET26b-Kana-T7-pelB-127D01-CET40-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
 
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccgaggttcaattagtggaatctggcgggggattggttcaa
gccggtgagtccttgcgtttgagctgcgccgcatctgggagcacatttgatttcaaagtgatgggctggtatcgtcagccgcc
cggaaaacagcgcgagggtgtagcagccatccgcttgagcggaaatatgcactacgccgagtctgtaaaagggcgctttgcta
tttccaaagcgaacgctaagaacacagtgtatcttcagatgaactccttgcgtcccgaagacactgccgtttattattgcaaa
gtaaacatccgtgggcaagactactggggtcaaggaacgcaggtcacagtctcttctggctctggcGAAGCACTGGCAGCACA
TCGTGTTTGCGGAGTTCCACTGGAAACGCTGGCACGTAGCCGTAAACCGCGTGATCTTCCGGATGATCTGTCATGCGCCTATC
AGGCACAGAACATCGTTAGCCTGTTTGTGGCAACACGCATCTTGTTCAGCCATCTGGATAGCGTGTTTACGCTGAACCTGGAT
GAACAAGAACCGGAAGTGGCAGAACGCCTTAGCGCACTGAGACAGATCAACGAGAACAATCCGGGCATGGTGACACAGGTGCT
GACCGTTGCACGCCAGATTTACAACGATTACGTGACGCATCATCCGGGCTTAACTCCGGAACAGACTAGCGCGGGTGCACAAG
CCGCAGATATCTTGAGCCTGTTTTGTCCGGATGCAGATAAACCGTGCGTTGCGAGCAACAACGATCAGGCGAACATCAACATC
GAGTCACGTAGCGGACGCAGCTATCTGCCGGAGAATCGCGCCGTGATTACGCCTCAGGGTGTGACCAACTGGACCTATCAAGA
ACTGGAAGCAACGCATCAGGCACTGACACGCGAAGGTTATGTGTTTGTTGGCTATCATGGCACCAACCATGTTGCGGCACAGA
CCATTGTGAACCGCATTGCACCGGTTCCACGTGGTAACAACACCGAAAACGAAGAGAAATGGGGCGGTCTGTATGTTGCCACG
CACGCAGAAGTTGCTCATGGCTATGCACGCATCAAAGAAGGTACGGGCGAATATGGCTTACCGACGCGTGCAGAACGTGATGC
CAGAGGCGTTATGCTGCGTGTGTACATTCCGAGAGCGAGCTTAGAACGCTTCTATCGCACCAACACACCGCTGGAAAACGCGG
AAGAACACATTACTCAGGTGATTGGCCATAGCCTGCCGTTACGCAACGAAGCCTTTACCGGTCCGGAAAGCGCAGGTGGTGAA
GATGAAACCGTGATTGGCTGGGATATGGCGATTCATGCCGTTGCCATACCGAGCACCATACCGGGCAATGCGTATGAAGAGCT
GGCAATTGACGAAGAAGCAGTTGCCAAAGAACAGAGCATTAGCACCAAACCGCCGTATAAAGAGCGCAAAGACGAACTGAAGt
acccctatgacgtgcctgattacgccggctctggccaccaccaccaccaccactgagatccggctgctaacaaagcccgaaag
gaagctgagttggctgctgccaccgctgagcaataactagcataaccccttggggcctctaaacgggtcttgaggggtttttt
gctgaaaggaggaactatatccggat
SEQ ID NO: 105 pET26b-Kana-T7-pe1B-127D01-DT390-HA-his 
tggcgaatgggacgcgccctgtagcggcgcattaagcgcggcgggtgtggtggttacgcgcagcgtgaccgctacacttgcca
gcgccctagcgcccgctcctttcgctttcttcccttcctttctcgccacgttcgccggctttccccgtcaagctctaaatcgg
gggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaa
caacactcaaccctatctcggtctattcttttgatttataagggattttgccgatttcggcctattggttaaaaaatgagctg
atttaacaaaaatttaacgcgaattttaacaaaatattaacgcttacaatttaggtggcacttttcggggaaatgtgcgcgga
acccctatttgtttatttttctaaatacattcaaatatgtatccgctcatgaattaattcttagaaaaactcatcgagcatca
aatgaaactgcaatttattcatatcaggattatcaataccatatttttgaaaaagccgtttctgtaatgaaggagaaaactca
ccgaggcagttccataggatggcaagatcctggtatcggtctgcgattccgactcgtccaacatcaatacaacctattaattt
cccctcgtcaaaaataaggttatcaagtgagaaatcaccatgagtgacgactgaatccggtgagaatggcaaaagtttatgca
tttctttccagacttgttcaacaggccagccattacgctcgtcatcaaaatcactcgcatcaaccaaaccgttattcattcgt
gattgcgcctgagcgagacgaaatacgcgatcgctgttaaaaggacaattacaaacaggaatcgaatgcaaccggcgcaggaa
cactgccagcgcatcaacaatattttcacctgaatcaggatattcttctaatacctggaatgctgttttcccggggatcgcag
tggtgagtaaccatgcatcatcaggagtacggataaaatgcttgatggtcggaagaggcataaattccgtcagccagtttagt
ctgaccatctcatctgtaacatcattggcaacgctacctttgccatgtttcagaaacaactctggcgcatcgggcttcccata
caatcgatagattgtcgcacctgattgcccgacattatcgcgagcccatttatacccatataaatcagcatccatgttggaat
ttaatcgcggcctagagcaagacgtttcccgttgaatatggctcataacaccccttgtattactgtttatgtaagcagacagt
tttattgttcatgaccaaaatcccttaacgtgagttttcgttccactgagcgtcagaccccgtagaaaagatcaaaggatctt
 
cttgagatcctttttttctgcgcgtaatctgctgcttgcaaacaaaaaaaccaccgctaccagcggtggtttgtttgccggat
caagagctaccaactctttttccgaaggtaactggcttcagcagagcgcagataccaaatactgtccttctagtgtagccgta
gttaggccaccacttcaagaactctgtagcaccgcctacatacctcgctctgctaatcctgttaccagtggctgctgccagtg
gcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcc
cgaagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagc
ctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcg
agtcagtgagcgaggaagcggaagagcgcctgatgcggtattttctccttacgcatctgtgcggtatttcacaccgcaatggt
gcactctcagtacaatctgctctgatgccgcatagttaagccagtatacactccgctatcgctacgtgactgggtcatggctg
cgccccgacacccgccaacacccgctgacgcgccctgacgggcttgtctgctcccggcatccgcttacagacaagctgtgacc
gtctccgggagctgcatgtgtgatgcctccgtgtaagggggatttctgttcatgggggtaatgataccgatgaaacgagagag
gatgctcacgatacgggttactgatgatgaacatgcccggttactggaacgttgtgagggtaaacaactggcggtatggatgc
ggcgggaccagagaaaaatcactcagggtcaatgccagcgcttcgttaatacagatgtaggtgttccacagggtagccagcag
catcctgcgatgcagatccggaacataatggtgcagggcgctgacttccgcgtttccagactttacgaaacacggaaaccgaa
gaccattcatgttgttgctcaggtcgcagacgttttgcagcagcagtcgcttcacgttcgctcgcgtatcggtgattcattct
gctaaccagtaaggcaaccccgccagcctagccgggtcctcaacgacaggagcacgatcatgcgcacccgtggggccgccatg
ccggcgataatggcctgcttctcgccgaaacgtttggtggcgggaccagtgacgaaggcttgagcgagggcgtgcaagattcc
gaataccgcaagcgacaggccgatcatcgtcgcgctccagcgaaagcggtcctcgccgaaaatgacccagagcgctgccggca
cctgtcctacgagttgcatgataaagaagacagtcataagtgcggcgacgatagtcatgccccgcgcccaccggaaggagctg
actgggttgaaggctctcaagggcatcggtcgagatcccggtgcctaatgagtgagctaacttacattaattgcgttgcgctc
actgcccgctttccagtcgggaaacctgtcgtgccagctgcattaatgaatcggccaacgcgcggggagaggcggtttgcgta
ttgggcgccagggtggtttttcttttcaccagtgagacgggcaacagctgattgcccttcaccgcctggccctgagagagttg
cagcaagcggtccacgctggtttgccccagcaggcgaaaatcctgtttgatggtggttaacggcgggatataacatgagctgt
cttcggtatcgtcgtatcccactaccgagatatccgcaccaacgcgcagcccggactcggtaatggcgcgcattgcgcccagc
gccatctgatcgttggcaaccagcatcgcagtgggaacgatgccctcattcagcatttgcatggtttgttgaaaaccggacat
ggcactccagtcgccttcccgttccgctatcggctgaatttgattgcgagtgagatatttatgccagccagccagacgcagac
gcgccgagacagaacttaatgggcccgctaacagcgcgatttgctggtgacccaatgcgaccagatgctccacgcccagtcgc
gtaccgtcttcatgggagaaaataatactgttgatgggtgtctggtcagagacatcaagaaataacgccggaacattagtgca
ggcagcttccacagcaatggcatcctggtcatccagcggatagttaatgatcagcccactgacgcgttgcgcgagaagattgt
gcaccgccgctttacaggcttcgacgccgcttcgttctaccatcgacaccaccacgctggcacccagttgatcggcgcgagat
ttaatcgccgcgacaatttgcgacggcgcgtgcagggccagactggaggtggcaacgccaatcagcaacgactgtttgcccgc
cagttgttgtgccacgcggttgggaatgtaattcagctccgccatcgccgcttccactttttcccgcgttttcgcagaaacgt
ggctggcctggttcaccacgcgggaaacggtctgataagagacaccggcatactctgcgacatcgtataacgttactggtttc
acattcaccaccctgaattgactctcttccgggcgctatcatgccataccgcgaaaggttttgcgccattcgatggtgtccgg
gatctcgacgctctcccttatgcgactcctgcattaggaagcagcccagtagtaggttgaggccgttgagcaccgccgccgca
aggaatggtgcatgcaaggagatggcgcccaacagtcccccggccacggggcctgccaccatacccacgccgaaacaagcgct
catgagcccgaagtggcgagcccgatcttccccatcggtgatgtcggcgatataggcgccagcaaccgcacctgtggcgccgg
tgatgccggccacgatgcgtccggcgtagaggatcgagatctcgatcccgcgaaattaatacgactcactataggggaattgt
gagcggataacaattcccctctagaaataattttgtttaactttaagaaggagatatacatatgaaatacctgctgccgaccg
ctgctgctggtctgctgctcctcgctgcccagccggcgatggccgaggttcaattagtggaatctggcgggggattggttcaa
gccggtgagtccttgcgtttgagctgcgccgcatctgggagcacatttgatttcaaagtgatgggctggtatcgtcagccgcc
cggaaaacagcgcgagggtgtagcagccatccgcttgagcggaaatatgcactacgccgagtctgtaaaagggcgctttgcta
tttccaaagcgaacgctaagaacacagtgtatcttcagatgaactccttgcgtcccgaagacactgccgtttattattgcaaa
gtaaacatccgtgggcaagactactggggtcaaggaacgcaggtcacagtctcttctggctctggcGGCGCGGATGATGTCGT
GGATTCAAGCAAATCATTTGTCATGGAGAACTTCAGCAGCTATCACGGCACCAAACCTGGCTATGTGGATTCGATTCAGAAAG
GCATTCAGAAACCGAAATCCGGTACGCAGGGCAACTATGACGATGATTGGAAAGGCTTCTACAGCACCGATAACAAGTACGAT
GCGGCGGGTTACTCCGTGGATAATGAGAATCCACTGAGCGGTAAAGCTGGCGGTGTGGTGAAAGTCACCTATCCGGGCCTGAC
GAAAGTGCTGGCACTGAAGGTGGATAATGCCGAAACCATTAAGAAAGAACTGGGCCTGAGCCTGACCGAACCTCTGATGGAAC
AGGTTGGCACAGAAGAGTTTATCAAACGCTTTGGCGATGGTGCGTCAAGAGTTGTGCTGAGCCTTCCGTTTGCCGAAGGTAGC
AGCAGCGTTGAGTACATCAACAATTGGGAACAGGCCAAAGCACTGTCAGTGGAACTGGAAATCAACTTTGAGACACGTGGTAA
GAGAGGCCAAGATGCCATGTACGAGTACATGGCGCAAGCGTGCGCAGGCAACCGCGTTCGTAGAAGCGTTGGCAGCAGCCTGA
GCTGCATCAACCTGGACTGGGATGTGATTCGCGATAAGACCAAGACGAAGATCGAAAGCCTGAAAGAACACGGACCAATCAAG
AACAAGATGAGCGAAAGTCCGAACAAGACGGTGAGCGAAGAGAAAGCGAAACAGTATCTGGAAGAGTTTCACCAGACCGCACT
GGAACATCCGGAACTGAGCGAGCTTAAGACCGTGACGGGCACAAATCCGGTGTTTGCAGGCGCAAACTATGCGGCATGGGCGG
TGAACGTGGCACAGGTCATTGACAGCGAAACTGCGGATAATCTGGAGAAAACGACCGCTGCACTGAGCATACTGCCGGGAATT
GGCAGCGTAATGGGCATCGCGGATGGTGCTGTTCACCACAACACCGAAGAGATTGTTGCGCAGAGCATCGCACTGAGCAGCCT
GATGGTGGCACAAGCGATTCCGCTGGTGGGCGAATTGGTCGATATCGGCTTTGCGGCCTATAACTTCGTCGAAAGCATTATCA
ACCTGTTTCAGGTGGTTCACAACAGCTACAATCGTCCGGCCTATAGTCCGGGACACAAGACGCAGCCGTTTCTGtacccctat
gacgtgcctgattacgccggctctggccaccaccaccaccaccactgagatccggctgctaacaaagcccgaaaggaagctga
gttggctgctgccaccgctgagcaataactagcataaccccttggggcctctaaacgggtcttgaggggttttttgctgaaag
gaggaactatatccggat
SEQ ID NO: 106 pSC101-Cm-FNRSP-0033-pelB-238D2-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTCCAACTTTATCCCCCTCCATCCACTCTATTAATTGTTGCCGGCAAGCTAGAGTAAGTAGTTCCCCACTTAATAGTTTCC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
 
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGAAGTGCAACTGGTGGAAAGCGGTGGAGGCCTTGTTCAGACCGGCGGC
AGCCTGCGTCTGTCATGCGCTGCAAGCGGCTTTACGTTCAGCAGCTACGCGATGAGCTGGGTGCGCCAGGCACCGGGCAAAGG
CTTGGAATGGGTTAGCGGCATCAAGTCAAGCGGCGATAGCACCAGATATGCCGGAAGCGTTAAAGGCCGCTTTACCATTTCAC
GCGATAACGCCAAGAACATGCTGTATCTGCAAATGTATAGCCTGAAACCGGAAGATACCGCAGTGTATTACTGCGCCAAGAGC
CGCGTGAGTCGCACGGGTCTGTATACCTATGACAACCGTGGTCAGGGCACGCAGGTGACTGTGTCAAGCCATCATCACCATCA
TCATTAAgatccggggctctggaagatccgcgcgtaccgagttctaattcactggccgtcgttttacaacatacgatgaaggc
gataagtcgcgtaagTTGCTAGCccacgcGGCTCACCTTCACGGGTGGGCCTTTCTTCGCGATCCAcatg
SEQ ID NO: 107 pSC101-Cm-FNRSP-0033-pelB-238D4-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
 
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGAAGTCCAGCTGGTTGAAAGCGGTGGAGGCCTGGTTCAGACCGGCGGA
AGCCTGCGTCTGAGCTGTGCAGCCTCTGGCCGCACGTTTAACAACTACGCGATGGGCTGGTTTCGCCGTGCACCGGGCAAAGA
ACGCGAATTTGTGGCGGCAATCACTCGTAGCGGTGTGAGAAGCGGAGTGTCAGCGATCTATGGCGATAGCGTGAAAGATCGCT
TCACCATCAGCCGCGATAACGCCAAGAACACCTTGTACTTGCAGATGAACAGCCTGAAACCGGAAGATACTGCGGTGTATACC
TGCGCCGCAAGTGCGATCGGCAGCGGTGCACTGCGTCGTTTCGAATACGACTATAGCGGTCAGGGCACACAGGTGACCGTGAG
CAGCCATCATCACCATCATCATTAAgatccggggctctggaagatccgcgcgtaccgagttctaattcactggccgtcgtttt
acaacatacgatgaaggcgataagtcgcgtaagTTGCTAGCccacgcGGCTCACCTTCACGGGTGGGCCTTTCTTCGCGATCC
Acatg
SEQ ID NO: 108 pSC101-Cm-FNRSP-0033-pelB-EGa1-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
CCAACGTTGTTCCATTCCTACAGCCATCCTCGTCTCACGCTCGTCCTTTCGTATGCCTTCATTCACCTCCGCTTCCCAACCA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
 
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccCAGGTTCAGCTGCAAGAAAGCGGAGGTGGTCTGGTTCAGCCGGGAGGT
AGCTTACGCCTGTCATGCGCAGCAAGCGGTCGTACCTTTAGCAGCTATGCGATGGGCTGGTTTCGTCAGGCACCTGGCAAAGA
ACGCGAATTTGTGGCGGCAATTCGCTGGTCAGGCGGCTATACCTACTACACCGATAGCGTGAAAGGCCGCTTTACCATTAGCC
GCGATAACGCCAAGACCACCGTGTACCTGCAAATGAACAGCCTGAAACCGGAAGATACCGCAGTGTACTACTGCGCAGCGACC
TATCTGAGCAGCGATTATAGCCGCTATGCCTTACCGCAGCGTCCGCTGGATTACGACTATTGGGGCCAAGGTACGCAGGTTAC
CGTTAGCAGCGCAGCAGCGCATCATCACCATCATCATTAAgatccggggctctggaagatccgcgcgtaccgagttctaattc
actggccgtcgttttacaacatacgatgaaggcgataagtcgcgtaagTTGCTAGCccacgcGGCTCACCTTCACGGGTGGGC
CTTTCTTCGCGATCCAcatg
SEQ ID NO: 109 pSC101-Cm-FNRSP-0033-pelB-VHH1-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAACTGCCACCTCACGTCTAACAAACCATTATTATCATCACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
 
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGGCGGTGGCAGCGTTCAGGCAGGTGGCAGCCTGAAACTGAGCTGCGAA
GCCAGCCTGCCTAGCACGACCACGCCGTATAGCATGGCCTGGTTTCGTCAGGCGCCTGGACGTGAAAGAGAAGCGGTGGCCAC
CATCAGCGCAAGAGGTGGCACTAAACTGTACAGCGATTCTGTGCGCGAACGCTTCACGATTAGCCGTGATAACCGCAAGAACA
CGGTCTACCTGAAGATGGATCATCTGAACACCGATGATACCGCCATCTACTATTGCGCCGCAACGGGCAGCATTCTGTTTAAT
AGCCGTCCATTCGCGGAAAGCACGTATCCGTTCTGGGGTCAGGGTACTCTGGTCACGGTGAGCAGCCATCATCACCATCATCA
TTAAgatccggggctctggaagatccgcgcgtaccgagttctaattcactggccgtcgttttacaacatacgatgaaggcgat
aagtcgcgtaagTTGCTAGCccacgcGGCTCACCTTCACGGGTGGGCCTTTCTTCGCGATCCAcatg
SEQ ID NO: 110 pSC101-Cm-FNRSP-0033-pelB-VHH26-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
CGCCCCACTCTTATCACTCATGCTTATGCCAGCACTCCATAATTCTCTTACTGTCATGCCATCCCTAAGATGCTTTTCTGTCA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
 
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGGCGGTGGCACGGTACAGGCTGGCGGTAGCCTGAAACTGAGCTGCGCC
GCGAGAGGTTGGCGCTTTAGCGGCTATTGCGTAGCCTGGTTTCGTCAAGCACCGGGCAAAGAACGCGAAGCGGTAGCGATCAT
TCCGGGTGCTGGCGTGATGTATACCGATGATTCTGTGAAAGGCCGTTTCACCTATTCGCGCGATAATGTGAAGAACACCGTTA
GTCTGCAAATGAACAGCCTGGAACCGGAAGATACCGCAACGTACTACTGCGCTGTGGATTGGGCATACAGCCATAGCTGTCCG
AAGAACACGGAAGATTACCGTTATTGGGGCCAGGGCACGCAGGTGACCGTGTCATCACATCATCACCATCATCATTAAgatcc
ggctctggaagatccgcgcgtaccgagttctaattcactggccgtcgttttacaacatacgatgaaggcgataagtcgcgtaa
gTTGCTAGCccacgcGGCTCACCTTCACGGGTGGGCCTTTCTTCGCGATCCAcatg
SEQ ID NO: 111 pSC101-Cm-FNRSP-0033-pelB-238D2-PE38-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGgcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGAAGTGCAACTGGTGGAAAGCGGTGGAGGCCTTGTTCAGACCGGCGGC
AGCCTGCGTCTGTCATGCGCTGCAAGCGGCTTTACGTTCAGCAGCTACGCGATGAGCTGGGTGCGCCAGGCACCGGGCAAAGG
CTTGGAATGGGTTAGCGGCATCAAGTCAAGCGGCGATAGCACCAGATATGCCGGAAGCGTTAAAGGCCGCTTTACCATTTCAC
GCGATAACGCCAAGAACATGCTGTATCTGCAAATGTATAGCCTGAAACCGGAAGATACCGCAGTGTATTACTGCGCCAAGAGC
CGCGTGAGTCGCACGGGTCTGTATACCTATGACAACCGTGGTCAGGGCACGCAGGTGACTGTGTCAAGCCCTGAAGGTGGTAG
CCTGGCGGCACTGACCGCACACCAAGCATGTCATCTGCCACTGGAAACCTTTACCCGTCATCGTCAGCCGCGTGGCTGGGAAC
AACTGGAACAATGTGGATATCCGGTGCAGAGACTGGTCGCATTATATCTGGCAGCACGTCTGAGTTGGAATCAGGTTGATCAA
GTTATTCGTAACGCACTTGCCTCACCGGGTAGCGGTGGTGATTTAGGTGAGGCAATTCGTGAACAACCTGAACAGGCACGCTT
 
AGCATTAACCCTTGCAGCCGCAGAAAGTGAAAGATTTGTGCGTCAGGGTACTGGTAATGATGAGGCCGGTGCAGCAAATGGGC
CGGCAGACAGCGGAGATGCATTATTAGAGAGAAATTATCCTACCGGCGCAGAATTTCTGGGTGATGGGGGTGATGTTAGTTTT
TCAACACGCGGGACACAGAATTGGACCGTGGAACGTTTACTGCAGGCACATCGTCAGCTGGAAGAACGCGGTTATGTTTTTGT
TGGTTATCATGGGACGTTTCTGGAGGCAGCACAGAGCATTGTCTTTGGCGGTGTGCGGGCGCGTAGCCAGGATCTTGATGCAA
TTTGGCGTGGTTTTTATATTGCGGGTGATCCTGCGCTGGCGTATGGATATGCGCAGGATCAGGAGCCGGATGCGCGTGGTCGT
ATTCGTAATGGTGCCCTGCTGCGGGTTTATGTTCCGCGTAGCTCACTGCCGGGGTTTTATAGAACAAGCTTAACACTGGCTGC
GCCGGAAGCAGCAGGTGAAGTTGAACGTCTGATTGGCCATCCGCTGCCGTTACGTTTAGATGCAATTACTGGTCCGGAAGAAG
AAGGTGGTCGCCTGGAAACCATTCTGGGTTGGCCGCTGGCAGAAAGAACCGTGGTTATACCTTCCGCAATACCGACCGATCCT
CGGAATGTGGGGGGTGATTTAGATCCTAGCAGCATTCCTGATAAAGAACAGGCAATTTCTGCTCTGCCTGATTATGCATCACA
GCCGGGTAAACCGCCGCGTGAAGATTTAAAGCATCATCACCATCATCATTAAgatccggggctctggaagatccgcgcgtacc
gagttctaattcactggccgtcgttttacaacatacgatgaaggcgataagtcgcgtaagTTGCTAGCccacgcGGCTCACCT
TCACGGGTGGGCCTTTCTTCGCGATCCAcatg
SEQ ID NO: 112 pSC101-Cm-FNRSP-0033-pelB-238D4-PE38-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGAAGTCCAGCTGGTTGAAAGCGGTGGAGGCCTGGTTCAGACCGGCGGA
AGCCTGCGTCTGAGCTGTGCAGCCTCTGGCCGCACGTTTAACAACTACGCGATGGGCTGGTTTCGCCGTGCACCGGGCAAAGA
ACGCGAATTTGTGGCGGCAATCACTCGTAGCGGTGTGAGAAGCGGAGTGTCAGCGATCTATGGCGATAGCGTGAAAGATCGCT
TCACCATCAGCCGCGATAACGCCAAGAACACCTTGTACTTGCAGATGAACAGCCTGAAACCGGAAGATACTGCGGTGTATACC
TGCGCCGCAAGTGCGATCGGCAGCGGTGCACTGCGTCGTTTCGAATACGACTATAGCGGTCAGGGCACACAGGTGACCGTGAG
CAGCCCTGAAGGTGGTAGCCTGGCGGCACTGACCGCACACCAAGCATGTCATCTGCCACTGGAAACCTTTACCCGTCATCGTC
 
AGCCGCGTGGCTGGGAACAACTGGAACAATGTGGATATCCGGTGCAGAGACTGGTCGCATTATATCTGGCAGCACGTCTGAGT
TGGAATCAGGTTGATCAAGTTATTCGTAACGCACTTGCCTCACCGGGTAGCGGTGGTGATTTAGGTGAGGCAATTCGTGAACA
ACCTGAACAGGCACGCTTAGCATTAACCCTTGCAGCCGCAGAAAGTGAAAGATTTGTGCGTCAGGGTACTGGTAATGATGAGG
CCGGTGCAGCAAATGGGCCGGCAGACAGCGGAGATGCATTATTAGAGAGAAATTATCCTACCGGCGCAGAATTTCTGGGTGAT
GGGGGTGATGTTAGTTTTTCAACACGCGGGACACAGAATTGGACCGTGGAACGTTTACTGCAGGCACATCGTCAGCTGGAAGA
ACGCGGTTATGTTTTTGTTGGTTATCATGGGACGTTTCTGGAGGCAGCACAGAGCATTGTCTTTGGCGGTGTGCGGGCGCGTA
GCCAGGATCTTGATGCAATTTGGCGTGGTTTTTATATTGCGGGTGATCCTGCGCTGGCGTATGGATATGCGCAGGATCAGGAG
CCGGATGCGCGTGGTCGTATTCGTAATGGTGCCCTGCTGCGGGTTTATGTTCCGCGTAGCTCACTGCCGGGGTTTTATAGAAC
AAGCTTAACACTGGCTGCGCCGGAAGCAGCAGGTGAAGTTGAACGTCTGATTGGCCATCCGCTGCCGTTACGTTTAGATGCAA
TTACTGGTCCGGAAGAAGAAGGTGGTCGCCTGGAAACCATTCTGGGTTGGCCGCTGGCAGAAAGAACCGTGGTTATACCTTCC
GCAATACCGACCGATCCTCGGAATGTGGGGGGTGATTTAGATCCTAGCAGCATTCCTGATAAAGAACAGGCAATTTCTGCTCT
GCCTGATTATGCATCACAGCCGGGTAAACCGCCGCGTGAAGATTTAAAGCATCATCACCATCATCATTAAgatccggggctct
ggaagatccgcgcgtaccgagttctaattcactggccgtcgttttacaacatacgatgaaggcgataagtcgcgtaagTTGCT
AGCccacgcGGCTCACCTTCACGGGTGGGCCTTTCTTCGCGATCCAcatg
SEQ ID NO: 113 pSC101-Cm-FNRSP-0033-pelB-EGal-PE38-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccCAGGTTCAGCTGCAAGAAAGCGGAGGTGGTCTGGTTCAGCCGGGAGGT
AGCTTACGCCTGTCATGCGCAGCAAGCGGTCGTACCTTTAGCAGCTATGCGATGGGCTGGTTTCGTCAGGCACCTGGCAAAGA
ACGCGAATTTGTGGCGGCAATTCGCTGGTCAGGCGGCTATACCTACTACACCGATAGCGTGAAAGGCCGCTTTACCATTAGCC
GCGATAACGCCAAGACCACCGTGTACCTGCAAATGAACAGCCTGAAACCGGAAGATACCGCAGTGTACTACTGCGCAGCGACC
 
TATCTGAGCAGCGATTATAGCCGCTATGCCTTACCGCAGCGTCCGCTGGATTACGACTATTGGGGCCAAGGTACGCAGGTTAC
CGTTAGCAGCGCAGCAGCGCCTGAAGGTGGTAGCCTGGCGGCACTGACCGCACACCAAGCATGTCATCTGCCACTGGAAACCT
TTACCCGTCATCGTCAGCCGCGTGGCTGGGAACAACTGGAACAATGTGGATATCCGGTGCAGAGACTGGTCGCATTATATCTG
GCAGCACGTCTGAGTTGGAATCAGGTTGATCAAGTTATTCGTAACGCACTTGCCTCACCGGGTAGCGGTGGTGATTTAGGTGA
GGCAATTCGTGAACAACCTGAACAGGCACGCTTAGCATTAACCCTTGCAGCCGCAGAAAGTGAAAGATTTGTGCGTCAGGGTA
CTGGTAATGATGAGGCCGGTGCAGCAAATGGGCCGGCAGACAGCGGAGATGCATTATTAGAGAGAAATTATCCTACCGGCGCA
GAATTTCTGGGTGATGGGGGTGATGTTAGTTTTTCAACACGCGGGACACAGAATTGGACCGTGGAACGTTTACTGCAGGCACA
TCGTCAGCTGGAAGAACGCGGTTATGTTTTTGTTGGTTATCATGGGACGTTTCTGGAGGCAGCACAGAGCATTGTCTTTGGCG
GTGTGCGGGCGCGTAGCCAGGATCTTGATGCAATTTGGCGTGGTTTTTATATTGCGGGTGATCCTGCGCTGGCGTATGGATAT
GCGCAGGATCAGGAGCCGGATGCGCGTGGTCGTATTCGTAATGGTGCCCTGCTGCGGGTTTATGTTCCGCGTAGCTCACTGCC
GGGGTTTTATAGAACAAGCTTAACACTGGCTGCGCCGGAAGCAGCAGGTGAAGTTGAACGTCTGATTGGCCATCCGCTGCCGT
TACGTTTAGATGCAATTACTGGTCCGGAAGAAGAAGGTGGTCGCCTGGAAACCATTCTGGGTTGGCCGCTGGCAGAAAGAACC
GTGGTTATACCTTCCGCAATACCGACCGATCCTCGGAATGTGGGGGGTGATTTAGATCCTAGCAGCATTCCTGATAAAGAACA
GGCAATTTCTGCTCTGCCTGATTATGCATCACAGCCGGGTAAACCGCCGCGTGAAGATTTAAAGCATCATCACCATCATCATT
AAgatccggggctctggaagatccgcgcgtaccgagttctaattcactggccgtcgttttacaacatacgatgaaggcgataa
gtcgcgtaagTTGCTAGCccacgcGGCTCACCTTCACGGGTGGGCCTTTCTTCGCGATCCAcatg
SEQ ID NO: 114 pSC101-Cm-FNRSP-0033-pelB-VHH1-PE38-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGGCGGTGGCAGCGTTCAGGCAGGTGGCAGCCTGAAACTGAGCTGCGAA
GCCAGCCTGCCTAGCACGACCACGCCGTATAGCATGGCCTGGTTTCGTCAGGCGCCTGGACGTGAAAGAGAAGCGGTGGCCAC
 
CATCAGCGCAAGAGGTGGCACTAAACTGTACAGCGATTCTGTGCGCGAACGCTTCACGATTAGCCGTGATAACCGCAAGAACA
CGGTCTACCTGAAGATGGATCATCTGAACACCGATGATACCGCCATCTACTATTGCGCCGCAACGGGCAGCATTCTGTTTAAT
AGCCGTCCATTCGCGGAAAGCACGTATCCGTTCTGGGGTCAGGGTACTCTGGTCACGGTGAGCAGCCCTGAAGGTGGTAGCCT
GGCGGCACTGACCGCACACCAAGCATGTCATCTGCCACTGGAAACCTTTACCCGTCATCGTCAGCCGCGTGGCTGGGAACAAC
TGGAACAATGTGGATATCCGGTGCAGAGACTGGTCGCATTATATCTGGCAGCACGTCTGAGTTGGAATCAGGTTGATCAAGTT
ATTCGTAACGCACTTGCCTCACCGGGTAGCGGTGGTGATTTAGGTGAGGCAATTCGTGAACAACCTGAACAGGCACGCTTAGC
ATTAACCCTTGCAGCCGCAGAAAGTGAAAGATTTGTGCGTCAGGGTACTGGTAATGATGAGGCCGGTGCAGCAAATGGGCCGG
CAGACAGCGGAGATGCATTATTAGAGAGAAATTATCCTACCGGCGCAGAATTTCTGGGTGATGGGGGTGATGTTAGTTTTTCA
ACACGCGGGACACAGAATTGGACCGTGGAACGTTTACTGCAGGCACATCGTCAGCTGGAAGAACGCGGTTATGTTTTTGTTGG
TTATCATGGGACGTTTCTGGAGGCAGCACAGAGCATTGTCTTTGGCGGTGTGCGGGCGCGTAGCCAGGATCTTGATGCAATTT
GGCGTGGTTTTTATATTGCGGGTGATCCTGCGCTGGCGTATGGATATGCGCAGGATCAGGAGCCGGATGCGCGTGGTCGTATT
CGTAATGGTGCCCTGCTGCGGGTTTATGTTCCGCGTAGCTCACTGCCGGGGTTTTATAGAACAAGCTTAACACTGGCTGCGCC
GGAAGCAGCAGGTGAAGTTGAACGTCTGATTGGCCATCCGCTGCCGTTACGTTTAGATGCAATTACTGGTCCGGAAGAAGAAG
GTGGTCGCCTGGAAACCATTCTGGGTTGGCCGCTGGCAGAAAGAACCGTGGTTATACCTTCCGCAATACCGACCGATCCTCGG
AATGTGGGGGGTGATTTAGATCCTAGCAGCATTCCTGATAAAGAACAGGCAATTTCTGCTCTGCCTGATTATGCATCACAGCC
GGGTAAACCGCCGCGTGAAGATTTAAAGCATCATCACCATCATCATTAAgatccggggctctggaagatccgcgcgtaccgag
ttctaattcactggccgtcgttttacaacatacgatgaaggcgataagtcgcgtaagTTGCTAGCccacgcGGCTCACCTTCA
CGGGTGGGCCTTTCTTCGCGATCCAcatg
SEQ ID NO: 115 pSC101-Cm-FNRSP-0033-pelB-VHH26-PE38-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
 
gtctgctgctcctcgctgcccagccggcgatggccGGCGGTGGCACGGTACAGGCTGGCGGTAGCCTGAAACTGAGCTGCGCC
GCGAGAGGTTGGCGCTTTAGCGGCTATTGCGTAGCCTGGTTTCGTCAAGCACCGGGCAAAGAACGCGAAGCGGTAGCGATCAT
TCCGGGTGCTGGCGTGATGTATACCGATGATTCTGTGAAAGGCCGTTTCACCTATTCGCGCGATAATGTGAAGAACACCGTTA
GTCTGCAAATGAACAGCCTGGAACCGGAAGATACCGCAACGTACTACTGCGCTGTGGATTGGGCATACAGCCATAGCTGTCCG
AAGAACACGGAAGATTACCGTTATTGGGGCCAGGGCACGCAGGTGACCGTGTCATCACCTGAAGGTGGTAGCCTGGCGGCACT
GACCGCACACCAAGCATGTCATCTGCCACTGGAAACCTTTACCCGTCATCGTCAGCCGCGTGGCTGGGAACAACTGGAACAAT
GTGGATATCCGGTGCAGAGACTGGTCGCATTATATCTGGCAGCACGTCTGAGTTGGAATCAGGTTGATCAAGTTATTCGTAAC
GCACTTGCCTCACCGGGTAGCGGTGGTGATTTAGGTGAGGCAATTCGTGAACAACCTGAACAGGCACGCTTAGCATTAACCCT
TGCAGCCGCAGAAAGTGAAAGATTTGTGCGTCAGGGTACTGGTAATGATGAGGCCGGTGCAGCAAATGGGCCGGCAGACAGCG
GAGATGCATTATTAGAGAGAAATTATCCTACCGGCGCAGAATTTCTGGGTGATGGGGGTGATGTTAGTTTTTCAACACGCGGG
ACACAGAATTGGACCGTGGAACGTTTACTGCAGGCACATCGTCAGCTGGAAGAACGCGGTTATGTTTTTGTTGGTTATCATGG
GACGTTTCTGGAGGCAGCACAGAGCATTGTCTTTGGCGGTGTGCGGGCGCGTAGCCAGGATCTTGATGCAATTTGGCGTGGTT
TTTATATTGCGGGTGATCCTGCGCTGGCGTATGGATATGCGCAGGATCAGGAGCCGGATGCGCGTGGTCGTATTCGTAATGGT
GCCCTGCTGCGGGTTTATGTTCCGCGTAGCTCACTGCCGGGGTTTTATAGAACAAGCTTAACACTGGCTGCGCCGGAAGCAGC
AGGTGAAGTTGAACGTCTGATTGGCCATCCGCTGCCGTTACGTTTAGATGCAATTACTGGTCCGGAAGAAGAAGGTGGTCGCC
TGGAAACCATTCTGGGTTGGCCGCTGGCAGAAAGAACCGTGGTTATACCTTCCGCAATACCGACCGATCCTCGGAATGTGGGG
GGTGATTTAGATCCTAGCAGCATTCCTGATAAAGAACAGGCAATTTCTGCTCTGCCTGATTATGCATCACAGCCGGGTAAACC
GCCGCGTGAAGATTTAAAGCATCATCACCATCATCATTAAgatccggggctctggaagatccgcgcgtaccgagttctaattc
actggccgtcgttttacaacatacgatgaaggcgataagtcgcgtaagTTGCTAGCccacgcGGCTCACCTTCACGGGTGGGC
CTTTCTTCGCGATCCAcatg
SEQ ID NO: 116 pSC101-Cm-FNRSP-0033-pelB-CET40-HA-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
 
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccGAAGCACTGGCAGCACATCGTGTTTGCGGAGTTCCACTGGAAACGCTG
GCACGTAGCCGTAAACCGCGTGATCTTCCGGATGATCTGTCATGCGCCTATCAGGCACAGAACATCGTTAGCCTGTTTGTGGC
AACACGCATCTTGTTCAGCCATCTGGATAGCGTGTTTACGCTGAACCTGGATGAACAAGAACCGGAAGTGGCAGAACGCCTTA
GCGCACTGAGACAGATCAACGAGAACAATCCGGGCATGGTGACACAGGTGCTGACCGTTGCACGCCAGATTTACAACGATTAC
GTGACGCATCATCCGGGCTTAACTCCGGAACAGACTAGCGCGGGTGCACAAGCCGCAGATATCTTGAGCCTGTTTTGTCCGGA
TGCAGATAAACCGTGCGTTGCGAGCAACAACGATCAGGCGAACATCAACATCGAGTCACGTAGCGGACGCAGCTATCTGCCGG
AGAATCGCGCCGTGATTACGCCTCAGGGTGTGACCAACTGGACCTATCAAGAACTGGAAGCAACGCATCAGGCACTGACACGC
GAAGGTTATGTGTTTGTTGGCTATCATGGCACCAACCATGTTGCGGCACAGACCATTGTGAACCGCATTGCACCGGTTCCACG
TGGTAACAACACCGAAAACGAAGAGAAATGGGGCGGTCTGTATGTTGCCACGCACGCAGAAGTTGCTCATGGCTATGCACGCA
TCAAAGAAGGTACGGGCGAATATGGCTTACCGACGCGTGCAGAACGTGATGCCAGAGGCGTTATGCTGCGTGTGTACATTCCG
AGAGCGAGCTTAGAACGCTTCTATCGCACCAACACACCGCTGGAAAACGCGGAAGAACACATTACTCAGGTGATTGGCCATAG
CCTGCCGTTACGCAACGAAGCCTTTACCGGTCCGGAAAGCGCAGGTGGTGAAGATGAAACCGTGATTGGCTGGGATATGGCGA
TTCATGCCGTTGCCATACCGAGCACCATACCGGGCAATGCGTATGAAGAGCTGGCAATTGACGAAGAAGCAGTTGCCAAAGAA
CAGAGCATTAGCACCAAACCGCCGTATAAAGAGCGCAAAGACGAACTGAAGCATCATCACCATCATCATTAAgatccggctct
ggaagatccgcgcgtaccgagttctaattcactggccgtcgttttacaacatacgatgaaggcgataagtcgcgtaagTTGCT
AGCccacgcGGCTCACCTTCACGGGTGGGCCTTTCTTCGCGATCCAcatg
SEQ ID NO: 117 pSC101-Cm-FNRSP-0033-pelB-DT390-HA-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
CGCCCCACTCTTATCACTCATGCTTATCGCACCACTCCATAATTCTCTTACTGTCATGCCATCCCTAACATCCTTTTCTCTCA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
 
gtctgctgctcctcgctgcccagccggcgatggccGGCGCGGATGATGTCGTGGATTCAAGCAAATCATTTGTCATGGAGAAC
TTCAGCAGCTATCACGGCACCAAACCTGGCTATGTGGATTCGATTCAGAAAGGCATTCAGAAACCGAAATCCGGTACGCAGGG
CAACTATGACGATGATTGGAAAGGCTTCTACAGCACCGATAACAAGTACGATGCGGCGGGTTACTCCGTGGATAATGAGAATC
CACTGAGCGGTAAAGCTGGCGGTGTGGTGAAAGTCACCTATCCGGGCCTGACGAAAGTGCTGGCACTGAAGGTGGATAATGCC
GAAACCATTAAGAAAGAACTGGGCCTGAGCCTGACCGAACCTCTGATGGAACAGGTTGGCACAGAAGAGTTTATCAAACGCTT
TGGCGATGGTGCGTCAAGAGTTGTGCTGAGCCTTCCGTTTGCCGAAGGTAGCAGCAGCGTTGAGTACATCAACAATTGGGAAC
AGGCCAAAGCACTGTCAGTGGAACTGGAAATCAACTTTGAGACACGTGGTAAGAGAGGCCAAGATGCCATGTACGAGTACATG
GCGCAAGCGTGCGCAGGCAACCGCGTTCGTAGAAGCGTTGGCAGCAGCCTGAGCTGCATCAACCTGGACTGGGATGTGATTCG
CGATAAGACCAAGACGAAGATCGAAAGCCTGAAAGAACACGGACCAATCAAGAACAAGATGAGCGAAAGTCCGAACAAGACGG
TGAGCGAAGAGAAAGCGAAACAGTATCTGGAAGAGTTTCACCAGACCGCACTGGAACATCCGGAACTGAGCGAGCTTAAGACC
GTGACGGGCACAAATCCGGTGTTTGCAGGCGCAAACTATGCGGCATGGGCGGTGAACGTGGCACAGGTCATTGACAGCGAAAC
TGCGGATAATCTGGAGAAAACGACCGCTGCACTGAGCATACTGCCGGGAATTGGCAGCGTAATGGGCATCGCGGATGGTGCTG
TTCACCACAACACCGAAGAGATTGTTGCGCAGAGCATCGCACTGAGCAGCCTGATGGTGGCACAAGCGATTCCGCTGGTGGGC
GAATTGGTCGATATCGGCTTTGCGGCCTATAACTTCGTCGAAAGCATTATCAACCTGTTTCAGGTGGTTCACAACAGCTACAA
TCGTCCGGCCTATAGTCCGGGACACAAGACGCAGCCGTTTCTGCATCATCACCATCATCATTAAgatccggctctggaagatc
cgcgcgtaccgagttctaattcactggccgtcgttttacaacatacgatgaaggcgataagtcgcgtaagTTGCTAGCccacg
cGGCTCACCTTCACGGGTGGGCCTTTCTTCGCGATCCAcatg
SEQ ID NO: 118 pSC101-Cm-FNRSP-0033-pelB-127D01-DT390-HA-His 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTCCTCACTACTCAACCAAGTCATTCTCAGAATAGTCTATCCGGCGACCGAGTTCCTCTTCCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG
GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccgaggttcaattagtggaatctggcgggggattggttcaagccggtgag
 
tccttgcgtttgagctgcgccgcatctgggagcacatttgatttcaaagtgatgggctggtatcgtcagccgcccggaaaaca
gcgcgagggtgtagcagccatccgcttgagcggaaatatgcactacgccgagtctgtaaaagggcgctttgctatttccaaag
cgaacgctaagaacacagtgtatcttcagatgaactccttgcgtcccgaagacactgccgtttattattgcaaagtaaacatc
cgtgggcaagactactggggtcaaggaacgcaggtcacagtctcttctggctctggcGGCGCGGATGATGTCGTGGATTCAAG
CAAATCATTTGTCATGGAGAACTTCAGCAGCTATCACGGCACCAAACCTGGCTATGTGGATTCGATTCAGAAAGGCATTCAGA
AACCGAAATCCGGTACGCAGGGCAACTATGACGATGATTGGAAAGGCTTCTACAGCACCGATAACAAGTACGATGCGGCGGGT
TACTCCGTGGATAATGAGAATCCACTGAGCGGTAAAGCTGGCGGTGTGGTGAAAGTCACCTATCCGGGCCTGACGAAAGTGCT
GGCACTGAAGGTGGATAATGCCGAAACCATTAAGAAAGAACTGGGCCTGAGCCTGACCGAACCTCTGATGGAACAGGTTGGCA
CAGAAGAGTTTATCAAACGCTTTGGCGATGGTGCGTCAAGAGTTGTGCTGAGCCTTCCGTTTGCCGAAGGTAGCAGCAGCGTT
GAGTACATCAACAATTGGGAACAGGCCAAAGCACTGTCAGTGGAACTGGAAATCAACTTTGAGACACGTGGTAAGAGAGGCCA
AGATGCCATGTACGAGTACATGGCGCAAGCGTGCGCAGGCAACCGCGTTCGTAGAAGCGTTGGCAGCAGCCTGAGCTGCATCA
ACCTGGACTGGGATGTGATTCGCGATAAGACCAAGACGAAGATCGAAAGCCTGAAAGAACACGGACCAATCAAGAACAAGATG
AGCGAAAGTCCGAACAAGACGGTGAGCGAAGAGAAAGCGAAACAGTATCTGGAAGAGTTTCACCAGACCGCACTGGAACATCC
GGAACTGAGCGAGCTTAAGACCGTGACGGGCACAAATCCGGTGTTTGCAGGCGCAAACTATGCGGCATGGGCGGTGAACGTGG
CACAGGTCATTGACAGCGAAACTGCGGATAATCTGGAGAAAACGACCGCTGCACTGAGCATACTGCCGGGAATTGGCAGCGTA
ATGGGCATCGCGGATGGTGCTGTTCACCACAACACCGAAGAGATTGTTGCGCAGAGCATCGCACTGAGCAGCCTGATGGTGGC
ACAAGCGATTCCGCTGGTGGGCGAATTGGTCGATATCGGCTTTGCGGCCTATAACTTCGTCGAAAGCATTATCAACCTGTTTC
AGGTGGTTCACAACAGCTACAATCGTCCGGCCTATAGTCCGGGACACAAGACGCAGCCGTTTCTGCATCATCACCATCATCAT
TAAgatccggctctggaagatccgcgcgtaccgagttctaattcactggccgtcgttttacaacatacgatgaaggcgataag
tcgcgtaagTTGCTAGCccacgcGGCTCACCTTCACGGGTGGGCCTTTCTTCGCGATCCAcatg
SEQ ID NO: 119 pSC101-Cm-FNRSP-0033-pelB-127D01-CET40-HA-His

CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCgacttcCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTactgtctagggcggcggatttgtcctactcaggagagcgttcaccgacaaacaacagata
aaacgaaaggcccagtctttcgactgagcctttcgttttatttgatgcaCTAGTgccgatcatattcaataacccttaatata
acttcgtataatgtatgctatacgaagttattagGTCTGAAGAGGAGTTTACGTCCCATATGGGcgagctcgatatcaaatta
cgccccgccctgccactcatcgcagtactgttgtaattcattaagcattctgccgacatggaagccatcacagacggcatgat
gaacctgaatcgccagcggcatcagcaccttgtcgccttgcgtataatatttgcccatggtgaaaacgggggcgaagaagttg
tccatattggccacgtttaaatcaaaactggtgaaactcacccagggattggctgagacgaaaaacatattctcaataaaccc
tttagggaaataggccaggttttcaccgtaacacgccacatcttgcgaatatatgtgtagaaactgccggaaatcgtcgtggt
attcactccagagcgatgaaaacgtttcagtttgctcatggaaaacggtgtaacaagggtgaacactatcccatatcaccagc
tcaccgtctttcattgccatacgaaattccggatgagcattcatcaggcgggcaagaatgtgaataaaggccggataaaactt
gtgcttatttttctttacggtctttaaaaaggccgtaatatccagctgaacggtctggttataggtacattgagcaactgact
gaaatgcctcaaaatgttctttacgatgccattgggatatatcaacggtggtatatccagtgatttttttctccattttagct
tccttagctcctgaaaatctcgataactcaaaaaatacgcccggtagtgatcttatttcattatggtgaaagttggaacctct
tacgtgccgatcaacgtctcattttcgccagatatcGCCAAGCTTAGGATCCGGAACccttaatataacttcgtataatgtat
gctatacgaagttattaggtccctcgaagaggttcactagctaTctagagcttggactcctgttgatagatccagtaatgacc
tcagaactccatctggatttgttcagaacgctcggttgccgccgggcgttttttattggtgagaatccaagcccgGGAAacgG

GATCCtGatCTTGATCCGCCGCAAAGTTTGAGCGAAGTCAATAAACTCTCTACCCATTCAGGGCAATATCTCTCTTGCAGGTG
AATGCAACGTCAAGCGATGGCCtacttGCGGCCTCACACAGGAAAGTACTAGatgaaatacctgctgccgaccgctgctgctg
gtctgctgctcctcgctgcccagccggcgatggccgaggttcaattagtggaatctggcgggggattggttcaagccggtgag
tccttgcgtttgagctgcgccgcatctgggagcacatttgatttcaaagtgatgggctggtatcgtcagccgcccggaaaaca
gcgcgagggtgtagcagccatccgcttgagcggaaatatgcactacgccgagtctgtaaaagggcgctttgctatttccaaag
cgaacgctaagaacacagtgtatcttcagatgaactccttgcgtcccgaagacactgccgtttattattgcaaagtaaacatc
cgtgggcaagactactggggtcaaggaacgcaggtcacagtctcttctggctctggcGAAGCACTGGCAGCACATCGTGTTTG
CGGAGTTCCACTGGAAACGCTGGCACGTAGCCGTAAACCGCGTGATCTTCCGGATGATCTGTCATGCGCCTATCAGGCACAGA
ACATCGTTAGCCTGTTTGTGGCAACACGCATCTTGTTCAGCCATCTGGATAGCGTGTTTACGCTGAACCTGGATGAACAAGAA
CCGGAAGTGGCAGAACGCCTTAGCGCACTGAGACAGATCAACGAGAACAATCCGGGCATGGTGACACAGGTGCTGACCGTTGC
ACGCCAGATTTACAACGATTACGTGACGCATCATCCGGGCTTAACTCCGGAACAGACTAGCGCGGGTGCACAAGCCGCAGATA
TCTTGAGCCTGTTTTGTCCGGATGCAGATAAACCGTGCGTTGCGAGCAACAACGATCAGGCGAACATCAACATCGAGTCACGT
AGCGGACGCAGCTATCTGCCGGAGAATCGCGCCGTGATTACGCCTCAGGGTGTGACCAACTGGACCTATCAAGAACTGGAAGC
AACGCATCAGGCACTGACACGCGAAGGTTATGTGTTTGTTGGCTATCATGGCACCAACCATGTTGCGGCACAGACCATTGTGA
ACCGCATTGCACCGGTTCCACGTGGTAACAACACCGAAAACGAAGAGAAATGGGGCGGTCTGTATGTTGCCACGCACGCAGAA
GTTGCTCATGGCTATGCACGCATCAAAGAAGGTACGGGCGAATATGGCTTACCGACGCGTGCAGAACGTGATGCCAGAGGCGT
TATGCTGCGTGTGTACATTCCGAGAGCGAGCTTAGAACGCTTCTATCGCACCAACACACCGCTGGAAAACGCGGAAGAACACA
TTACTCAGGTGATTGGCCATAGCCTGCCGTTACGCAACGAAGCCTTTACCGGTCCGGAAAGCGCAGGTGGTGAAGATGAAACC
GTGATTGGCTGGGATATGGCGATTCATGCCGTTGCCATACCGAGCACCATACCGGGCAATGCGTATGAAGAGCTGGCAATTGA
CGAAGAAGCAGTTGCCAAAGAACAGAGCATTAGCACCAAACCGCCGTATAAAGAGCGCAAAGACGAACTGAAGCATCATCACC
ATCATCATTAAgatccggctctggaagatccgcgcgtaccgagttctaattcactggccgtcgttttacaacatacgatgaag
gcgataagtcgcgtaagTTGCTAGCccacgcGGCTCACCTTCACGGGTGGGCCTTTCTTCGCGATCCAcatg

## Claims

1. A fusion protein comprising an antibody that binds to a neutrophil or an antigen-binding fragment thereof and a cytotoxin or an active fragment thereof.

2. The fusion protein of claim 1, wherein the antibody is a nanobody.

3. The fusion protein of claim 2, wherein the antibody comprises the complementary determining regions (CDRs) selected from the group consisting of:
CDR1 comprising residues 31-35 of SEQ ID NO: 19, CDR2 comprising residues 50-65 of SEQ ID NO: 19, and CDR3 comprising residues 98-104 of SEQ ID NO: 19;
CDR1 comprising residues 31-35 of SEQ ID NO: 20, CDR2 comprising residues 50-67 of SEQ ID NO: 20, and CDR3 comprising residues 100-111 of SEQ ID NO: 20; and
CDR1 comprising residues 31-35 of SEQ ID NO: 21, CDR2 comprising residues 50-64 of SEQ ID NO: 21, and CDR3 comprising residues 98-115 of SEQ ID NO: 21 ₒ

4. The fusion protein of any one of claims 1-3, wherein the antibody comprises an amino acid sequence selected from the group consisting of SEQ ID NOs: 19, 20 and 21.

5. The fusion protein of any one of claims 1-4, wherein the cytotoxin is Pseudomonas exotoxin (PE).

6. The fusion protein of any one of claims 1-5, wherein the cytotoxin comprises the amino acid sequence of SEQ ID NO: 25.

7. The fusion protein of any one of claims 1-6, wherein the fragment of the cytotoxin comprises the amino acid sequence of SEQ ID NO: 26 or 27.

8. The fusion protein of any one of claims 1-7, further comprising a signal peptide.

9. The fusion protein of claim 8, wherein the signal peptide comprises the amino acid sequence of SEQ ID NO: 28 or 29.

10. The fusion protein of any one of claims 1-9, further comprising a linker and/or a tag sequence.

11. An expression construct comprising a nucleotide sequence encoding an antibody that binds to neutrophils or antigen-binding fragment thereof operably linked to a strictly hypoxia inducible promoter.

12. The expression construct of claim 11, wherein the antibody is a nanobody.

13. The expression construct of claim 12, wherein the antibody comprises complementary determining regions (CDRs) selected from the group consisting of:
CDR1 comprising residues 31-35 of SEQ ID NO: 19, CDR2 comprising residues 50-65 of SEQ ID NO: 19, and CDR3 comprising residues 98-104 of SEQ ID NO: 19;
CDR1 comprising residues 31-35 of SEQ ID NO: 20, CDR2 comprising residues 50-67 of SEQ ID NO: 20, and CDR3 comprising residues 100-111 of SEQ ID NO: 20; and
CDR1 comprising residues 31-35 of SEQ ID NO: 21, CDR2 comprising residues 50-64 of SEQ ID NO: 21, and CDR3 comprising residues 98-115 of SEQ ID NO: 21ₒ

14. The expression construct of any one of claims 11-13, wherein the antibody comprises an amino acid sequence selected from the group consisting of SEQ ID NOs: 19, 20 and 21.

15. The expression construct of any one of claims 11-14, wherein the nucleotide sequence further encodes a signal peptide.

16. The expression construct of claim 15, wherein the signal peptide comprises the amino acid sequence of SEQ ID NO: 28 or 29.

17. The expression construct of any one of claims 11-16, wherein the nucleotide sequence further encodes a tag sequence.

18. An expression construct comprising a nucleotide sequence encoding the fusion protein of any one of claims 1-10 operably linked to a strictly hypoxia inducible promoter.

19. The expression construct of any one of claims 11-18, wherein the strictly hypoxia inducible promoter is selected from the group consisting of pepTp, fnrSp, ysgAp, ssbp1, Hip1, BBa_I14018, BBa_R1074, Ptet-arcA and Ptet-Fnr promoters.

20. A modified bacterium, wherein the bacterium comprises the expression construct of any one of claims 11-19 as compared to an unmodified starting strain.

21. The modified bacterium of claim 20, wherein the bacterium further comprises a hypoxia regulated essential gene expression cassette comprising an essential gene of the bacterium under the control of a strictly hypoxia inducible promoter, and the bacterium is deficient in at least one gene involved in or regulating the endogenous anti-oxidative stress response pathway or a functional expression product thereof.

22. The modified bacterium of claim 21, wherein the strictly hypoxia inducible promoter is selected from pepTp, fnrSp, ysgAp, ssbp1, Hip1, BBa_I14018, BBa_R1074, Ptet-arcA, and Ptet-Fnr promoters.

23. The modified bacterium of claim 21, wherein the strictly hypoxia inducible promoter is the ssbp1 promoter.

24. The modified bacterium of any one of claims 21-23, wherein the expression product of the essential gene is responsible for the synthesis of 2,6-diaminopimelic acid (DAP) within the bacterium, and when cultured under aerobic conditions, the bacterial growth is dependent on the additional DAP or an analog thereof added to the medium.

25. The modified bacterium of claim 24, wherein the essential gene is selected from dapA, dapB, dapD, dapE, argD, dapF, and any combination thereof.

26. The modified bacterium of claim 24, wherein the essential gene is selected from dapA and dapE.

27. The modified bacterium of any one of the claims 21-26, wherein the gene involved in or regulating the endogenous anti-oxidative stress response pathway is an HtrA serine protease family gene.

28. The modified bacterium of any one of claims 21-27, wherein the functional expression product of the gene involved in or regulating the endogenous anti-oxidative stress response pathway is an HtrA serine protease family related protein.

29. The modified bacterium of any one of claims 21-28, wherein the bacterium is deficient in the activity of HtrA serine protease.

30. The modified bacterium of any one of claims 21-29, wherein the gene involved in or regulating the endogenous anti-oxidative stress response pathway is htrA.

31. The modified bacterium of any one of claims 21-30, wherein the bacterium is deficient in htrA.

32. The modified bacterium of any one of claims 21-31, wherein the essential gene is a gene naturally occurring in the bacterial chromosome, wherein the native promoter of the essential gene is functionally replaced by the strictly hypoxia inducible promoter, whereby the expression of the essential gene in the bacterium is completely under the control of the strictly hypoxia inducible promoter.

33. The modified bacterium of any one of claims 21-31, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

34. The modified bacterium of claim 33, wherein the exogenous essential gene expression cassette is integrated into the bacterial chromosome.

35. The modified bacterium of claim 33, wherein the exogenous essential gene expression cassette is outside of the bacterial chromosome.

36. The modified bacterium of claim 35, wherein the exogenous essential gene expression cassette is present in the plasmid carried by the bacterium.

37. The modified bacterium of any one of claims 21-36, which further comprises a pH regulated expression cassette comprising a gene encoding a bacteria-derived hemolysin protein under the control of a promoter that is active under acid pH condition.

38. The modified bacterium of claim 37, wherein the bacteria-derived hemolysin protein is a gram-negative bacterial hemolysin protein.

39. The modified bacterium of claim 38, wherein the gene encoding the gram-negative bacterial hemolysin protein is hlyA or hlyE.

40. The modified bacterium of claim 39, wherein the hlyA or hlyE is derived from Listeria monocytogenes, Vibrio cholerae, or Escherichia coli.

41. The modified bacterium of any one of claims 37-40, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

42. The modified bacterium of any one of claims 37-41, wherein the promoter that is active under acid pH condition is selected from the group consisting of sseA, ssrA, ssaB, ssaG, ssaM, and ssaR.

43. The modified bacterium of any one of claims 37-41, wherein the promoter that is active under acid pH condition is sseA.

44. The modified bacterium of any one of claims 21-43, wherein the unmodified starting strain is a facultative anaerobic bacterium.

45. The modified bacterium of any one of claims 21-44, wherein the bacterium is a bacteirum of Enterobacteriaceae.

46. The modified bacterium of any one of claims 21-45, wherein the bacterium is a bacteirum of Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.

47. The modified bacterium of any one of claims 21-46, wherein the bacterium is selected from the group consisting of Escherichia coli, Enterobacter blattae (E. blattae), Enterobacter fergusonii (E. fergusonii), Enterobacter hermannii (E. hermannii), Enterobacter vulneris (E. vulneris), Salmonella enterica (S. enterica), Salmonella bongori (S. bongori), Salmonella typhi (S. typhi), Salmonella choleraesuis (S. choleraesuis), Salmonella typhimurium (S. typhimurium), Shigella dysenteriae (S. dysenteriae), Shigella flexneri (S. flexneri), Shigella boydii (S. boydii), Shigella sonnei (S. sonnei), Klebsiella pneumoniae (K. peumoniae), Klebsiella oxytoca (K. oxytoca), Yersinia pestis (Y. pestos), Yersinia enterocolitica (Y. enterocolitica), Yersinia pseudotuberculosis (Y. pseudotuberculosis), Yersinia aldouae (Y. aldouae), Yersinia bercovieri (Y. bercovieri), Yersinia frederiksenii (Y. frederiksenii), Yersinia intermedia (Y. intermedia), Yersinia kristersenii (Y. kristersenii), Yersinia mollaretti (Y. mollaretti), Yersinia rohdei (Y. rohdei), Yersinia ruckeri (Y. ruckeri), Citrobacter freundii (C. freundii), Citrobacter koseri (C. kaseri), Citrobacter braakii (C. braakii), Enterobacter aerogenes (E. aerogenes), Enterobacter cloacae (E. cloacae), Enterobacter gergoviae (E. gergoviac), Enterobacter sakazakii (E. sakazakii), Enterobacter taylorae (E. tavlorae), Enterobacter amnigenus (E. aminigenus), Enterobacter intermedius (E. intermedius), Enterobacter asburiae (E. asburiac), Enterobacter cancerogenus (E. cancerogenus), Enterobacter dissolvens (E. dissolvens), Enterobacter nimipressuralis (E. nimipressuralis), Serratia marcescens (S. marcescens), Serratia entomophila (S. entomophila), Serratia ficaria (S. ficaria), Serratia fonticola (S. fonticola), Serratia grimesii (S. grimesii), Serratia liquefaciens (S. liquefaciens), Serratia odorifera (S. odorifera), Serratia plymuthica (S. plymuthica), Serratia proteamaculans (S. proteamaculans), Serratia rubidaea (S. rubidaea), Serratia ureilytica (S. ureilytica), Proteus mirabilis (P. mirabilis), Proteus vulgaris (P. vulgaris), Proteus myxofaciens (P. myxofaciens), Proteus penneri (P. penneri), Proteus hauseri (P. hauseri)), Morganella morganii (M. morganii), Providencia alcalifaciens (P. alcalifaciens), Providencia rustigianii (P. rustigianii), Providencia stuartii (P. stuartii), Providencia rettgeri (P. rettgeri), Providencia heimbachae (P. heimbochae), Hafnia alvei (H. alvei), and Pantoea agglomerans (P. agglomerans).

48. The modified bacterium of any one of claims 21-47, wherein the bacterium is Salmonella typhimurium.

49. The modified bacterium of claim 48, wherein the starting strain is Salmonella typhimurium SL7207.

50. The modified bacterium of any one of claims 21-49, wherein the bacterium expresses a wild-type lipopolysaccharide (LPS).

51. The modified bacterium of any one of claims 21-50, wherein, when administrated to a subject with a tumor, the bacterium is capable of surviving and proliferating in the tumor tissue, but is rapidly cleared in normal tissue.

52. The modified bacterium of any one of claims 21-51, wherein upon administration to a subject having a tumor, the bacterium is capable of expressing and secreting the antibody that binds to neutrophils or antigen-binding fragment thereof, or the fusion protein of any one of claims 1-10 in tumor tissue.

53. The modified bacterium of claim 52, wherein the secreted antibody or antigen-binding fragment thereof or fusion protein is capable of inhibiting the recruitment of neutrophils or killing neutrophils.
